# EUROPEAN PATENT APPLICATION

(11) **EP 4 825 392 A1**
(43) Date of publication of application: **21.10.2026**
(21) Application number: 24902890.3
(22) Date of filing: 12.12.2024
(51) Int. Cl.: A61K 38/26, C07K 14/605, A61P 3/10, A61P 3/04

(54) **METHOD FOR TREATING METABOLIC DISEASES AND PHARMACEUTICAL USE OF GLP-1 ANALOGUE**

(30) Priority: 12.12.2023 CN 202311696184; 12.12.2023 CN 202311696354; 08.03.2024 CN 202410264918; 08.03.2024 CN 202410265036; 05.07.2024 CN 202410899570; 05.07.2024 CN 202410899701; 05.07.2024 CN 202410900383; 05.07.2024 CN 202410900557; 06.09.2024 CN 202411248832; 06.09.2024 CN 202411249019; 17.10.2024 CN 202411450978; 17.10.2024 CN 202411451040
(71) Applicant: Jiangsu Hengrui Pharmaceuticals Co., Ltd., Lianyungang, Jiangsu 222047 (CN)
(72) Inventor: WANG, Quanren, Lianyungang, Jiangsu 222047 (CN); CHEN, Hong, Lianyungang, Jiangsu 222047 (CN); ZENG, Meifang, Lianyungang, Jiangsu 222047 (CN); ZUO, Yue, Lianyungang, Jiangsu 222047 (CN); ZHANG, Lei, Lianyungang, Jiangsu 222047 (CN); LIANG, Bo, Lianyungang, Jiangsu 222047 (CN); YU, Ting, Lianyungang, Jiangsu 222047 (CN); WU, Xueyan, Lianyungang, Jiangsu 222047 (CN)
(74) Representative: J A Kemp LLP
(86) International application number: PCT/CN2024/138786
(87) International publication number: WO 2025/124473

(57) **Abstract**

A method for treating metabolic diseases and pharmaceutical use of a GLP-1 analogue. Specifically, the present invention relates to a method for preventing or treating metabolic diseases, comprising administering the GLP-1 analogue or a pharmaceutically acceptable salt thereof once a week.

## Description

The present disclosure claims priority to the following Chinese patent applications:
CN202311696184.5 and CN202311696354. X submitted on December 12, 2023;
CN202410264918.0 and CN202410265036.6 submitted on March 8, 2024;
CN202410900383.1, CN202410900557.4, CN202410899570.2, and CN202410899701.7 submitted on July 5, 2024;
CN202411248832.5 and CN202411249019. X submitted on September 6, 2024; and
CN202411450978.8 and CN202411451040.8 submitted on October 17, 2024.

### Technical Field

The present disclosure belongs to the field of medicine, and relates to a method for treating metabolic diseases and pharmaceutical use of a GLP-1 analogue.

### Background Art

GLP-1 and GIP are polypeptide hormones synthesized and secreted by the intestines after eating. Both are incretins, and their main physiological effect is to promote insulin release in a glucose-dependent manner by activating corresponding receptors on pancreatic β cells. GLP-1 receptors and GIP receptors are both members of the class B family of G protein-coupled receptors and share similar structures.

GLP-1 is mainly secreted by L cells in the ileum and colon. Its active forms are mainly GLP-1(7-36) and GLP-1(7-37), which are obtained by translational cleavage of proglucagon and have 50% homology with glucagon. GLP-1 receptors are widely distributed in the pancreas, intestines, and central nervous system, and are also expressed in the lungs, heart, kidneys, and immune cells. GLP-1 stimulates insulin secretion by pancreatic β-cells and inhibits glucagon secretion by α-cells in the pancreas in a glucose-dependent manner, thereby lowering blood glucose. Its expression in other tissues also provides multiple benefits, including appetite suppression, delayed gastric emptying, improved memory, and cardiovascular protection.

GIP is mainly secreted by K cells in the duodenum and proximal small intestine, and its receptors are mainly distributed in the pancreatic islets, white fat, bones, and central nervous system. In addition to the glucose-dependent insulinotropic effect, GIP promotes glucagon secretion during hypoglycemia and inhibits glucagon secretion during hyperglycemia. It also exerts effects such as improving lipid metabolism and insulin resistance, as well as suppressing appetite.

The mechanisms of action of GLP-1 and GIP are both overlapping and complementary. Concurrent agonism of GLP-1 and GIP receptors may further enhance insulin secretion, suppress appetite and ameliorate insulin resistance. In recent years, several single-molecule GLP-1/GIP dual receptor agonists have entered clinical development. Among them, tirzepatide developed by Eli Lilly represents the most advanced candidate. It was approved by the FDA in May 2022 for the treatment of type 2 diabetes and granted approval for weight loss indication in November 2023. Clinical trial results of tirzepatide have demonstrated that it exhibits superior hypoglycemic and weight-loss potential compared with GLP-RAs such as dulaglutide and semaglutide, with a favorable safety profile. Its main adverse reactions are gastrointestinal events (nausea, diarrhea, decreased appetite, vomiting, etc.). However, the telpotetide package insert recommends maintenance doses of 5 mg, 10 mg, and 15 mg, administered once a week.

Therefore, for dual-target agonists of GLP-1 and GIP, the development of new, safe and effective therapeutic regimens with reduced administration frequency is an important problem to be urgently addressed at present.

### Summary of the Invention

The present disclosure provides a method for treating metabolic diseases and pharmaceutical use of a GLP-1 analogue.

In a first aspect, the present disclosure provides new dosing regimens for GLP-1 analogues or pharmaceutically acceptable salts thereof.

In a second aspect, the present disclosure provides a method for preventing or treating metabolic diseases, comprising administering a target drug to a subject in need thereof according to the following dosing regimens, wherein the target drug is selected from GLP-1 analogues or pharmaceutically acceptable salts thereof.

In a third aspect, the present disclosure provides a method for improving weight control in a subject in need thereof, comprising administering a target drug to the subject in need thereof in accordance with the following dosing regimens, wherein the target drug is selected from GLP-1 analogues or pharmaceutically acceptable salts thereof.

In a fourth aspect, the present disclosure provides a method for improving blood glucose control in a subject in need thereof, comprising administering a target drug to the subject in accordance with the following dosing regimens, wherein the target drug is selected from GLP-1 analogues or pharmaceutically acceptable salts thereof.

In a fifth aspect, the present disclosure provides a method for improving blood lipid control in a subject in need thereof, comprising administering a target drug to the subject in accordance with the following dosing regimens, wherein the target drug is selected from GLP-1 analogues or pharmaceutically acceptable salts thereof.

In a sixth aspect, the present disclosure provides a target drug, which is administered to a subject in need thereof via the following dosing regimen, wherein the target drug is used for:
1) prevention or treatment of metabolic diseases; 2) improvement of weight control in a subject in need thereof; 3) improvement of blood glucose control in a subject in need thereof; 4) improvement of lipid control in a subject in need thereof. In some embodiments, the subject in need thereof suffers from metabolic diseases.

In some embodiments, the metabolic disease is selected from obesity or overweight. In some implementations, the subject has BMI≥28.0 kg/m² and/or BMI≤40.0 kg/m².

In some embodiments, the subject is obese or overweight and accompanied by at least one weight-related comorbidity. In some embodiments, the comorbidity includes one or more of prediabetes, hypertension, and dyslipidemia. In some embodiments, the subject has BMI≥24.0 kg/m² and/or BMI≤28.0 kg/m², and is accompanied by at least one weight-related comorbidity. For example, the comorbidity includes one or more of prediabetes, hypertension, and dyslipidemia.

In some embodiments, the prediabetic subjects include those with impaired fasting glucose, impaired glucose tolerance, and a combination of both.

In some embodiments, the prediabetic subjects are those with a fasting blood glucose level of 6.1 mmol/L < fasting blood glucose < 7.0 mmol/L, or an HbA1c level of 5.7% < HbA1c < 6.5%.

In some embodiments, the metabolic disease is diabetes.

In some embodiments, the metabolic disease is type 2 diabetes.

In some embodiments, the subject with type 2 diabetes has HbA1c ≥ 7.5% and/or HbA1c ≤ 11%.

In some embodiments, the subjects with type 2 diabetes fail to achieve satisfactory outcomes with lifestyle intervention alone (e.g., dietary and/or exercise management), such as poor blood glucose control following exclusive lifestyle intervention, or failure to reach the target HbA1c level after lifestyle intervention alone. In some embodiments, the type 2 diabetes subject is one whose HbA1c is still greater than 7% after lifestyle intervention alone, for example, HbA1c ≥ 7.5%.

In some embodiments, the subjects with type 2 diabetes exhibit an inadequate response to metformin treatment, such as poor blood glucose control despite metformin therapy, or failure to achieve the target HbA1c level with metformin treatment. In some embodiments, the subject has HbA1c > 7% after receiving metformin treatment, for example, HbA1c ≥ 7.5%.

In some embodiments, the subjects with type 2 diabetes have an insufficient response to combination therapy of metformin and an SGLT2 inhibitor, such as unsatisfactory blood glucose control after treatment with metformin combined with an SGLT2 inhibitor, or failure to achieve the target HbA1c level after treatment with metformin combined with an SGLT2 inhibitor. In some embodiments, the subjects have HbA1c > 7%, such as HbA1c ≥ 7.5%, after treatment with metformin combined with an SGLT2 inhibitor.

In some embodiments, the subjects with type 2 diabetes receiving the target drug are further administered metformin.

In some embodiments, the subjects with type 2 diabetes receiving the target drug are additionally administered metformin as the original stable treatment.

In some embodiments, the subjects with type 2 diabetes receiving the target drug are further administered metformin and an SGLT2 inhibitor.

In some embodiments, the subjects with type 2 diabetes receiving the target drug are further administered metformin and an SGLT2 inhibitor as the original stable treatment.

In some embodiments, stable treatment is defined as no changes in the baseline medications (metformin and SGLT2 inhibitor) and total daily doses prior to administration of the target drug. For example, the metformin dose is ≥ 1500 mg per day or the maximum tolerated dose (≥ 1000 mg per day); the SGLT2 inhibitor dose is the daily dose recommended in the product instructions.

In some embodiments, the subjects with type 2 diabetes have BMI ≥ 22kg/m² and/or BMI ≤ 40.0kg/m².

In some embodiments, the subjects with type 2 diabetes are obese.

In some embodiments, the subjects with type 2 diabetes are non-obese.

In some embodiments, the subjects with type 2 diabetes satisfy at least one of the following conditions:
1) poor control with lifestyle intervention alone (e.g., dietary and/or exercise management) or metformin monotherapy;
2) HbA1c ≥ 7.5% and/or HbA1c ≤ 10.5%;
3) BMI ≥ 22kg/m² and/or BMI ≤ 40.0kg/m².

In some embodiments, the subjects with type 2 diabetes satisfy at least one of the following conditions:
1) poor glucose control despite treatment with metformin or metformin combined with an SGLT2 inhibitor;
2) HbA1c ≥ 7.5% and/or HbA1c ≤ 11%;
3) BMI ≥ 22kg/m² and/or BMI ≤ 40.0kg/m².

In some embodiments, the subjects with diabetes satisfy at least one of the following conditions:
1) being receiving treatment with metformin or metformin combined with an SGLT2 inhibitor;
2) HbA1c ≥ 7.5% and/or HbA1c ≤ 11%;
3) BMI ≥ 22kg/m² and/or BMI ≤ 40.0kg/m².

### <Dosing regimen>

In some embodiments, the dosing regimen I comprises: administering the target drug approximately once a week.

In some embodiments, the dosing regimen II comprises:
administering the target drug approximately once a week during the first treatment period; and,
administering the target drug about once every two weeks or once a week during the second treatment period.

### First treatment period of dosing regimen I or dosing regimen II

In some embodiments, the first treatment period comprises:
administering an initial dose of the target drug once a week for at least about 1 week, followed by,
administering an escalating dose of the target drug once a week for at least about 1 week, followed by,
administering a maintenance dose of the target drug once a week for at least about 2 weeks,
   or,
administering an initial dose of the target drug once a week for at least about 1 week, followed by,
administering a maintenance dose of the target drug once a week for at least about 2 weeks.

In some embodiments, the initial dose is selected from about 0.1 mg to about 2.5 mg, or about 0.5 mg to about 1.5 mg; or any range between these point values. In some embodiments, the initial dose is selected from about 0.1 mg, about 0.2 mg, about 0.3 mg, about 0.4 mg, about 0.5 mg, about 0.6 mg, about 0.7 mg, about 0.8 mg, about 0.9 mg, about 1.0 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, 2.1 mg, about 2.2 mg, about 2.3 mg, about 2.4 mg, about 2.5 mg; or any range between any of the above point values. In some embodiments, the initial dose is selected from about 0.5 mg, about 1.0 mg, or about 1.5 mg.

In some embodiments, the escalating dose is selected from about 0.5 mg to about 10.0 mg, about 1.0 mg to about 10.0 mg, about 1.0 mg to about 6.0 mg, about 1.0 mg to about 5.0 mg, about 2.0 mg to about 5.0 mg, about 2.0 mg to about 4.0 mg, about 2.0 mg to about 3.0 mg, about 2.0 mg to about 2.5 mg, about 1.0 mg to about 7.5 mg, about 1.0 mg to about 4.5 mg, about 1.5 mg to about 4.5 mg, about 2.0 mg to about 4.5 mg, about 3.0 mg to about 5.0 mg, about 3.0 mg to about 4.5 mg, and about 4.0 mg to about 6.0 mg; or any range between these point values.

In some embodiments, the escalating dose is selected from about 1.0 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.6 mg, about 2.7 mg, about 2.8 mg, about 2.9 mg, about 3.0 mg, about 3.1 mg, about 3.2 mg, about 3.3 mg, about 3.4 mg, about 3.5 mg, about 3.6 mg, about 3.7 mg, about 3.8 mg, about 3.9 mg, about 4.0 mg, about 4.1 mg, about 4.2 mg, about 4.3 mg, about 4.4 mg, about 4.5 mg, about 4.6 mg, about 4.7 mg, about 4.8 mg, about 4.9 mg, about 5.0 mg, about 5.1 mg, about 5.2 mg, about 5.3 mg, about 5.4 mg, about 5.5 mg, about 5.6 mg, about 5.7 mg, about 5.8 mg, about 5.9 mg, about 6.0 mg, about 6.1 mg, about 6.2 mg, about 6.3 mg, about 6.4 mg, about 6.5 mg, about 6.6 mg, about 6.7 mg, about 6.8 mg, about 6.9 mg, about 7.0 mg, about 7.1 mg, about 7.2 mg, about 7.3 mg, about 7.4 mg, about 7.5 mg, about 7.6 mg, about 7.7 mg, about 7.8 mg, about 7.9 mg, about 8.0 mg, about 8.1 mg, about 8.2 mg, about 8.3 mg, about 8.4 mg, about 8.5 mg, about 8.6 mg, about 8.7 mg, about 8.8 mg, about 8.9 mg, about 9.0 mg, about 9.1 mg, about 9.2 mg, about 9.3 mg, about 9.4 mg, about 9.5 mg, about 9.6 mg, about 9.7 mg, about 9.8 mg, about 9.9 mg, and about 10.0 mg; or any range between these point values. In some embodiments, the escalating dose is selected from about 2.0 mg, about 3.0 mg, about 4.0 mg, about 4.5 mg, about 5.0 mg, or about 6.0 mg.

In some embodiments, the escalating dose includes a second dose, or a second dose and a third dose, or a second dose, a third dose and a fourth dose, or a second dose, a third dose, a fourth dose and a fifth dose, or a second dose, a third dose, a fourth dose, a fifth dose and a sixth dose.

In some embodiments, the second dose is selected from about 1.0 mg to about 5.0 mg, about 1.0 mg to about 3.0 mg, or about 2.0 mg to about 3.0 mg. In some embodiments, the second dose is selected from about 1.0 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.6 mg, about 2.7 mg, about 2.8 mg, about 2.9 mg, about 3.0 mg, about 3.1 mg, about 3.2 mg, about 3.3 mg, about 3.4 mg, about 3.5 mg, about 3.6 mg, about 3.7 mg, about 3.8 mg, about 3.9 mg, about 4.0 mg, about 4.1 mg, about 4.2 mg, about 4.3 mg, about 4.4 mg, about 4.5 mg, about 4.6 mg, about 4.7 mg, about 4.8 mg, about 4.9 mg, about 5.0 mg; or any range between these point values. In some embodiments, the second dose is selected from about 2.0 mg or about 3.0 mg.

In some embodiments, the third dose is selected from about 1.0 mg to about 7.5 mg, about 1.0 mg to about 4.5 mg, about 2.0 mg to about 4.5 mg, or about 3.0 mg to about 4.5 mg. In some embodiments, the third dose is selected from about 1.0 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.6 mg, about 2.7 mg, about 2.8 mg, about 2.9 mg, about 3.0 mg, about 3.1 mg, about 3.2 mg, about 3.3 mg, about 3.4 mg, about 3.5 mg, about 3.6 mg, about 3.7 mg, about 3.8 mg, about 3.9 mg, about 4.0 mg, about 4.1 mg, about 4.2 mg, about 4.3 mg, about 4.4 mg, about 4.5 mg, about 4.6 mg, about 4.7 mg, about 4.8 mg, about 4.9 mg, about 5.0 mg, about 5.1 mg, about 5.2 mg, about 5.3 mg, about 5.4 mg, about 5.5 mg, about 5.6 mg, about 5.7 mg, about 5.8 mg, about 5.9 mg, about 6.0 mg, about 6.1 mg, about 6.2 mg, about 6.3 mg, about 6.4 mg, about 6.5 mg, about 6.6 mg, about 6.7 mg, about 6.8 mg, about 6.9 mg, about 7.0 mg, about 7.1 mg, about 7.2 mg, about 7.3 mg, about 7.4 mg, about 7.5 mg; or any range between these point values. In some embodiments, the third dose is selected from about 3.0 mg, about 4.0 mg, or about 4.5 mg.

In some embodiments, the fourth dose is selected from about 1.0 mg to about 9.0 mg, about 1.0 mg to about 4.0 mg, about 2.0 mg to about 4.0 mg, or about 4.0 mg to about 5.0 mg. In some embodiments, the fourth dose is selected from about 1.0 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.6 mg, about 2.7 mg, about 2.8 mg, about 2.9 mg, about 3.0 mg, about 3.1 mg, about 3.2 mg, about 3.3 mg, about 3.4 mg, about 3.5 mg, about 3.6 mg, about 3.7 mg, about 3.8 mg, about 3.9 mg, about 4.0 mg, about 4.1 mg, about 4.2 mg, about 4.3 mg, about 4.4 mg, about 4.5 mg, about 4.6 mg, about 4.7 mg, about 4.8 mg, about 4.9 mg, about 5.0 mg, about 5.1 mg, about 5.2 mg, about 5.3 mg, about 5.4 mg, about 5.5 mg, about 5.6 mg, about 5.7 mg, about 5.8 mg, about 5.9 mg, about 6.1 mg, about 6.2 mg, about 6.3 mg, about 6.4 mg, about 6.5 mg, about 6.6 mg, about 6.7 mg, about 6.8 mg, about 6.9 mg, about 7.1 mg, about 7.2 mg, about 7.3 mg, about 7.4 mg, about 7.5 mg, about 7.6 mg, about 7.7 mg, about 7.8 mg, about 7.9 mg, about 8.1 mg, about 8.2 mg, about 8.3 mg, about 8.4 mg, about 8.5 mg, about 8.6 mg, about 8.7 mg, about 8.8 mg, about 8.9 mg, and about 9.0 mg; or any range between these point values. In some embodiments, the fourth dose is selected from about 4.0 mg or about 5.0 mg.

In some embodiments, the fifth dose or the sixth dose is selected from about 1.0 mg to about 11.0 mg, about 1.0 mg to about 4.0 mg, about 2.0 mg to about 4.0 mg, about 5.0 mg to about 6.0 mg, and about 6.0 mg to about 7.0 mg. In some embodiments, the fifth dose or the sixth dose is selected from about 1.0 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.6 mg, about 2.7 mg, about 2.8 mg, about 2.9 mg, about 3.0 mg, about 3.1 mg, about 3.2 mg, about 3.3 mg, about 3.4 mg, about 3.5 mg, about 3.6 mg, about 3.7 mg, about 3.8 mg, about 3.9 mg, about 4.0 mg, about 4.1 mg, about 4.2 mg, about 4.3 mg, about 4.4 mg, about 4.5 mg, about 4.6 mg, about 4.7 mg, about 4.8 mg, about 4.9 mg, about 5.0 mg, about 5.1 mg, about 5.2 mg, about 5.3 mg, about 5.4 mg, about 5.5 mg, about 5.6 mg, about 5.7 mg, about 5.8 mg, about 5.9 mg, about 6.1 mg, about 6.2 mg, about 6.3 mg, about 6.4 mg, about 6.5 mg, about 6.6 mg, about 6.7 mg, about 6.8 mg, about 6.9 mg, about 7.1 mg, about 7.2 mg, about 7.3 mg, about 7.4 mg, about 7.5 mg, about 7.6 mg, about 7.7 mg, about 7.8 mg, about 7.9 mg, about 8.1 mg, about 8.2 mg, about 8.3 mg, about 8.4 mg, about 8.5 mg, about 8.6 mg, about 8.7 mg, about 8.8 mg, about 8.9 mg, about 9.0 mg, about 9.1 mg, about 9.2 mg, about 9.3 mg, about 9.4 mg, about 9.5 mg, about 9.6 mg, about 9.7 mg, about 9.8 mg, about 9.9 mg, about 10.1 mg, about 10.2 mg, about 10.3 mg, about 10.4 mg, about 10.5 mg, about 10.6 mg, about 10.7 mg, about 10.8 mg, about 10.9 mg, and about 11.0 mg; or any range between these point values. In some embodiments, the fifth dose is selected from about 5.0 mg or about 6.0 mg. In some embodiments, the sixth dose is about 6.0 mg.

In some embodiments, the maintenance dose is selected from about 0.5 mg to about 10.0 mg, about 3.0 mg to about 10.0 mg, about 1.0 mg to about 10.0 mg, about 1.0 mg to about 8.0 mg, about 1.0 mg to about 6.0 mg, about 1.0 mg to about 5.0 mg, about 2.0 mg to about 6.0 mg, about 3.0 mg to about 6.0 mg, about 4.0 mg to about 6.0 mg, or about 4.5 mg to about 6.0 mg. In some embodiments, the maintenance dose is selected from about 0.5 mg, about 0.6 mg, about 0.7 mg, about 0.8 mg, about 0.9 mg, 1.0 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.6 mg, about 2.7 mg, about 2.8 mg, about 2.9 mg, about 3.0 mg, about 3.1 mg, about 3.2 mg, about 3.3 mg, about 3.4 mg, about 3.5 mg, about 3.6 mg, about 3.7 mg, about 3.8 mg, about 3.9 mg, about 4.0 mg, about 4.1 mg, about 4.2 mg, about 4.3 mg, about 4.4 mg, about 4.5 mg, about 4.6 mg, about 4.7 mg, about 4.8 mg, about 4.9 mg, about 5.0 mg, about 5.1 mg, about 5.2 mg, about 5.3 mg, about 5.4 mg, about 5.5 mg, about 5.6 mg, about 5.7 mg, about 5.8 mg, about 5.9 mg, about 6.0 mg, about 6.1 mg, about 6.2 mg, about 6.3 mg, about 6.4 mg, about 6.5 mg, about 6.6 mg, about 6.7 mg, about 6.8 mg, about 6.9 mg, about 7.0 mg, about 7.1 mg, about 7.2 mg, about 7.3 mg, about 7.4 mg, about 7.5 mg, about 7.6 mg, about 7.7 mg, about 7.8 mg, about 7.9 mg, about 8.0 mg, about 8.1 mg, about 8.2 mg, about 8.3 mg, about 8.4 mg, about 8.5 mg, about 8.6 mg, about 8.7 mg, about 8.8 mg, about 8.9 mg, about 9.0 mg, about 9.1 mg, about 9.2 mg, about 9.3 mg, about 9.4 mg, about 9.5 mg, about 9.6 mg, about 9.7 mg, about 9.8 mg, about 9.9 mg, about 10.0 mg; or any range between these point values. In some embodiments, the maintenance dose is selected from about 1.0 mg, about 2.0 mg, about 3.0 mg, about 4.0 mg, about 4.5 mg, about 6.0 mg, or 8.0 mg.

In some embodiments, the initial dose of the target drug is administered once a week for at least about 1 week, at least about 2 weeks, at least about 3 weeks, at least about 4 weeks, and the like. For example, the administration duration of the initial dose is about 1 week, about 2 weeks, about 3 weeks, about 4 weeks, about 5 weeks, about 6 weeks, about 7 weeks, about 8 weeks, about 10 weeks, about 12 weeks, about 14 weeks, about 16 weeks, and the like. In some embodiments, the initial dose of the target drug is administered for about 4 weeks.

In some embodiments, the escalating dose of the target drug is administered once a week for at least about 1 week, at least about 2 weeks, at least about 3 weeks, at least about 4 weeks, at least about 5 weeks, at least about 6 weeks, at least about 8 weeks, and the like. For example, the administration duration of the escalating dose is about 1 week, about 2 weeks, about 3 weeks, about 4 weeks, about 5 weeks, about 6 weeks, about 7 weeks, about 8 weeks, about 10 weeks, about 12 weeks, about 14 weeks, about 16 weeks, and the like. In some embodiments, the escalating dose of the target drug is administered for about 4 weeks, about 8 weeks, or about 16 weeks. In some embodiments, each escalating dose of the target drug is administered for at least about 4 weeks.

In some embodiments, the maintenance dose of the target drug is administered once a week for at least about 2 weeks, at least about 4 weeks, at least about 6 weeks, at least about 8 weeks, at least about 10 weeks, at least about 12 weeks, at least about 14 weeks, at least about 16 weeks, at least about 18 weeks, at least about 20 weeks, at least about 22 weeks, at least about 24 weeks, and the like. For example, the administration duration of the maintenance dose is about 2 weeks, about 3 weeks, about 4 weeks, about 5 weeks, about 6 weeks, about 7 weeks, about 8 weeks, about 10 weeks, about 12 weeks, about 14 weeks, about 16 weeks, about 18 weeks, about 20 weeks, about 24 weeks, about 26 weeks, about 28 weeks, about 30 weeks, about 32 weeks, about 34 weeks, about 36 weeks, about 40 weeks, about 44 weeks, about 48 weeks, and the like. In some embodiments, during the first treatment period, the maintenance dose of the target drug is administered once a week for about 12 weeks, about 16 weeks, about 20 weeks, about 24 weeks, about 28 weeks, about 32 weeks, about 36 weeks, about 38 weeks, or about 48 weeks.

In some embodiments, the duration of the first treatment period is at least 3 weeks, at least 4 weeks, at least 6 weeks, at least 8 weeks, at least 12 weeks, at least 14 weeks, at least 16 weeks, at least 18 weeks, at least 20 weeks, at least 22 weeks, at least 24 weeks, at least 26 weeks, at least 28 weeks, at least 30 weeks, at least 34 weeks, at least 36 weeks, at least 38 weeks, at least 40 weeks, at least 44 weeks, at least 48 weeks, at least 50 weeks, and the like.

In some embodiments, the duration of the first treatment period of the dosing regimen I or dosing regimen II is about 24 weeks or about 36 weeks.

In some embodiments, the dosing regimen I or first treatment period I-1 comprises:
a) administering an initial dose of the target drug once a week for at least about 1 week, followed by
d) administering a maintenance dose of the target drug once a week for at least about 2 weeks;
   or,
a) administering an initial dose of the target drug once a week for at least about 1 week, followed by
b) administering a second dose of the target drug once a week for at least about 1 week, followed by
d) administering a maintenance dose of the target drug once a week for at least about 2 weeks;
   or,
a) administering an initial dose of the target drug once a week for at least about 1 week, followed by
b) administering a second dose of the target drug once a week for at least about 1 week, followed by
c) administering a third dose of the target drug once a week for at least about 2 weeks, followed by
d) administering a maintenance dose of the target drug once a week for at least about 2 weeks;
   or,
a) administering an initial dose of the target drug once a week for at least about 1 week, followed by
b) administering a second dose of the target drug once a week for at least about 1 week, followed by
c) administering a third dose of the target drug once a week for at least about 2 weeks, followed by
   administering a fourth dose of the target drug once a week for at least about 2 weeks, followed by
   administering a fifth dose of the target drug once a week for at least about 2 weeks, followed by
d) administering a maintenance dose of the target drug once a week for at least about 2 weeks; or,
a) administering an initial dose of the target drug once a week for at least about 1 week, followed by
b) administering a second dose of the target drug once a week for at least about 1 week, followed by
c) administering a third dose of the target drug once a week for at least about 1 week, followed by
   administering a fourth dose of the target drug once a week for at least about 1 week, followed by
   administering a fifth dose of the target drug once a week for at least about 1 week, followed by
   administering a sixth dose of the target drug once a week for at least about 1 week, followed by
d) administering a maintenance dose of the target drug once a week for at least about 2 weeks.

In some embodiments, the dosing regimen I or first treatment period I-2 comprises:
a) administering an initial dose of the target drug once a week for at least about 2 weeks, followed by
d) administering a maintenance dose of the target drug once a week for at least about 4 weeks;
   or,
a) administering an initial dose of the target drug once a week for at least about 2 weeks, followed by
b) administering a second dose of the target drug once a week for at least about 2 weeks, followed by
d) administering a maintenance dose of the target drug once a week for at least about 4 weeks;
   or,
a) administering an initial dose of the target drug once a week for at least about 2 weeks, followed by
b) administering a second dose of the target drug once a week for at least about 2 weeks, followed by
c) administering a third dose of the target drug once a week for at least about 2 weeks, followed by
d) administering a maintenance dose of the target drug once a week for at least about 4 weeks;
   or,
a) administering an initial dose of the target drug once a week for at least about 2 weeks, followed by
b) administering a second dose of the target drug once a week for at least about 2 weeks, followed by
c) administering a third dose of the target drug once a week for at least about 4 weeks, followed by
   administering a fourth dose of the target drug once a week for at least about 4 weeks, followed by
   administering a fifth dose of the target drug once a week for at least about 4 weeks, followed by
d) administering a maintenance dose of the target drug once a week for at least about 4 weeks;
   or,
a) administering an initial dose of the target drug once a week for at least about 2 weeks, followed by
b) administering a second dose of the target drug once a week for at least about 2 weeks, followed by
c) administering a third dose of the target drug once a week for at least about 2 weeks, followed by
   administering a fourth dose of the target drug once a week for at least about 2 weeks, followed by
   administering a fifth dose of the target drug once a week for at least about 2 weeks, followed by
   administering a sixth dose of the target drug once a week for at least about 2 weeks, followed by
d) administering a maintenance dose of the target drug once a week for at least about 4 weeks.

In some embodiments, the dosing regimen I or first treatment period I-3 comprises:
a) administering an initial dose of the target drug once a week for at least about 4 weeks, followed by
d) administering a maintenance dose of the target drug once a week for at least about 4 weeks, such as about 20 weeks;
   or,
a) administering an initial dose of the target drug once a week for at least about 4 weeks, followed by
b) administering a second dose of the target drug once a week for at least about 4 weeks, followed by
d) administering a maintenance dose of the target drug once a week for at least about 4 weeks, such as about 16 weeks;
   or,
a) administering an initial dose of the target drug once a week for at least about 4 weeks, followed by
b) administering a second dose of the target drug once a week for at least about 4 weeks, followed by
c) administering a third dose of the target drug once a week for at least about 4 weeks, followed by
d) administering a maintenance dose of the target drug once a week for at least about 4 weeks, such as about 12 weeks;
   or,
a) administering an initial dose of the target drug once a week for at least about 4 weeks, followed by
b) administering a second dose of the target drug once a week for at least about 4 weeks, followed by
c) administering a third dose of the target drug once a week for at least about 4 weeks, followed by
   administering a fourth dose of the target drug once a week for at least about 4 weeks, followed by
   administering a fifth dose of the target drug once a week for at least about 4 weeks, followed by
d) administering a maintenance dose of the target drug once a week for at least about 4 weeks; or,
a) administering an initial dose of the target drug once a week for at least about 4 weeks, followed by
b) administering a second dose of the target drug once a week for at least about 4 weeks, followed by
c) administering a third dose of the target drug once a week for at least about 4 weeks, followed by
   administering a fourth dose of the target drug once a week for at least about 4 weeks, followed by
   administering a fifth dose of the target drug once a week for at least about 4 weeks, followed by
   administering a sixth dose of the target drug once a week for at least about 4 weeks, followed by
d) administering a maintenance dose of the target drug once a week for at least about 4 weeks, such as about 12 weeks.

In some embodiments, the dosing regimen I or first treatment period I-1-1 comprises:
a) administering the target drug at a dose of about 0.5 mg to about 1.5 mg once a week for at least about 1 week, followed by
d) administering the target drug at a dose of about 1.0 mg to about 6.0 mg once a week for at least about 1 week;
   or,
a) administering the target drug at a dose of about 0.5 mg to about 1.5 mg once a week for at least about 1 week, followed by
b) administering the target drug at a dose of about 2.0 mg to about 3.0 mg once a week for at least about 1 week, followed by
d) administering the target drug at a dose of about 1.0 mg to about 6.0 mg once a week for at least about 2 weeks;
   or,
a) administering the target drug at a dose of about 0.5 mg to about 1.5 mg once a week for at least about 1 week, followed by
b) administering the target drug at a dose of about 2.0 mg to about 3.0 mg once a week for at least about 1 week, followed by
c) administering the target drug at a dose of about 3.0 mg to about 4.5 mg once a week for at least about 2 weeks, followed by
d) administering the target drug at a dose of about 1.0 mg to about 6.0 mg once a week for at least about 2 weeks;
   or,
a) administering the target drug at a dose of about 0.5 mg to about 1.5 mg once a week for at least about 1 week, followed by
b) administering the target drug at a dose of about 2.0 mg to about 3.0 mg once a week for at least about 1 week, followed by
c) administering the target drug at a dose of about 3.0 mg to about 4.5 mg once a week for at least about 1 week, followed by
   administering the target drug at a dose of about 4.0 mg to about 5.0 mg once a week for at least about 1 week, followed by
   administering the target drug at a dose of about 5.0 mg to about 6.0 mg once a week for at least about 1 week, followed by
d) administering the target drug at a dose of about 1.0 mg to about 6.0 mg once a week for at least about 2 weeks;
   or,
a) administering the target drug at a dose of about 0.5 mg to about 1.5 mg once a week for at least about 1 week, followed by
b) administering the target drug at a dose of about 2.0 mg to about 3.0 mg once a week for at least about 1 week, followed by
c) administering the target drug at a dose of about 3.0 mg to about 4.5 mg once a week for at least about 1 week, followed by
   administering the target drug at a dose of about 4.0 mg to about 5.0 mg once a week for at least about 1 week, followed by
   administering the target drug at a dose of about 5.0 mg to about 6.0 mg once a week for at least about 1 week, followed by
   administering the target drug at a dose of about 6.0 mg to about 7.0 mg once a week for at least about 1 week, followed by
d) administering the target drug at a dose of about 1.0 mg to about 8.0 mg once a week for at least about 2 weeks.

In some embodiments, the dosing regimen I or first treatment period I-2-1 comprises:
a) administering the target drug at a dose of about 0.5 mg to about 1.5 mg once a week for at least about 2 weeks, followed by
d) administering the target drug at a dose of about 1.0 mg to about 6.0 mg once a week for at least about 4 weeks;
   or,
a) administering the target drug at a dose of about 0.5 mg to about 1.5 mg once a week for at least about 2 weeks, followed by
b) administering the target drug at a dose of about 2.0 mg to about 3.0 mg once a week for at least about 2 weeks, followed by
d) administering the target drug at a dose of about 1.0 mg to about 6.0 mg once a week for at least about 4 weeks;
   or,
a) administering the target drug at a dose of about 0.5 mg to about 1.5 mg once a week for at least about 2 weeks, followed by
b) administering the target drug at a dose of about 2.0 mg to about 3.0 mg once a week for at least about 2 weeks, followed by
c) administering the target drug at a dose of about 3.0 mg to about 4.5 mg once a week for at least about 2 weeks, followed by
d) administering the target drug at a dose of about 1.0 mg to about 6.0 mg once a week for at least about 4 weeks;
   or,
a) administering the target drug at a dose of about 0.5 mg to about 1.5 mg once a week for at least about 2 weeks, followed by
b) administering the target drug at a dose of about 2.0 mg to about 3.0 mg once a week for at least about 2 weeks, followed by
c) administering the target drug at a dose of about 3.0 mg to about 4.5 mg once a week for at least about 2 weeks, followed by
   administering the target drug at a dose of about 4.0 mg to about 5.0 mg once a week for at least about 2 weeks, followed by
   administering the target drug at a dose of about 5.0 mg to about 6.0 mg once a week for at least about 2 weeks, followed by
d) administering the target drug at a dose of about 1.0 mg to about 6.0 mg once a week for at least about 4 weeks;
   or,
a) administering the target drug at a dose of about 0.5 mg to about 1.5 mg once a week for at least about 2 weeks, followed by
b) administering the target drug at a dose of about 2.0 mg to about 3.0 mg once a week for at least about 2 weeks, followed by
c) administering the target drug at a dose of about 3.0 mg to about 4.5 mg once a week for at least about 2 weeks, followed by
   administering the target drug at a dose of about 4.0 mg to about 5.0 mg once a week for at least about 2 weeks, followed by
   administering the target drug at a dose of about 5.0 mg to about 6.0 mg once a week for at least about 2 weeks, followed by
   administering the target drug at a dose of about 6.0 mg to about 7.0 mg once a week for at least about 2 weeks, followed by
d) administering the target drug at a dose of about 1.0 mg to about 8.0 mg once a week for at least about 4 weeks.

In some embodiments, the dosing regimen I or first treatment period I-3-1 comprises:
a) administering the target drug at a dose of about 0.5 mg to about 1.5 mg once a week for at least about 4 weeks, followed by
d) administering the target drug at a dose of about 1.0 mg to about 6.0 mg once a week for at least about 4 weeks, such as about 20 weeks;
   or,
a) administering the target drug at a dose of about 0.5 mg to about 1.5 mg once a week for at least about 4 weeks, followed by
b) administering the target drug at a dose of about 2.0 mg to about 3.0 mg once a week for at least about 4 weeks, followed by
d) administering the target drug at a dose of about 1.0 mg to about 6.0 mg once a week for at least about 4 weeks, such as about 16 weeks;
   or,
a) administering the target drug at a dose of about 0.5 mg to about 1.5 mg once a week for at least about 4 weeks, followed by
b) administering the target drug at a dose of about 2.0 mg to about 3.0 mg once a week for at least about 4 weeks, followed by
c) administering the target drug at a dose of about 3.0 mg to about 4.5 mg once a week for at least about 4 weeks, followed by
d) administering the target drug at a dose of about 1.0 mg to about 6.0 mg once a week for at least about 4 weeks, such as about 12 weeks;
   or,
a) administering the target drug at a dose of about 0.5 mg to about 1.5 mg once a week for at least about 4 weeks, followed by
b) administering the target drug at a dose of about 2.0 mg to about 3.0 mg once a week for at least about 4 weeks, followed by
c) administering the target drug at a dose of about 3.0 mg to about 4.5 mg once a week for at least about 4 weeks, followed by
   administering the target drug at a dose of about 4.0 mg to about 5.0 mg once a week for at least about 4 weeks, followed by
   administering the target drug at a dose of about 5.0 mg to about 6.0 mg once a week for at least about 4 weeks, followed by
d) administering the target drug at a dose of about 1.0 mg to about 6.0 mg once a week for at least about 4 weeks;
   or,
a) administering the target drug at a dose of about 0.5 mg to about 1.5 mg once a week for at least about 4 weeks, followed by
b) administering the target drug at a dose of about 2.0 mg to about 3.0 mg once a week for at least about 4 weeks, followed by
c) administering the target drug at a dose of about 3.0 mg to about 4.5 mg once a week for at least about 4 weeks, followed by
   administering the target drug at a dose of about 4.0 mg to about 5.0 mg once a week for at least about 4 weeks, followed by
   administering the target drug at a dose of about 5.0 mg to about 6.0 mg once a week for at least about 4 weeks, followed by
   administering the target drug at a dose of about 6.0 mg to about 7.0 mg once a week for at least about 4 weeks, followed by
d) administering the target drug at a dose of about 1.0 mg to about 8.0 mg once a week for at least about 4 weeks, such as about 12 weeks.

In some embodiments, the dosing regimen I or first treatment period I-1-2 comprises:
a) administering the target drug at a dose of about 0.5 mg once a week for at least about 1 week, followed by
d) administering the target drug at a dose of about 1.0 mg once a week for at least about 1 week; or,
a) administering the target drug at a dose of about 1.0 mg once a week for at least about 1 week;
   followed by
d) administering the target drug at a dose of about 1.0 mg once a week for at least about 1 week;
   or,
a) administering the target drug at a dose of about 1.0 mg once a week for at least about 1 week, followed by
d) administering the target drug at a dose of about 2.0 mg once a week for at least about 1 week; or,
a) administering the target drug at a dose of about 1.0 mg once a week for at least about 1 week, followed by
b) administering the target drug at a dose of about 2.0 mg once a week for at least about 1 week, followed by
d) administering the target drug at a dose of about 3.0 mg once a week for at least about 2 weeks;
   or,
a) administering the target drug at a dose of about 1.5 mg once a week for at least about 1 week, followed by
b) administering the target drug at a dose of about 3.0 mg once a week for at least about 1 week, followed by
d) administering the target drug at a dose of about 4.5 mg once a week for at least about 2 weeks;
   or,
a) administering the target drug at a dose of about 1.0 mg once a week for at least about 1 week, followed by
b) administering the target drug at a dose of about 2.0 mg once a week for at least about 1 week, followed by
c) administering the target drug at a dose of about 3.0 mg once a week for at least about 2 weeks, followed by
d) administering the target drug at a dose of about 4.5 mg once a week for at least about 2 weeks;
   or,
a) administering the target drug at a dose of about 1.5 mg once a week for at least about 1 week, followed by
b) administering the target drug at a dose of about 3.0 mg once a week for at least about 1 week, followed by
c) administering the target drug at a dose of about 4.5 mg once a week for at least about 2 weeks, followed by
d) administering the target drug at a dose of about 6.0 mg once a week for at least about 2 weeks;
   or,
a) administering the target drug at a dose of about 1.0 mg once a week for at least about 1 week, followed by
b) administering the target drug at a dose of about 2.0 mg once a week for at least about 1 week, followed by
c) administering the target drug at a dose of about 3.0 mg once a week for at least about 2 weeks, followed by
d) administering the target drug at a dose of about 4.0 mg once a week for at least about 2 weeks;
   or,
a) administering the target drug at a dose of about 1.0 mg once a week for at least about 1 week, followed by
b) administering the target drug at a dose of about 2.0 mg once a week for at least about 1 week, followed by
c) administering the target drug at a dose of about 3.0 mg once a week for at least about 1 week, followed by
   administering the target drug at a dose of about 4.0 mg once a week for at least about 1 week, followed by
   administering the target drug at a dose of about 5.0 mg once a week for at least about 1 week, followed by
d) administering the target drug at a dose of about 6.0 mg once a week for at least about 2 weeks;
   or,
a) administering the target drug at a dose of about 1.0 mg once a week for at least about 1 week, followed by
b) administering the target drug at a dose of about 2.0 mg once a week for at least about 1 week, followed by
c) administering the target drug at a dose of about 3.0 mg once a week for at least about 1 week, followed by
   administering the target drug at a dose of about 4.0 mg once a week for at least about 1 week, followed by
   administering the target drug at a dose of about 5.0 mg once a week for at least about 1 week, followed by
   administering the target drug at a dose of about 6.0 mg once a week for at least about 1 week, followed by
d) administering the target drug at a dose of about 8.0 mg once a week for at least about 2 weeks.

In some embodiments, the dosing regimen I or first treatment period I-2-2 comprises:
a) administering the target drug at a dose of about 0.5 mg once a week for at least about 2 weeks, followed by
d) administering the target drug at a dose of about 1.0 mg once a week for at least about 4 weeks;
   or,
a) administering the target drug at a dose of about 1.0 mg once a week for at least about 2 weeks, followed by
d) administering the target drug at a dose of about 1.0 mg once a week for at least about 4 weeks;
   or,
a) administering the target drug at a dose of about 1.0 mg once a week for at least about 2 weeks, followed by
d) administering the target drug at a dose of about 2.0 mg once a week for at least about 4 weeks;
   or,
a) administering the target drug at a dose of about 1.0 mg once a week for at least about 2 weeks, followed by
b) administering the target drug at a dose of about 2.0 mg once a week for at least about 2 weeks, followed by
d) administering the target drug at a dose of about 3.0 mg once a week for at least about 4 weeks;
   or,
a) administering the target drug at a dose of about 1.5 mg once a week for at least about 2 weeks, followed by
b) administering the target drug at a dose of about 3.0 mg once a week for at least about 2 weeks, followed by
d) administering the target drug at a dose of about 4.5 mg once a week for at least about 4 weeks;
   or,
a) administering the target drug at a dose of about 1.0 mg once a week for at least about 2 weeks, followed by
b) administering the target drug at a dose of about 2.0 mg once a week for at least about 2 weeks, followed by
c) administering the target drug at a dose of about 3.0 mg once a week for at least about 2 weeks, followed by
d) administering the target drug at a dose of about 4.5 mg once a week for at least about 4 weeks;
   or,
a) administering the target drug at a dose of about 1.5 mg once a week for at least about 2 weeks, followed by
b) administering the target drug at a dose of about 3.0 mg once a week for at least about 2 weeks, followed by
c) administering the target drug at a dose of about 4.5 mg once a week for at least about 2 weeks, followed by
d) administering the target drug at a dose of about 6.0 mg once a week for at least about 4 weeks;
   or,
a) administering the target drug at a dose of about 1.0 mg once a week for at least about 2 weeks, followed by
b) administering the target drug at a dose of about 2.0 mg once a week for at least about 2 weeks, followed by
c) administering the target drug at a dose of about 3.0 mg once a week for at least about 2 weeks, followed by
d) administering the target drug at a dose of about 4.0 mg once a week for at least about 4 weeks;
   or,
a) administering the target drug at a dose of about 1.0 mg once a week for at least about 2 weeks, followed by
b) administering the target drug at a dose of about 2.0 mg once a week for at least about 2 weeks, followed by
c) administering the target drug at a dose of about 3.0 mg once a week for at least about 2 weeks, followed by
   administering the target drug at a dose of about 4.0 mg once a week for at least about 2 weeks, followed by
   administering the target drug at a dose of about 5.0 mg once a week for at least about 2 weeks, followed by
d) administering the target drug at a dose of about 6.0 mg once a week for at least about 4 weeks;
   or,
a) administering the target drug at a dose of about 1.0 mg once a week for at least about 2 weeks, followed by
b) administering the target drug at a dose of about 2.0 mg once a week for at least about 2 weeks, followed by
c) administering the target drug at a dose of about 3.0 mg once a week for at least about 2 weeks, followed by
   administering the target drug at a dose of about 4.0 mg once a week for at least about 2 weeks, followed by
   administering the target drug at a dose of about 5.0 mg once a week for at least about 2 weeks, followed by
   administering the target drug at a dose of about 6.0 mg once a week for at least about 2 weeks, followed by
d) administering the target drug at a dose of about 8.0 mg once a week for at least about 4 weeks.

In some embodiments, the dosing regimen I or first treatment period (I-3-2) comprises:
a) administering the target drug at a dose of about 1.0 mg once a week for at least about 4 weeks; followed by
d) administering the target drug at a dose of about 1.0 mg once a week for at least about 4 weeks, such as about 20 weeks;
   or,
a) administering the target drug at a dose of about 0.5 mg once a week for at least about 4 weeks, followed by
d) administering the target drug at a dose of about 1.0 mg once a week for at least about 4 weeks, such as about 20 weeks;
   or,
a) administering the target drug at a dose of about 1.0 mg once a week for at least about 4 weeks, followed by
d) administering the target drug at a dose of about 2.0 mg once a week for at least about 4 weeks, such as about 20 weeks;
   or,
a) administering the target drug at a dose of about 1.0 mg once a week for at least about 4 weeks, followed by
b) administering the target drug at a dose of about 2.0 mg once a week for at least about 4 weeks, followed by
d) administering the target drug at a dose of about 3.0 mg once a week for at least about 4 weeks, such as about 16 weeks;
   or,
a) administering the target drug at a dose of about 1.0 mg once a week for at least about 4 weeks, followed by
b) administering the target drug at a dose of about 2.0 mg once a week for at least about 4 weeks, followed by
d) administering the target drug at a dose of about 3.0 mg once a week for at least about 4 weeks, such as about 12 weeks or about 16 weeks;
   or,
a) administering the target drug at a dose of about 1.0 mg once a week for at least about 4 weeks, followed by
b) administering the target drug at a dose of about 2.0 mg once a week for at least about 4 weeks, followed by
c) administering the target drug at a dose of about 3.0 mg once a week for at least about 4 weeks, followed by
d) administering the target drug at a dose of about 4.5 mg once a week for at least about 4 weeks, such as about 12 weeks;
   or,
a) administering the target drug at a dose of about 1.5 mg once a week for at least about 4 weeks, followed by
b) administering the target drug at a dose of about 3.0 mg once a week for at least about 4 weeks, followed by
d) administering the target drug at a dose of about 4.5 mg once a week for at least about 4 weeks, such as about 16 weeks;
   or,
a) administering the target drug at a dose of about 1.5 mg once a week for at least about 4 weeks, followed by
b) administering the target drug at a dose of about 3.0 mg once a week for at least about 4 weeks, followed by
c) administering the target drug at a dose of about 4.5 mg once a week for at least about 4 weeks, followed by
d) administering the target drug at a dose of about 6.0 mg once a week for at least about 4 weeks, such as about 12 weeks;
   or,
a) administering the target drug at a dose of about 1.0 mg once a week for at least about 4 weeks, followed by
b) administering the target drug at a dose of about 2.0 mg once a week for at least about 4 weeks, followed by
c) administering the target drug at a dose of about 3.0 mg once a week for at least about 4 weeks, followed by
d) administering the target drug at a dose of about 4.0 mg once a week for at least about 4 weeks, such as about 12 weeks;
   or,
a) administering the target drug at a dose of about 1.0 mg once a week for at least about 4 weeks, followed by
b) administering the target drug at a dose of about 2.0 mg once a week for at least about 4 weeks, followed by
c) administering the target drug at a dose of about 3.0 mg once a week for at least about 4 weeks, followed by
   administering the target drug at a dose of about 4.0 mg once a week for at least about 4 weeks, followed by
   administering the target drug at a dose of about 5.0 mg once a week for at least about 4 weeks, followed by
d) administering the target drug at a dose of about 6.0 mg once a week for at least about 4 weeks;
   or,
a) administering the target drug at a dose of about 1.0 mg once a week for at least about 4 weeks, followed by
b) administering the target drug at a dose of about 2.0 mg once a week for at least about 4 weeks, followed by
c) administering the target drug at a dose of about 3.0 mg once a week for at least about 4 weeks, followed by
   administering the target drug at a dose of about 4.0 mg once a week for at least about 4 weeks, followed by
   administering the target drug at a dose of about 5.0 mg once a week for at least about 4 weeks, followed by
   administering the target drug at a dose of about 6.0 mg once a week for at least about 4 weeks, followed by
d) administering the target drug at a dose of about 8.0 mg once a week for at least about 4 weeks, such as about 12 weeks.

In some embodiments, the dosing regimen I or first treatment period I-1-3 comprises:
a) administering the target drug at a dose of about 0.1 mg to about 2.5 mg once a week for at least about 1 week, followed by
d) administering the target drug at a dose of about 0.5 mg to about 10.0 mg once a week for at least about 1 week;
   or,
a) administering the target drug at a dose of about 0.1 mg to about 2.5 mg once a week for at least about 1 week, followed by
b) administering the target drug at a dose of about 1.0 mg to about 5.0 mg once a week for at least about 1 week, followed by
d) administering the target drug at a dose of about 0.5 mg to about 10.0 mg once a week for at least about 2 weeks;
   or,
a) administering the target drug at a dose of about 0.1 mg to about 2.5 mg once a week for at least about 1 week, followed by
b) administering the target drug at a dose of about 1.0 mg to about 5.0 mg once a week for at least about 1 week, followed by
c) administering the target drug at a dose of about 1.0 mg to about 7.5 mg once a week for at least about 2 weeks, followed by
d) administering the target drug at a dose of about 0.5 mg to about 10.0 mg once a week for at least about 2 weeks;
   or,
a) administering the target drug at a dose of about 0.1 mg to about 2.5 mg once a week for at least about 1 week, followed by
b) administering the target drug at a dose of about 1.0 mg to about 4.0 mg once a week for at least about 1 week, followed by
c) administering the target drug at a dose of about 1.0 mg to about 6.5 mg once a week for at least about 1 week, followed by
   administering the target drug at a dose of about 1.0 mg to about 8.0 mg once a week for at least about 1 week, followed by
   administering the target drug at a dose of about 1.0 mg to about 9.5 mg once a week for at least about 1 week, followed by
d) administering the target drug at a dose of about 1.0 mg to about 11.0 mg once a week for at least about 2 weeks;
   or,
a) administering the target drug at a dose of about 0.5 mg to about 1.5 mg once a week for at least about 1 week, followed by
b) administering the target drug at a dose of about 2.0 mg to about 3.0 mg once a week for at least about 1 week, followed by
c) administering the target drug at a dose of about 3.0 mg to about 4.5 mg once a week for at least about 1 week, followed by
   administering the target drug at a dose of about 4.0 mg to about 5.0 mg once a week for at least about 1 week, followed by
   administering the target drug at a dose of about 5.0 mg to about 6.0 mg once a week for at least about 1 week, followed by
   administering the target drug at a dose of about 6.0 mg to about 7.0 mg once a week for at least about 1 week, followed by
d) administering the target drug at a dose of about 1.0 mg to about 8.0 mg once a week for at least about 2 weeks.

In some embodiments, the dosing regimen I or first treatment period I-2-3 comprises:
a) administering the target drug at a dose of about 0.1 mg to about 2.5 mg once a week for at least about 2 weeks, followed by
d) administering the target drug at a dose of about 0.5 mg to about 10.0 mg once a week for at least about 4 weeks;
   or,
a) administering the target drug at a dose of about 0.1 mg to about 2.5 mg once a week for at least about 2 weeks, followed by
b) administering the target drug at a dose of about 1.0 mg to about 5.0 mg once a week for at least about 2 weeks, followed by
d) administering the target drug at a dose of about 0.5 mg to about 10.0 mg once a week for at least about 4 weeks;
   or,
a) administering the target drug at a dose of about 0.1 mg to about 2.5 mg once a week for at least about 2 weeks, followed by
b) administering the target drug at a dose of about 1.0 mg to about 5.0 mg once a week for at least about 2 weeks, followed by
c) administering the target drug at a dose of about 1.0 mg to about 7.5 mg once a week for at least about 2 weeks, followed by
d) administering the target drug at a dose of about 0.5 mg to about 10.0 mg once a week for at least about 4 weeks,
   or,
a) administering the target drug at a dose of about 0.1 mg to about 2.5 mg once a week for at least about 2 weeks, followed by
b) administering the target drug at a dose of about 1.0 mg to about 4.0 mg once a week for at least about 2 weeks, followed by
c) administering the target drug at a dose of about 1.0 mg to about 6.5 mg once a week for at least about 2 weeks, followed by
   administering the target drug at a dose of about 1.0 mg to about 8.0 mg once a week for at least about 2 weeks, followed by
   administering the target drug at a dose of about 1.0 mg to about 9.5 mg once a week for at least about 2 weeks, followed by
d) administering the target drug at a dose of about 1.0 mg to about 11.0 mg once a week for at least about 4 weeks;
   or,
a) administering the target drug at a dose of about 0.5 mg to about 1.5 mg once a week for at least about 2 weeks, followed by
b) administering the target drug at a dose of about 2.0 mg to about 3.0 mg once a week for at least about 2 weeks, followed by
c) administering the target drug at a dose of about 3.0 mg to about 4.5 mg once a week for at least about 2 weeks, followed by
   administering the target drug at a dose of about 4.0 mg to about 5.0 mg once a week for at least about 2 weeks, followed by
   administering the target drug at a dose of about 5.0 mg to about 6.0 mg once a week for at least about 2 weeks, followed by
   administering the target drug at a dose of about 6.0 mg to about 7.0 mg once a week for at least about 2 weeks, followed by
d) administering the target drug at a dose of about 1.0 mg to about 8.0 mg once a week for at least about 4 weeks.

In some embodiments, the dosing regimen I or first treatment period I-3-3 comprises:
a) administering the target drug at a dose of about 0.1 mg to about 2.5 mg once a week for at least about 4 weeks, followed by
d) administering the target drug at a dose of about 0.5 mg to about 10.0 mg once a week for at least about 4 weeks, such as about 20 weeks;
   or,
a) administering the target drug at a dose of about 0.1 mg to about 2.5 mg once a week for at least about 4 weeks, followed by
b) administering the target drug at a dose of about 1.0 mg to about 5.0 mg once a week for at least about 4 weeks, followed by
d) administering the target drug at a dose of about 0.5 mg to about 10.0 mg once a week for at least about 4 weeks, such as about 16 weeks;
   or,
a) administering the target drug at a dose of about 0.1 mg to about 2.5 mg once a week for at least about 4 weeks, followed by
b) administering the target drug at a dose of about 1.0 mg to about 5.0 mg once a week for at least about 4 weeks, followed by
c) administering the target drug at a dose of about 1.0 mg to about 7.5 mg once a week for at least about 4 weeks, followed by
d) administering the target drug at a dose of about 0.5 mg to about 10.0 mg once a week for at least about 4 weeks, such as about 12 weeks;
   or,
a) administering the target drug at a dose of about 0.1 mg to about 2.5 mg once a week for at least about 4 weeks, followed by
b) administering the target drug at a dose of about 1.0 mg to about 4.0 mg once a week for at least about 4 weeks, followed by
c) administering the target drug at a dose of about 1.0 mg to about 6.5 mg once a week for at least about 4 weeks, followed by
   administering the target drug at a dose of about 1.0 mg to about 8.0 mg once a week for at least about 4 weeks, followed by
   administering the target drug at a dose of about 1.0 mg to about 9.5 mg once a week for at least about 4 weeks, followed by
d) administering the target drug at a dose of about 1.0 mg to about 11.0 mg once a week for at least about 4 weeks;
   or,
a) administering the target drug at a dose of about 0.5 mg to about 1.5 mg once a week for at least about 4 weeks, followed by
b) administering the target drug at a dose of about 2.0 mg to about 3.0 mg once a week for at least about 4 weeks, followed by
c) administering the target drug at a dose of about 3.0 mg to about 4.5 mg once a week for at least about 4 weeks, followed by
   administering the target drug at a dose of about 4.0 mg to about 5.0 mg once a week for at least about 4 weeks, followed by
   administering the target drug at a dose of about 5.0 mg to about 6.0 mg once a week for at least about 4 weeks, followed by
   administering the target drug at a dose of about 6.0 mg to about 7.0 mg once a week for at least about 4 weeks, followed by
d) administering the target drug at a dose of about 1.0 mg to about 8.0 mg once a week for at least about 4 weeks, such as about 12 weeks.

### Second treatment period

In some embodiments, the second treatment period comprises:
administering a maintenance dose of the target drug once a week for at least about 1 week;
   or,
administering a maintenance dose of the target drug once every two weeks for at least about 2 weeks;
   or,
administering a maintenance dose of the target drug once a week for at least about 1 week, followed by
administering a maintenance dose of the target drug once every two weeks for at least about 2 weeks;
   or,
administering a maintenance dose of the target drug once a week for at least about 1 week, followed by
administering a seventh dose of the target drug once a week for at least about 1 week, followed by
administering an eighth dose of the target drug once a week for at least about 2 weeks.

In some embodiments, the maintenance dose is selected from about 0.5 mg to about 10.0 mg, about 3.0 mg to about 10.0 mg, about 1.0 mg to about 8.0 mg, about 1.0 mg to about 6.0 mg, about 1.0 mg to about 5.0 mg, about 3.0 mg to about 6.0 mg, about 4.0 mg to about 6.0 mg, or about 4.5 mg to about 6.0 mg. In some embodiments, the maintenance dose is selected from about 0.5 mg, about 0.6 mg, about 0.7 mg, about 0.8 mg, about 0.9 mg, 1.0 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.6 mg, about 2.7 mg, about 2.8 mg, about 2.9 mg, about 3.0 mg, about 3.1 mg, about 3.2 mg, about 3.3 mg, about 3.4 mg, about 3.5 mg, about 3.6 mg, about 3.7 mg, about 3.8 mg, about 3.9 mg, about 4.0 mg, about 4.1 mg, about 4.2 mg, about 4.3 mg, about 4.4 mg, about 4.5 mg, about 4.6 mg, about 4.7 mg, about 4.8 mg, about 4.9 mg, about 5.0 mg, about 5.1 mg, about 5.2 mg, about 5.3 mg, about 5.4 mg, about 5.5 mg, about 5.6 mg, about 5.7 mg, about 5.8 mg, about 5.9 mg, about 6.0 mg, about 6.1 mg, about 6.2 mg, about 6.3 mg, about 6.4 mg, about 6.5 mg, about 6.6 mg, about 6.7 mg, about 6.8 mg, about 6.9 mg, about 7.0 mg, about 7.1 mg, about 7.2 mg, about 7.3 mg, about 7.4 mg, about 7.5 mg, about 7.6 mg, about 7.7 mg, about 7.8 mg, about 7.9 mg, about 8.0 mg, about 8.1 mg, about 8.2 mg, about 8.3 mg, about 8.4 mg, about 8.5 mg, about 8.6 mg, about 8.7 mg, about 8.8 mg, about 8.9 mg, about 9.0 mg, about 9.1 mg, about 9.2 mg, about 9.3 mg, about 9.4 mg, about 9.5 mg, about 9.6 mg, about 9.7 mg, about 9.8 mg, about 9.9 mg, about 10.0 mg; or any range between these point values. In some embodiments, the maintenance dose is selected from about 1.0 mg, about 2.0 mg, about 3.0 mg, about 4.0 mg, about 4.5 mg, about 6.0 mg, or about 8.0 mg.

In some embodiments, the seventh dose and the eighth dose are each independently selected from about 0.5 mg to about 10.0 mg, about 3.0 mg to about 10.0 mg, about 1.0 mg to about 6.0 mg, about 1.0 mg to about 5.0 mg, about 1.0 mg to about 4.5 mg, about 1.0 mg to about 3.0 mg, about 2.0 mg to about 3.0 mg, about 3.0 mg to about 6.0 mg, or about 4.5 mg to about 6.0 mg. In some embodiments, the seventh dose and the eighth dose are each independently selected from about 0.5 mg, about 0.6 mg, about 0.7 mg, about 0.8 mg, about 0.9 mg, 1.0 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.6 mg, about 2.7 mg, about 2.8 mg, about 2.9 mg, about 3.0 mg, about 3.1 mg, about 3.2 mg, about 3.3 mg, about 3.4 mg, about 3.5 mg, about 3.6 mg, about 3.7 mg, about 3.8 mg, about 3.9 mg, about 4.0 mg, about 4.1 mg, about 4.2 mg, about 4.3 mg, about 4.4 mg, about 4.5 mg, about 4.6 mg, about 4.7 mg, about 4.8 mg, about 4.9 mg, about 5.0 mg, about 5.1 mg, about 5.2 mg, about 5.3 mg, about 5.4 mg, about 5.5 mg, about 5.6 mg, about 5.7 mg, about 5.8 mg, about 5.9 mg, about 6.0 mg, about 6.1 mg, about 6.2 mg, about 6.3 mg, about 6.4 mg, about 6.5 mg, about 6.6 mg, about 6.7 mg, about 6.8 mg, about 6.9 mg, about 7.0 mg, about 7.1 mg, about 7.2 mg, about 7.3 mg, about 7.4 mg, about 7.5 mg, about 7.6 mg, about 7.7 mg, about 7.8 mg, about 7.9 mg, about 8.0 mg, about 8.1 mg, about 8.2 mg, about 8.3 mg, about 8.4 mg, about 8.5 mg, about 8.6 mg, about 8.7 mg, about 8.8 mg, about 8.9 mg, about 9.0 mg, about 9.1 mg, about 9.2 mg, about 9.3 mg, about 9.4 mg, about 9.5 mg, about 9.6 mg, about 9.7 mg, about 9.8 mg, about 9.9 mg, about 10.0 mg; or any range between these point values. In some embodiments, the seventh dose and the eighth dose are each independently selected from about 1.0 mg, about 1.5 mg, about 2.0 mg, about 3.0 mg, about 4.5 mg, or about 6.0 mg. In some embodiments, the seventh dose is about 2.0 mg. In some embodiments, the eighth dose is about 3.0 mg.

In some embodiments, the maintenance dose of the target drug is administered once a week for at least about 1 week, at least about 2 weeks, at least about 4 weeks, at least about 6 weeks, at least about 8 weeks, at least about 10 weeks, at least about 12 weeks, at least about 14 weeks, at least about 16 weeks, at least about 18 weeks, at least about 20 weeks, at least about 22 weeks, at least about 24 weeks, and the like. For example, the administration duration of the maintenance dose is about 2 weeks, about 3 weeks, about 4 weeks, about 5 weeks, about 6 weeks, about 7 weeks, about 8 weeks, about 10 weeks, about 12 weeks, about 14 weeks, about 16 weeks, about 18 weeks, about 20 weeks, about 24 weeks, about 26 weeks, about 30 weeks, about 32 weeks, about 34 weeks, about 36 weeks, about 40 weeks, and the like. In some embodiments, during the second treatment period, the maintenance dose of the target drug is administered once a week for about 8 weeks, about 12 weeks, about 24 weeks, or about 28 weeks.

In some embodiments, the maintenance dose of the target drug is administered once every two weeks for at least about 2 weeks, at least about 4 weeks, at least about 6 weeks, at least about 8 weeks, at least about 10 weeks, at least about 12 weeks, at least about 14 weeks, at least about 16 weeks, at least about 18 weeks, at least about 20 weeks, at least about 22 weeks, at least about 24 weeks, and the like. For example, the administration duration of the maintenance dose is about 2 weeks, about 3 weeks, about 4 weeks, about 5 weeks, about 6 weeks, about 7 weeks, about 8 weeks, about 10 weeks, about 12 weeks, about 14 weeks, about 16 weeks, about 18 weeks, about 20 weeks, about 24 weeks, about 26 weeks, about 30 weeks, about 32 weeks, about 34 weeks, about 36 weeks, about 40 weeks, and the like. In some embodiments, the maintenance dose of the target drug is administered once every two weeks for about 20 weeks.

In some embodiments, the seventh dose of the target drug is administered once a week for at least about 1 week, at least about 2 weeks, at least about 3 weeks, at least about 4 weeks, at least about 5 weeks, at least about 6 weeks, at least about 8 weeks, and the like. For example, the administration duration of the escalating dose is about 1 week, about 2 weeks, about 3 weeks, about 4 weeks, about 5 weeks, about 6 weeks, about 7 weeks, about 8 weeks, about 10 weeks, about 12 weeks, about 14 weeks, about 16 weeks, and the like. In some embodiments, the seventh dose of the target drug is administered for about 4 weeks.

In some embodiments, the eighth dose of the target drug is administered once a week for at least about 2 weeks, at least about 4 weeks, at least about 6 weeks, at least about 8 weeks, at least about 10 weeks, at least about 12 weeks, at least about 14 weeks, at least about 16 weeks, at least about 18 weeks, at least about 20 weeks, at least about 22 weeks, at least about 24 weeks, and the like. For example, the administration duration of the eighth dose is about 2 weeks, about 3 weeks, about 4 weeks, about 5 weeks, about 6 weeks, about 7 weeks, about 8 weeks, about 10 weeks, about 12 weeks, about 14 weeks, about 16 weeks, about 18 weeks, about 20 weeks, about 24 weeks, about 26 weeks, about 30 weeks, about 32 weeks, about 34 weeks, about 36 weeks, about 40 weeks, and the like. In some embodiments, the eighth dose of the target drug is administered for about 16 weeks.

In some embodiments, the second treatment period II-1 comprises:
e) administering a maintenance dose of the target drug once a week for at least about 1 week, followed by
f) administering a maintenance dose of the target drug once every two weeks for at least about 2 weeks;
   or,
e) administering a maintenance dose of the target drug once a week for at least about 2 weeks;
   or,
e) administering a maintenance dose of the target drug once a week for at least about 1 week, followed by
g) administering a seventh dose of the target drug once a week for at least about 1 week, followed by
h) administering an eighth dose of the target drug once a week for at least about 2 weeks.

In some embodiments, the second treatment period II-2 comprises:
e) administering a maintenance dose of the target drug once a week for at least about 2 weeks, followed by
f) administering a maintenance dose of the target drug once every two weeks for at least about 4 weeks;
   or,
e) administering a maintenance dose of the target drug once a week for at least about 4 weeks;
   or,
e) administering a maintenance dose of the target drug once a week for at least about 2 weeks, followed by
g) administering a seventh dose of the target drug once a week for at least about 2 weeks, followed by
h) administering an eighth dose of the target drug once a week for at least about 4 weeks.

In some embodiments, the second treatment period II-3 comprises:
e) administering a maintenance dose of the target drug once a week for at least about 4 weeks, such as about 8 weeks, followed by
f) administering a maintenance dose of the target drug once every two weeks for at least about 4 weeks, such as about 20 weeks;
   or,
e) administering a maintenance dose of the target drug once a week for at least about 4 weeks, such as about 4 weeks, about 8 weeks, about 12 weeks, about 16 weeks, about 20 weeks, about 24 weeks, or about 28 weeks;
   or,
e) administering a maintenance dose of the target drug once a week for at least about 4 weeks, such as about 8 weeks, followed by
g) administering a seventh dose of the target drug once a week for at least about 4 weeks, such as about 4 weeks, followed by
h) administering an eighth dose of the target drug once a week for at least about 4 weeks, such as about 16 weeks.

In some embodiments, the second treatment period II-1-1 comprises:
e) administering the target drug at a dose of about 1.0 mg to about 6.0 mg once a week for at least about 1 week, followed by
f) administering the target drug at a dose of about 1.0 mg to about 6.0 mg once every two weeks for at least about 2 weeks;
   or,
e) administering the target drug at a dose of about 1.0 mg to about 6.0 mg once a week for at least about 2 weeks;
   or,
e) administering the target drug at a dose of about 1.0 mg to about 6.0 mg once a week for at least about 1 week, followed by
g) administering the target drug at a dose of about 2.0 mg to about 3.0 mg once a week for at least about 1 week, followed by
h) administering the target drug at a dose of about 2.0 mg to about 3.0 mg once a week for at least about 2 weeks.

In some embodiments, the second treatment period II-2-1 comprises:
e) administering the target drug at a dose of about 1.0 mg to about 6.0 mg once a week for at least about 2 weeks, followed by
f) administering the target drug at a dose of about 1.0 mg to about 6.0 mg once every two weeks for at least about 4 weeks;
   or,
e) administering the target drug at a dose of about 1.0 mg to about 6.0 mg once a week for at least about 4 weeks;
   or,
e) administering the target drug at a dose of about 1.0 mg to about 6.0 mg once a week for at least about 2 weeks, followed by
g) administering the target drug at a dose of about 2.0 mg to about 3.0 mg once a week for at least about 2 weeks, followed by
h) administering the target drug at a dose of about 2.0 mg to about 3.0 mg once a week for at least about 4 weeks.

In some embodiments, the second treatment period II-3-1 comprises:
e) administering the target drug at a dose of about 1.0 mg to about 6.0 mg once a week for at least about 4 weeks, such as about 8 weeks, followed by
f) administering the target drug at a dose of about 1.0 mg to about 6.0 mg once every two weeks for at least about 4 weeks, such as about 20 weeks;
   or,
e) administering the target drug at a dose of about 1.0 mg to about 6.0 mg once a week for at least about 4 weeks, such as about 4 weeks, about 8 weeks, about 12 weeks, about 16 weeks, about 20 weeks, about 24 weeks, or about 28 weeks;
   or,
e) administering the target drug at a dose of about 1.0 mg to about 6.0 mg once a week for at least about 4 weeks, such as about 8 weeks, followed by
g) administering the target drug at a dose of about 2.0 mg to about 3.0 mg once a week for at least about 4 weeks, such as about 4 weeks, followed by
h) administering the target drug at a dose of about 2.0 mg to about 3.0 mg once a week for at least about 4 weeks, such as about 16 weeks.

In some embodiments, the second treatment period II-1-2 comprises:
e) administering the target drug at a dose of about 3.0 mg once a week for at least about 1 week, followed by
f) administering the target drug at a dose of about 3.0 mg once every two weeks for at least about 2 weeks;
   or,
e) administering the target drug at a dose of about 4.5 mg once a week for at least about 1 week, followed by
f) administering the target drug at a dose of about 4.5 mg once every two weeks for at least about 2 weeks;
   or,
e) administering the target drug at a dose of about 6.0 mg once a week for at least about 1 week, followed by
f) administering the target drug at a dose of about 6.0 mg once every two weeks for at least about 2 weeks;
   or,
e) administering the target drug at a dose of about 1.0 mg once a week for at least about 2 weeks;
   or,
e) administering the target drug at a dose of about 2.0 mg once a week for at least about 2 weeks;
   or,
e) administering the target drug at a dose of about 3.0 mg once a week for at least about 2 weeks;
   or,
e) administering the target drug at a dose of about 4.0 mg once a week for at least about 2 weeks;
   or,
e) administering the target drug at a dose of about 4.5 mg once a week for at least about 2 weeks;
   or,
e) administering the target drug at a dose of about 6.0 mg once a week for at least about 2 weeks;
   or,
e) administering the target drug at a dose of about 1.0 mg once a week for at least about 1 week, followed by
g) administering the target drug at a dose of about 2.0 mg once a week for at least about 1 week, followed by
h) administering the target drug at a dose of about 3.0 mg once a week for at least about 2 weeks.

In some embodiments, the second treatment period II-2-2 comprises:
e) administering the target drug at a dose of about 3.0 mg once a week for at least about 2 weeks, followed by
f) administering the target drug at a dose of about 3.0 mg once every two weeks for at least about 4 weeks;
   or,
e) administering the target drug at a dose of about 4.5 mg once a week for at least about 2 weeks, followed by
f) administering the target drug at a dose of about 4.5 mg once every two weeks for at least about 4 weeks;
   or,
e) administering the target drug at a dose of about 6.0 mg once a week for at least about 2 weeks, followed by
f) administering the target drug at a dose of about 6.0 mg once every two weeks for at least about 4 weeks;
   or,
e) administering the target drug at a dose of about 1.0 mg once a week for at least about 4 weeks;
   or,
e) administering the target drug at a dose of about 2.0 mg once a week for at least about 4 weeks;
   or,
e) administering the target drug at a dose of about 3.0 mg once a week for at least about 4 weeks;
   or,
e) administering the target drug at a dose of about 4.0 mg once a week for at least about 2 weeks;
   or,
e) administering the target drug at a dose of about 4.5 mg once a week for at least about 4 weeks;
   or,
e) administering the target drug at a dose of about 6.0 mg once a week for at least about 4 weeks;
   or,
e) administering the target drug at a dose of about 1.0 mg once a week for at least about 2 weeks, followed by
g) administering the target drug at a dose of about 2.0 mg once a week for at least about 2 weeks, followed by
h) administering the target drug at a dose of about 3.0 mg once a week for at least about 4 weeks.

In some embodiments, the second treatment period II-3-2 comprises:
e) administering the target drug at a dose of about 3.0 mg once a week for at least about 4 weeks, such as about 8 weeks, followed by
f) administering the target drug at a dose of about 3.0 mg once every two weeks for at least about 4 weeks, such as about 20 weeks;
   or,
e) administering the target drug at a dose of about 4.5 mg once a week for at least about 4 weeks, such as about 8 weeks, followed by
f) administering the target drug at a dose of about 4.5 mg once every two weeks for at least about 4 weeks, such as about 20 weeks;
   or,
e) administering the target drug at a dose of about 6.0 mg once a week for at least about 4 weeks, such as about 8 weeks, followed by
f) administering the target drug at a dose of about 6.0 mg once every two weeks for at least about 4 weeks, such as about 20 weeks;
   or,
e) administering the target drug at a dose of about 1.0 mg once a week for at least about 4 weeks, such as about 8 weeks;
   or,
e) administering the target drug at a dose of about 2.0 mg once a week for at least about 4 weeks, such as about 8 weeks, about 24 weeks, or about 28 weeks;
   or,
e) administering the target drug at a dose of about 3.0 mg once a week for at least about 4 weeks, such as about 8 week or about 28 weeks;
   or,
e) administering the target drug at a dose of about 4.0 mg once a week for at least about 4 weeks, such as about 24 weeks or about 28 weeks;
   or,
e) administering the target drug at a dose of about 4.5 mg once a week for at least about 4 weeks, such as about 8 week or about 28 weeks;
   or,
e) administering the target drug at a dose of about 6.0 mg once a week for at least about 4 weeks, such as about 24 weeks or about 28 weeks;
   or,
e) administering the target drug at a dose of about 1.0 mg once a week for at least about 4 weeks, such as about 8 weeks, followed by
g) administering the target drug at a dose of about 2.0 mg once a week for at least about 4 weeks, such as about 4 weeks, followed by
h) administering the target drug at a dose of about 3.0 mg once a week for at least about 4 weeks, such as about 16 weeks.

In some embodiments, the second treatment period II-1-3 comprises:
e) administering the target drug at a dose of about 0.5 mg to about 10.0 mg once a week for at least about 1 week, followed by
f) administering the target drug at a dose of about 0.5 mg to about 10.0 mg once every two weeks for at least about 2 weeks;
   or,
e) administering the target drug at a dose of about 0.5 mg to about 10.0 mg once a week for at least about 2 weeks;
   or,
e) administering the target drug at a dose of about 0.5 mg to about 10.0 mg once a week for at least about 1 week, followed by
g) administering the target drug at a dose of about 0.5 mg to about 10.0 mg once a week for at least about 1 week, followed by
h) administering the target drug at a dose of about 0.5 mg to about 10.0 mg once a week for at least about 2 weeks.

In some embodiments, the second treatment period II-2-3 comprises:
e) administering the target drug at a dose of about 0.5 mg to about 10.0 mg once a week for at least about 2 weeks, followed by
f) administering the target drug at a dose of about 0.5 mg to about 10.0 mg once every two weeks for at least about 4 weeks;
   or,
e) administering the target drug at a dose of about 0.5 mg to about 10.0 mg once a week for at least about 4 weeks;
   or,
e) administering the target drug at a dose of about 0.5 mg to about 10.0 mg once a week for at least about 2 weeks, followed by
g) administering the target drug at a dose of about 0.5 mg to about 10.0 mg once a week for at least about 2 weeks, followed by
h) administering the target drug at a dose of about 0.5 mg to about 10.0 mg once a week for at least about 4 weeks.

In some embodiments, the second treatment period II-3-3 comprises:
e) administering the target drug at a dose of about 0.5 mg to about 10.0 mg once a week for at least about 4 weeks, such as about 8 weeks, followed by
f) administering the target drug at a dose of about 0.5 mg to about 10.0 mg once every two weeks for at least about 4 weeks, such as about 20 weeks;
   or,
e) administering the target drug at a dose of about 0.5 mg to about 10.0 mg once a week for at least about 4 weeks, such as about 4 weeks, about 8 weeks, about 12 weeks, about 16 weeks, about 20 weeks, about 24 weeks, or about 28 weeks;
   or,
e) administering the target drug at a dose of about 0.5 mg to about 10.0 mg once a week for at least about 4 weeks, such as about 8 weeks, followed by
g) administering the target drug at a dose of about 0.5 mg to about 10.0 mg once a week for at least about 4 weeks, such as about 4 weeks, followed by
h) administering the target drug at a dose of about 0.5 mg to about 10.0 mg once a week for at least about 4 weeks, such as about 16 weeks.

### Dosing regimen II

In some embodiments, the dosing regimen comprises:
a) administering an initial dose of the target drug once a week for at least about 1 week, followed by
d) administering a maintenance dose of the target drug once a week for at least about 2 weeks, followed by
e) administering a maintenance dose of the target drug once a week for at least about 1 week, followed by
g) administering a seventh dose of the target drug once a week for at least about 1 week, followed by
h) administering an eighth dose of the target drug once a week for at least about 2 weeks;
   or,
a) administering an initial dose of the target drug once a week for at least about 1 week, followed by
b) administering a second dose of the target drug once a week for at least about 1 week, followed by
d) administering a maintenance dose of the target drug once a week for at least about 2 weeks, followed by
e) administering a maintenance dose of the target drug once a week for at least about 1 week, followed by
f) administering a maintenance dose of the target drug once every two weeks for at least about 2 weeks;
   or,
a) administering an initial dose of the target drug once a week for at least about 1 week, followed by
b) administering a second dose of the target drug once a week for at least about 1 week, followed by
d) administering a maintenance dose of the target drug once a week for at least about 2 weeks, followed by
e) administering a maintenance dose of the target drug once a week for at least about 2 weeks;
   or,
a) administering an initial dose of the target drug once a week for at least about 1 week, followed by
b) administering a second dose of the target drug once a week for at least about 1 week, followed by
c) administering a third dose of the target drug once a week for at least about 2 weeks, followed by
d) administering a maintenance dose of the target drug once a week for at least about 2 weeks, followed by
e) administering a maintenance dose of the target drug once a week for at least about 1 week, followed by
f) administering a maintenance dose of the target drug once every two weeks for at least about 2 weeks;
   or,

a) administering an initial dose of the target drug once a week for at least about 1 week, followed by
b) administering a second dose of the target drug once a week for at least about 1 week, followed by
c) administering a third dose of the target drug once a week for at least about 2 weeks, followed by
d) administering a maintenance dose of the target drug once a week for at least about 2 weeks, followed by
e) administering a maintenance dose of the target drug once a week for at least about 2 weeks;
   or,

a) administering an initial dose of the target drug once a week for at least about 1 week, followed by
d) administering a maintenance dose of the target drug once a week for at least about 2 weeks, followed by
e) administering a maintenance dose of the target drug once a week for at least about 1 week; or,
a) administering an initial dose of the target drug once a week for at least about 1 week, followed by
b) administering a second dose of the target drug once a week for at least about 1 week, followed by
c) administering a third dose of the target drug once a week for at least about 2 weeks, followed by
   administering a fourth dose of the target drug once a week for at least about 2 weeks, followed by
   administering a fifth dose of the target drug once a week for at least about 2 weeks, followed by
d) administering a maintenance dose of the target drug once a week for at least about 2 weeks, followed by
e) administering a maintenance dose of the target drug once a week for at least about 2 weeks.

In some embodiments, the dosing regimen comprises:
a) administering an initial dose of the target drug once a week for at least about 2 weeks, followed by
d) administering a maintenance dose of the target drug once a week for at least about 4 weeks, followed by
e) administering a maintenance dose of the target drug once a week for at least about 1 week, followed by
g) administering a seventh dose of the target drug once a week for at least about 1 week, followed by
h) administering an eighth dose of the target drug once a week for at least about 2 weeks;
   or,
a) administering an initial dose of the target drug once a week for at least about 1 week, followed by
b) administering a second dose of the target drug once a week for at least about 1 week, followed by
d) administering a maintenance dose of the target drug once a week for at least about 2 weeks, followed by
e) administering a maintenance dose of the target drug once a week for at least about 1 week, followed by
f) administering a maintenance dose of the target drug once every two weeks for at least about 2 weeks;
   or,
a) administering an initial dose of the target drug once a week for at least about 1 week, followed by
b) administering a second dose of the target drug once a week for at least about 1 week, followed by
d) administering a maintenance dose of the target drug once a week for at least about 2 weeks, followed by
e) administering a maintenance dose of the target drug once a week for at least about 2 weeks;
   or,
a) administering an initial dose of the target drug once a week for at least about 1 week, followed by
b) administering a second dose of the target drug once a week for at least about 1 week, followed by
c) administering a third dose of the target drug once a week for at least about 2 weeks, followed by
d) administering a maintenance dose of the target drug once a week for at least about 2 weeks, followed by
e) administering a maintenance dose of the target drug once a week for at least about 1 week, followed by
f) administering a maintenance dose of the target drug once every two weeks for at least about 2 weeks;
   or,
a) administering an initial dose of the target drug once a week for at least about 1 week, followed by
b) administering a second dose of the target drug once a week for at least about 1 week, followed by
c) administering a third dose of the target drug once a week for at least about 2 weeks, followed by
d) administering a maintenance dose of the target drug once a week for at least about 2 weeks, followed by
e) administering a maintenance dose of the target drug once a week for at least about 2 weeks;
   or,
a) administering an initial dose of the target drug once a week for at least about 1 week, followed by
d) administering a maintenance dose of the target drug once a week for at least about 2 weeks, followed by
e) administering a maintenance dose of the target drug once a week for at least about 2 weeks; or,
a) administering an initial dose of the target drug once a week for at least about 1 week, followed by
b) administering a second dose of the target drug once a week for at least about 1 week, followed by
c) administering a third dose of the target drug once a week for at least about 1 week, followed by
   administering a fourth dose of the target drug once a week for at least about 1 week, followed by
   administering a fifth dose of the target drug once a week for at least about 1 week, followed by
d) administering a maintenance dose of the target drug once a week for at least about 2 weeks, followed by
e) administering a maintenance dose of the target drug once a week for at least about 2 weeks.

In some embodiments, the dosing regimen comprises:
a) administering an initial dose of the target drug once a week for at least about 2 weeks, followed by
d) administering a maintenance dose of the target drug once a week for at least about 4 weeks, followed by
e) administering a maintenance dose of the target drug once a week for at least about 2 weeks, followed by
g) administering a seventh dose of the target drug once a week for at least about 2 weeks, followed by
h) administering an eighth dose of the target drug once a week for at least about 4 weeks;
   or,
a) administering an initial dose of the target drug once a week for at least about 2 weeks, followed by
b) administering a second dose of the target drug once a week for at least about 2 weeks, followed by
d) administering a maintenance dose of the target drug once a week for at least about 4 weeks, followed by
e) administering a maintenance dose of the target drug once a week for at least about 2 weeks, followed by
f) administering a maintenance dose of the target drug once every two weeks for at least about 4 weeks;
   or,
a) administering an initial dose of the target drug once a week for at least about 2 weeks, followed by
b) administering a second dose of the target drug once a week for at least about 2 weeks, followed by
d) administering a maintenance dose of the target drug once a week for at least about 4 weeks, followed by
e) administering a maintenance dose of the target drug once a week for at least about 4 weeks;
   or,
a) administering an initial dose of the target drug once a week for at least about 2 weeks, followed by
b) administering a second dose of the target drug once a week for at least about 2 weeks, followed by
c) administering a third dose of the target drug once a week for at least about 2 weeks, followed by
d) administering a maintenance dose of the target drug once a week for at least about 4 weeks, followed by
e) administering a maintenance dose of the target drug once a week for at least about 2 weeks, followed by
f) administering a maintenance dose of the target drug once every two weeks for at least about 4 weeks;
   or,
a) administering an initial dose of the target drug once a week for at least about 2 weeks, followed by
b) administering a second dose of the target drug once a week for at least about 2 weeks, followed by
c) administering a third dose of the target drug once a week for at least about 2 weeks, followed by
d) administering a maintenance dose of the target drug once a week for at least about 4 weeks, followed by
e) administering a maintenance dose of the target drug once a week for at least about 4 weeks;
   or,
a) administering an initial dose of the target drug once a week for at least about 2 weeks, followed by
d) administering a maintenance dose of the target drug once a week for at least about 2 weeks, followed by
e) administering a maintenance dose of the target drug once a week for at least about 4 weeks;
   or,
a) administering an initial dose of the target drug once a week for at least about 2 weeks, followed by
b) administering a second dose of the target drug once a week for at least about 2 weeks, followed by
c) administering a third dose of the target drug once a week for at least about 4 weeks, followed by
   administering a fourth dose of the target drug once a week for at least about 4 weeks, followed by
   administering a fifth dose of the target drug once a week for at least about 4 weeks, followed by
d) administering a maintenance dose of the target drug once a week for at least about 4 weeks, followed by
e) administering a maintenance dose of the target drug once a week for at least about 4 weeks.

In some embodiments, the dosing regimen comprises:
a) administering an initial dose of the target drug once a week for at least about 4 weeks, such as about 4 weeks, followed by
d) administering a maintenance dose of the target drug once a week for at least about 4 weeks, such as about 20 weeks, followed by
e) administering a maintenance dose of the target drug once a week for at least about 4 weeks, such as about 8 weeks, followed by
g) administering a seventh dose of the target drug once a week for at least about 4 weeks, such as about 4 weeks, followed by
h) administering an eighth dose of the target drug once a week for at least about 4 weeks, such as about 16 weeks;
   or,
a) administering an initial dose of the target drug once a week for at least about 4 weeks, such as about 4 weeks, followed by
b) administering a second dose of the target drug once a week for at least about 4 weeks, such as about 4 weeks, followed by
d) administering a maintenance dose of the target drug once a week for at least about 4 weeks, such as about 16 weeks, followed by
e) administering a maintenance dose of the target drug once a week for at least about 4 weeks, such as about 8 weeks, followed by
f) administering a maintenance dose of the target drug once every two weeks for at least about 4 weeks, such as about 20 weeks;
   or,
a) administering an initial dose of the target drug once a week for at least about 4 weeks, such as about 4 weeks, followed by
b) administering a second dose of the target drug once a week for at least about 4 weeks, such as about 4 weeks, followed by
d) administering a maintenance dose of the target drug once a week for at least about 4 weeks, such as about 16 weeks, followed by
e) administering a maintenance dose of the target drug once a week for at least about 4 weeks, such as about 8 weeks or about 28 weeks;
   or,
a) administering an initial dose of the target drug once a week for at least about 4 weeks, such as about 4 weeks, followed by
b) administering a second dose of the target drug once a week for at least about 4 weeks, such as about 4 weeks, followed by
c) administering a third dose of the target drug once a week for at least about 4 weeks, such as about 4 weeks, followed by
d) administering a maintenance dose of the target drug once a week for at least about 4 weeks, such as about 12 weeks, followed by
e) administering a maintenance dose of the target drug once a week for at least about 4 weeks, such as about 8 weeks, followed by
f) administering a maintenance dose of the target drug once every two weeks for at least about 4 weeks, such as about 20 weeks;
   or,
a) administering an initial dose of the target drug once a week for at least about 4 weeks, such as about 4 weeks, followed by
b) administering a second dose of the target drug once a week for at least about 4 weeks, such as about 4 weeks, followed by
c) administering a third dose of the target drug once a week for at least about 4 weeks, such as about 4 weeks, followed by
d) administering a maintenance dose of the target drug once a week for at least about 4 weeks, such as about 12 weeks, followed by
e) administering a maintenance dose of the target drug once a week for at least about 4 weeks, such as about 24 weeks or about 28 weeks;
   or,
a) administering an initial dose of the target drug once a week for at least about 4 weeks, such as about 4 weeks, followed by
d) administering a maintenance dose of the target drug once a week for at least about 4 weeks, such as about 20 weeks, followed by
e) administering a maintenance dose of the target drug once a week for at least about 4 weeks, such as about 8 weeks, about 24 weeks or about 28 weeks;
   or,
a) administering an initial dose of the target drug once a week for at least about 4 weeks, followed by
b) administering a second dose of the target drug once a week for at least about 4 weeks, followed by
c) administering a third dose of the target drug once a week for at least about 4 weeks, followed by
   administering a fourth dose of the target drug once a week for at least about 4 weeks, followed by
   administering a fifth dose of the target drug once a week for at least about 4 weeks, followed by
d) administering a maintenance dose of the target drug once a week for at least about 4 weeks, followed by
e) administering a maintenance dose of the target drug once a week for at least about 4 weeks, such as about 24 weeks.

In some embodiments, the dosing regimen comprises:
a) administering the target drug at a dose of about 0.5 mg to about 1.5 mg once a week for at least about 1 week, followed by
d) administering the target drug at a dose of about 1.0 mg to about 6.0 mg once a week for at least about 1 week, followed by
e) administering the target drug at a dose of about 1.0 mg to about 6.0 mg once a week for at least about 1 week, followed by
g) administering the target drug at a dose of about 2.0 mg to about 3.0 mg once a week for at least about 1 week, followed by
h) administering the target drug at a dose of about 2.0 mg to about 3.0 mg once a week for at least about 2 weeks;
   or,
a) administering the target drug at a dose of about 0.5 mg to about 1.5 mg once a week for at least about 1 week, followed by
b) administering the target drug at a dose of about 2.0 mg to about 3.0 mg once a week for at least about 1 week, followed by
d) administering the target drug at a dose of about 1.0 mg to about 6.0 mg once a week for at least about 2 weeks, followed by
e) administering the target drug at a dose of about 1.0 mg to about 6.0 mg once a week for at least about 1 week, followed by
f) administering the target drug at a dose of about 1.0 mg to about 6.0 mg once every two weeks for at least about 2 weeks;
   or,
a) administering the target drug at a dose of about 0.5 mg to about 1.5 mg once a week for at least about 1 week, followed by
b) administering the target drug at a dose of about 2.0 mg to about 3.0 mg once a week for at least about 1 week, followed by
d) administering the target drug at a dose of about 1.0 mg to about 6.0 mg once a week for at least about 2 weeks, followed by
e) administering the target drug at a dose of about 1.0 mg to about 6.0 mg once a week for at least about 2 weeks;
   or,
a) administering the target drug at a dose of about 0.5 mg to about 1.5 mg once a week for at least about 1 week, followed by
b) administering the target drug at a dose of about 2.0 mg to about 3.0 mg once a week for at least about 1 week, followed by
c) administering the target drug at a dose of about 3.0 mg to about 4.5 mg once a week for at least about 2 weeks, followed by
d) administering the target drug at a dose of about 1.0 mg to about 6.0 mg once a week for at least about 2 weeks, followed by
e) administering the target drug at a dose of about 1.0 mg to about 6.0 mg once a week for at least about 1 week, followed by
f) administering the target drug at a dose of about 1.0 mg to about 6.0 mg once every two weeks for at least about 2 weeks;
   or,
a) administering the target drug at a dose of about 0.5 mg to about 1.5 mg once a week for at least about 1 week, followed by
b) administering the target drug at a dose of about 2.0 mg to about 3.0 mg once a week for at least about 1 week, followed by
c) administering the target drug at a dose of about 3.0 mg to about 4.5 mg once a week for at least about 2 weeks, followed by
d) administering the target drug at a dose of about 1.0 mg to about 6.0 mg once a week for at least about 2 weeks, followed by
e) administering the target drug at a dose of about 1.0 mg to about 6.0 mg once a week for at least about 2 weeks;
   or,
a) administering the target drug at a dose of about 0.5 mg to about 1.5 mg once a week for at least about 1 week, followed by
d) administering the target drug at a dose of about 1.0 mg to about 6.0 mg once a week for at least about 2 weeks, followed by
e) administering the target drug at a dose of about 1.0 mg to about 6.0 mg once a week for at least about 2 weeks;
   or,
a) administering the target drug at a dose of about 0.5 mg to about 1.5 mg once a week for at least about 1 week, followed by
b) administering the target drug at a dose of about 2.0 mg to about 3.0 mg once a week for at least about 1 week, followed by
c) administering the target drug at a dose of about 3.0 mg to about 4.5 mg once a week for at least about 2 weeks, followed by
   administering the target drug at a dose of about 4.0 mg to about 5.0 mg once a week for at least about 2 weeks, followed by
   administering the target drug at a dose of about 5.0 mg to about 6.0 mg once a week for at least about 2 weeks, followed by
d) administering the target drug at a dose of about 1.0 mg to about 6.0 mg once a week for at least about 2 weeks, followed by
e) administering the target drug at a dose of about 1.0 mg to about 6.0 mg once a week for at least about 2 weeks.

In some embodiments, the dosing regimen comprises:
a) administering the target drug at a dose of about 0.5 mg to about 1.5 mg once a week for at least about 2 weeks, followed by
d) administering the target drug at a dose of about 1.0 mg to about 6.0 mg once a week for at least about 4 weeks, followed by
e) administering the target drug at a dose of about 1.0 mg to about 6.0 mg once a week for at least about 2 weeks, followed by
g) administering the target drug at a dose of about 2.0 mg to about 3.0 mg once a week for at least about 2 weeks, followed by
h) administering the target drug at a dose of about 2.0 mg to about 3.0 mg once a week for at least about 4 weeks;
   or,
a) administering the target drug at a dose of about 0.5 mg to about 1.5 mg once a week for at least about 2 weeks, followed by
b) administering the target drug at a dose of about 2.0 mg to about 3.0 mg once a week for at least about 2 weeks, followed by
d) administering the target drug at a dose of about 1.0 mg to about 6.0 mg once a week for at least about 4 weeks, followed by
e) administering the target drug at a dose of about 1.0 mg to about 6.0 mg once a week for at least about 2 weeks, followed by
f) administering the target drug at a dose of about 1.0 mg to about 6.0 mg once every two weeks for at least about 4 weeks;
   or,
a) administering the target drug at a dose of about 0.5 mg to about 1.5 mg once a week for at least about 2 weeks, followed by
b) administering the target drug at a dose of about 2.0 mg to about 3.0 mg once a week for at least about 2 weeks, followed by
d) administering the target drug at a dose of about 1.0 mg to about 6.0 mg once a week for at least about 4 weeks, followed by
e) administering the target drug at a dose of about 1.0 mg to about 6.0 mg once a week for at least about 4 weeks;
   or,
a) administering the target drug at a dose of about 0.5 mg to about 1.5 mg once a week for at least about 2 weeks, followed by
b) administering the target drug at a dose of about 2.0 mg to about 3.0 mg once a week for at least about 2 weeks, followed by
c) administering the target drug at a dose of about 3.0 mg to about 4.5 mg once a week for at least about 2 weeks, followed by
d) administering the target drug at a dose of about 1.0 mg to about 6.0 mg once a week for at least about 4 weeks, followed by
e) administering the target drug at a dose of about 1.0 mg to about 6.0 mg once a week for at least about 2 weeks, followed by
f) administering the target drug at a dose of about 1.0 mg to about 6.0 mg once every two weeks for at least about 4 weeks;
   or,
a) administering the target drug at a dose of about 0.5 mg to about 1.5 mg once a week for at least about 2 weeks, followed by
b) administering the target drug at a dose of about 2.0 mg to about 3.0 mg once a week for at least about 2 weeks, followed by
c) administering the target drug at a dose of about 3.0 mg to about 4.5 mg once a week for at least about 2 weeks, followed by
d) administering the target drug at a dose of about 1.0 mg to about 6.0 mg once a week for at least about 4 weeks, followed by
e) administering the target drug at a dose of about 1.0 mg to about 6.0 mg once a week for at least about 4 weeks;
   or,
a) administering the target drug at a dose of about 0.5 mg to about 1.5 mg once a week for at least about 2 weeks, followed by
d) administering the target drug at a dose of about 1.0 mg to about 6.0 mg once a week for at least about 4 weeks, followed by
e) administering the target drug at a dose of about 1.0 mg to about 6.0 mg once a week for at least about 4 weeks;
   or,
a) administering the target drug at a dose of about 0.5 mg to about 1.5 mg once a week for at least about 2 weeks, followed by
b) administering the target drug at a dose of about 2.0 mg to about 3.0 mg once a week for at least about 2 weeks, followed by
c) administering the target drug at a dose of about 3.0 mg to about 4.5 mg once a week for at least about 4 weeks, followed by
   administering the target drug at a dose of about 4.0 mg to about 5.0 mg once a week for at least about 4 weeks, followed by
   administering the target drug at a dose of about 5.0 mg to about 6.0 mg once a week for at least about 4 weeks, followed by
d) administering the target drug at a dose of about 1.0 mg to about 6.0 mg once a week for at least about 4 weeks, followed by
e) administering the target drug at a dose of about 1.0 mg to about 6.0 mg once a week for at least about 4 weeks.

In some embodiments, the dosing regimen comprises:
a) administering the target drug at a dose of about 0.5 mg to about 1.5 mg once a week for at least about 4 weeks, followed by
d) administering the target drug at a dose of about 1.0 mg to about 6.0 mg once a week for at least about 4 weeks, such as about 20 weeks, followed by
e) administering the target drug at a dose of about 1.0 mg to about 6.0 mg once a week for at least about 4 weeks, such as about 8 weeks, followed by
g) administering the target drug at a dose of about 2.0 mg to about 3.0 mg once a week for at least about 4 weeks, followed by
h) administering the target drug at a dose of about 2.0 mg to about 3.0 mg once a week for at least about 4 weeks, such as about 16 weeks;
   or,
a) administering the target drug at a dose of about 0.5 mg to about 1.5 mg once a week for at least about 4 weeks, followed by
b) administering the target drug at a dose of about 2.0 mg to about 3.0 mg once a week for at least about 4 weeks, followed by
d) administering the target drug at a dose of about 1.0 mg to about 6.0 mg once a week for at least about 4 weeks, such as about 16 weeks, followed by
e) administering the target drug at a dose of about 1.0 mg to about 6.0 mg once a week for at least about 4 weeks, such as about 8 weeks, followed by
f) administering the target drug at a dose of about 1.0 mg to about 6.0 mg once every two weeks for at least about 4 weeks, such as about 20 weeks;
   or,
a) administering the target drug at a dose of about 0.5 mg to about 1.5 mg once a week for at least about 4 weeks, followed by
b) administering the target drug at a dose of about 2.0 mg to about 3.0 mg once a week for at least about 4 weeks, followed by
c) administering the target drug at a dose of about 3.0 mg to about 4.5 mg once a week for at least about 4 weeks, followed by
d) administering the target drug at a dose of about 1.0 mg to about 6.0 mg once a week for at least about 4 weeks, such as about 12 weeks, followed by
e) administering the target drug at a dose of about 1.0 mg to about 6.0 mg once a week for at least about 4 weeks, such as about 8 weeks, followed by
f) administering the target drug at a dose of about 1.0 mg to about 6.0 mg once every two weeks for at least about 4 weeks, such as about 20 weeks;
   or,
a) administering the target drug at a dose of about 0.5 mg to about 1.5 mg once a week for at least about 4 weeks, followed by
b) administering the target drug at a dose of about 2.0 mg to about 3.0 mg once a week for at least about 4 weeks, followed by
c) administering the target drug at a dose of about 3.0 mg to about 4.5 mg once a week for at least about 4 weeks, followed by
d) administering the target drug at a dose of about 1.0 mg to about 6.0 mg once a week for at least about 4 weeks, such as about 12 weeks, followed by
e) administering the target drug at a dose of about 1.0 mg to about 6.0 mg once a week for at least about 4 weeks, such as about 24 weeks or about 28 weeks;
   or,
a) administering the target drug at a dose of about 0.5 mg to about 1.5 mg once a week for at least about 4 weeks, followed by
d) administering the target drug at a dose of about 1.0 mg to about 6.0 mg once a week for at least about 4 weeks, such as about 16 weeks or about 20 weeks, followed by
e) administering the target drug at a dose of about 1.0 mg to about 6.0 mg once a week for at least about 4 weeks, such as about 8 weeks, about 24 weeks or about 28 weeks;
   or,
a) administering the target drug at a dose of about 0.5 mg to about 1.5 mg once a week for at least about 4 weeks, followed by
b) administering the target drug at a dose of about 2.0 mg to about 3.0 mg once a week for at least about 4 weeks, followed by
d) administering the target drug at a dose of about 1.0 mg to about 6.0 mg once a week for at least about 4 weeks, such as about 16 weeks, followed by
e) administering the target drug at a dose of about 1.0 mg to about 6.0 mg once a week for at least about 4 weeks, such as about 8 weeks or 28 weeks;
   or,
a) administering the target drug at a dose of about 0.5 mg to about 1.5 mg once a week for at least about 4 weeks, followed by
b) administering the target drug at a dose of about 2.0 mg to about 3.0 mg once a week for at least about 4 weeks, followed by
c) administering the target drug at a dose of about 3.0 mg to about 4.5 mg once a week for at least about 4 weeks, followed by
   administering the target drug at a dose of about 4.0 mg to about 5.0 mg once a week for at least about 4 weeks, followed by
   administering the target drug at a dose of about 5.0 mg to about 6.0 mg once a week for at least about 4 weeks, followed by
d) administering the target drug at a dose of about 1.0 mg to about 6.0 mg once a week for at least about 4 weeks, followed by
e) administering the target drug at a dose of about 1.0 mg to about 6.0 mg once a week for at least about 4 weeks, such as about 24 weeks.

In some embodiments, the dosing regimen comprises:
a) administering the target drug at a dose of about 0.5 mg once a week for at least about 1 week, followed by
d) administering the target drug at a dose of about 1.0 mg once a week for at least about 1 week, followed by
e) administering the target drug at a dose of about 1.0 mg once a week for at least about 1 week, followed by
g) administering the target drug at a dose of about 2.0 mg once a week for at least about 1 week, followed by
h) administering the target drug at a dose of about 3.0 mg once a week for at least about 2 weeks;
   or,
a) administering the target drug at a dose of about 1.0 mg once a week for at least about 1 week, followed by
b) administering the target drug at a dose of about 2.0 mg once a week for at least about 1 week, followed by
d) administering the target drug at a dose of about 3.0 mg once a week for at least about 2 weeks, followed by
e) administering the target drug at a dose of about 3.0 mg once a week for at least about 1 week, followed by
f) administering the target drug at a dose of about 3.0 mg once every two weeks for at least about 2 weeks;
   or,
a) administering the target drug at a dose of about 1.0 mg once a week for at least about 1 week, followed by
b) administering the target drug at a dose of about 2.0 mg once a week for at least about 1 week, followed by
d) administering the target drug at a dose of about 3.0 mg once a week for at least about 2 weeks, followed by
e) administering the target drug at a dose of about 3.0 mg once a week for at least about 2 weeks;
   or,
a) administering the target drug at a dose of about 1.0 mg once a week for at least about 1 week, followed by
b) administering the target drug at a dose of about 2.0 mg once a week for at least about 1 week, followed by
c) administering the target drug at a dose of about 3.0 mg once a week for at least about 2 weeks, followed by
d) administering the target drug at a dose of about 4.5 mg once a week for at least about 2 weeks, followed by
e) administering the target drug at a dose of about 4.5 mg once a week for at least about 1 week, followed by
f) administering the target drug at a dose of about 4.5 mg once every two weeks for at least about 2 weeks;
   or,
a) administering the target drug at a dose of about 1.0 mg once a week for at least about 1 week, followed by
b) administering the target drug at a dose of about 2.0 mg once a week for at least about 1 week, followed by
c) administering the target drug at a dose of about 3.0 mg once a week for at least about 2 weeks, followed by
d) administering the target drug at a dose of about 4.5 mg once a week for at least about 2 weeks, followed by
e) administering the target drug at a dose of about 4.5 mg once a week for at least about 2 weeks;
   or,
a) administering the target drug at a dose of about 1.0 mg once a week for at least about 1 week, followed by
b) administering the target drug at a dose of about 2.0 mg once a week for at least about 1 week, followed by
c) administering the target drug at a dose of about 3.0 mg once a week for at least about 2 weeks, followed by
d) administering the target drug at a dose of about 4.0 mg once a week for at least about 2 weeks, followed by
e) administering the target drug at a dose of about 4.0 mg once a week for at least about 2 weeks;
   or,
a) administering the target drug at a dose of about 1.5 mg once a week for at least about 1 week, followed by
b) administering the target drug at a dose of about 3.0 mg once a week for at least about 1 week, followed by
c) administering the target drug at a dose of about 4.5 mg once a week for at least about 2 weeks, followed by
d) administering the target drug at a dose of about 6.0 mg once a week for at least about 2 weeks, followed by
e) administering the target drug at a dose of about 6.0 mg once a week for at least about 1 week, followed by
f) administering the target drug at a dose of about 6.0 mg once every two weeks for at least about 2 weeks;
   or,
a) administering the target drug at a dose of about 1.5 mg once a week for at least about 1 week, followed by
b) administering the target drug at a dose of about 3.0 mg once a week for at least about 1 week, followed by
c) administering the target drug at a dose of about 4.5 mg once a week for at least about 2 weeks, followed by
d) administering the target drug at a dose of about 6.0 mg once a week for at least about 2 weeks, followed by
e) administering the target drug at a dose of about 6.0 mg once a week for at least about 2 weeks;
   or
a) administering the target drug at a dose of about 1.0 mg once a week for at least about 1 week, followed by
d) administering the target drug at a dose of about 1.0 mg once a week for at least about 2 weeks, followed by
e) administering the target drug at a dose of about 1.0 mg once a week for at least about 2 weeks, followed by;
   or
a) administering the target drug at a dose of about 1.0 mg once a week for at least about 1 week, followed by
d) administering the target drug at a dose of about 2.0 mg once a week for at least about 2 weeks, followed by
e) administering the target drug at a dose of about 2.0 mg once a week for at least about 2 weeks, followed by;
   or
a) administering the target drug at a dose of about 1.5 mg once a week for at least about 1 week, followed by
b) administering the target drug at a dose of about 3.0 mg once a week for at least about 1 week, followed by
d) administering the target drug at a dose of about 4.5 mg once a week for at least about 2 weeks, followed by
e) administering the target drug at a dose of about 4.5 mg once a week for at least about 2 weeks, followed by;
   or,
a) administering the target drug at a dose of about 1.0 mg once a week for at least about 1 week, followed by
b) administering the target drug at a dose of about 2.0 mg once a week for at least about 1 week, followed by
c) administering the target drug at a dose of about 3.0 mg once a week for at least about 2 weeks, followed by
   administering the target drug at a dose of about 4.0 mg once a week for at least about 2 weeks, followed by
   administering the target drug at a dose of about 5.0 mg once a week for at least about 2 weeks, followed by
d) administering the target drug at a dose of about 6.0 mg once a week for at least about 2 weeks, followed by
e) administering the target drug at a dose of about 6.0 mg once a week for at least about 2 weeks.

In some embodiments, the dosing regimen comprises:
a) administering the target drug at a dose of about 0.5 mg once a week for at least about 2 weeks, followed by
d) administering the target drug at a dose of about 1.0 mg once a week for at least about 4 weeks, followed by
e) administering the target drug at a dose of about 1.0 mg once a week for at least about 2 weeks, followed by
g) administering the target drug at a dose of about 2.0 mg once a week for at least about 2 weeks, followed by
h) administering the target drug at a dose of about 3.0 mg once a week for at least about 4 weeks;
   or,
a) administering the target drug at a dose of about 1.0 mg once a week for at least about 2 weeks, followed by
b) administering the target drug at a dose of about 2.0 mg once a week for at least about 2 weeks, followed by
d) administering the target drug at a dose of about 3.0 mg once a week for at least about 4 weeks, followed by
e) administering the target drug at a dose of about 3.0 mg once a week for at least about 2 weeks, followed by
f) administering the target drug at a dose of about 3.0 mg once every two weeks for at least about 4 weeks;
   or,
a) administering the target drug at a dose of about 1.0 mg once a week for at least about 2 weeks, followed by
b) administering the target drug at a dose of about 2.0 mg once a week for at least about 2 weeks, followed by
d) administering the target drug at a dose of about 3.0 mg once a week for at least about 4 weeks, followed by
e) administering the target drug at a dose of about 3.0 mg once a week for at least about 4 weeks;
   or,
a) administering the target drug at a dose of about 1.0 mg once a week for at least about 2 weeks, followed by
b) administering the target drug at a dose of about 2.0 mg once a week for at least about 2 weeks, followed by
c) administering the target drug at a dose of about 3.0 mg once a week for at least about 2 weeks, followed by
d) administering the target drug at a dose of about 4.5 mg once a week for at least about 4 weeks, followed by
e) administering the target drug at a dose of about 4.5 mg once a week for at least about 2 weeks, followed by
f) administering the target drug at a dose of about 4.5 mg once every two weeks for at least about 4 weeks;
   or,
a) administering the target drug at a dose of about 1.0 mg once a week for at least about 2 weeks, followed by
b) administering the target drug at a dose of about 2.0 mg once a week for at least about 2 weeks, followed by
c) administering the target drug at a dose of about 3.0 mg once a week for at least about 2 weeks, followed by
d) administering the target drug at a dose of about 4.5 mg once a week for at least about 4 weeks, followed by
e) administering the target drug at a dose of about 4.5 mg once a week for at least about 4 weeks;
   or,
a) administering the target drug at a dose of about 1.0 mg once a week for at least about 2 weeks, followed by
b) administering the target drug at a dose of about 2.0 mg once a week for at least about 2 weeks, followed by
c) administering the target drug at a dose of about 3.0 mg once a week for at least about 2 weeks, followed by
d) administering the target drug at a dose of about 4.0 mg once a week for at least about 4 weeks, followed by
e) administering the target drug at a dose of about 4.0 mg once a week for at least about 4 weeks;
   or,
a) administering the target drug at a dose of about 1.5 mg once a week for at least about 2 weeks, followed by
b) administering the target drug at a dose of about 3.0 mg once a week for at least about 2 weeks, followed by
c) administering the target drug at a dose of about 4.5 mg once a week for at least about 2 weeks, followed by
d) administering the target drug at a dose of about 6.0 mg once a week for at least about 4 weeks, followed by
e) administering the target drug at a dose of about 6.0 mg once a week for at least about 2 weeks, followed by
f) administering the target drug at a dose of about 6.0 mg once every two weeks for at least about 4 weeks;
   or,
a) administering the target drug at a dose of about 1.5 mg once a week for at least about 2 weeks, followed by
b) administering the target drug at a dose of about 3.0 mg once a week for at least about 2 weeks, followed by
c) administering the target drug at a dose of about 4.5 mg once a week for at least about 2 weeks, followed by
d) administering the target drug at a dose of about 6.0 mg once a week for at least about 4 weeks, followed by
e) administering the target drug at a dose of about 6.0 mg once a week for at least about 4 weeks;
   or
a) administering the target drug at a dose of about 1.0 mg once a week for at least about 2 weeks, followed by
d) administering the target drug at a dose of about 1.0 mg once a week for at least about 4 weeks, followed by
e) administering the target drug at a dose of about 1.0 mg once a week for at least about 4 weeks, followed by;
   or
a) administering the target drug at a dose of about 1.0 mg once a week for at least about 2 weeks, followed by
d) administering the target drug at a dose of about 2.0 mg once a week for at least about 4 weeks, followed by
e) administering the target drug at a dose of about 2.0 mg once a week for at least about 4 weeks, followed by;
   or
a) administering the target drug at a dose of about 1.5 mg once a week for at least about 2 weeks, followed by
b) administering the target drug at a dose of about 3.0 mg once a week for at least about 2 weeks, followed by
d) administering the target drug at a dose of about 4.5 mg once a week for at least about 4 weeks, followed by
e) administering the target drug at a dose of about 4.5 mg once a week for at least about 4 weeks, followed by;
   or,
a) administering the target drug at a dose of about 1.0 mg once a week for at least about 2 weeks, followed by
b) administering the target drug at a dose of about 2.0 mg once a week for at least about 2 weeks, followed by
c) administering the target drug at a dose of about 3.0 mg once a week for at least about 4 weeks, followed by
   administering the target drug at a dose of about 4.0 mg once a week for at least about 4 weeks, followed by
   administering the target drug at a dose of about 5.0 mg once a week for at least about 4 weeks, followed by
d) administering the target drug at a dose of about 6.0 mg once a week for at least about 4 weeks, followed by
e) administering the target drug at a dose of about 6.0 mg once a week for at least about 4 weeks.

In some embodiments, the dosing regimen comprises:
a) administering the target drug at a dose of about 0.5 mg once a week for at least about 4 weeks, followed by
d) administering the target drug at a dose of about 1.0 mg once a week for at least about 4 weeks, such as about 20 weeks, followed by
e) administering the target drug at a dose of about 1.0 mg once a week for at least about 4 weeks, such as about 8 weeks, followed by
g) administering the target drug at a dose of about 2.0 mg once a week for at least about 4 weeks, followed by
h) administering the target drug at a dose of about 3.0 mg once a week for at least about 4 weeks, such as about 16 weeks;
   or,
a) administering the target drug at a dose of about 1.0 mg once a week for at least about 4 weeks, followed by
b) administering the target drug at a dose of about 2.0 mg once a week for at least about 4 weeks, followed by
d) administering the target drug at a dose of about 3.0 mg once a week for at least about 4 weeks, such as about 16 weeks, followed by
e) administering the target drug at a dose of about 3.0 mg once a week for at least about 4 weeks, such as about 8 weeks, followed by
f) administering the target drug at a dose of about 3.0 mg once every two weeks for at least about 4 weeks, such as about 20 weeks;
   or,
a) administering the target drug at a dose of about 1.0 mg once a week for at least about 4 weeks, followed by
b) administering the target drug at a dose of about 2.0 mg once a week for at least about 4 weeks, followed by
d) administering the target drug at a dose of about 3.0 mg once a week for at least about 4 weeks, such as about 16 weeks, followed by
e) administering the target drug at a dose of about 3.0 mg once a week for at least about 4 weeks, such as about 8 week or about 28 weeks;
   or,
a) administering the target drug at a dose of about 1.0 mg once a week for at least about 4 weeks, followed by
b) administering the target drug at a dose of about 2.0 mg once a week for at least about 4 weeks, followed by
c) administering the target drug at a dose of about 3.0 mg once a week for at least about 4 weeks, followed by
d) administering the target drug at a dose of about 4.5 mg once a week for at least about 4 weeks, such as about 12 weeks, followed by
e) administering the target drug at a dose of about 4.5 mg once a week for at least about 4 weeks, such as about 8 weeks, followed by
f) administering the target drug at a dose of about 4.5 mg once every two weeks for at least about 4 weeks, such as at least about 20 weeks;
   or,
a) administering the target drug at a dose of about 1.0 mg once a week for at least about 4 weeks, followed by
b) administering the target drug at a dose of about 2.0 mg once a week for at least about 4 weeks, followed by
c) administering the target drug at a dose of about 3.0 mg once a week for at least about 4 weeks, followed by
d) administering the target drug at a dose of about 4.5 mg once a week for at least about 4 weeks, such as about 12 weeks, followed by
e) administering the target drug at a dose of about 4.5 mg once a week for at least about 4 weeks, such as about 28 weeks;
   or,
a) administering the target drug at a dose of about 1.0 mg once a week for at least about 4 weeks, followed by
b) administering the target drug at a dose of about 2.0 mg once a week for at least about 4 weeks, followed by
c) administering the target drug at a dose of about 3.0 mg once a week for at least about 4 weeks, followed by
d) administering the target drug at a dose of about 4.0 mg once a week for at least about 4 weeks, such as about 12 weeks, followed by
e) administering the target drug at a dose of about 4.0 mg once a week for at least about 4 weeks, such as about 24 or about 28 weeks;
   or,
a) administering the target drug at a dose of about 1.5 mg once a week for at least about 4 weeks, followed by
b) administering the target drug at a dose of about 3.0 mg once a week for at least about 4 weeks, followed by
c) administering the target drug at a dose of about 4.5 mg once a week for at least about 4 weeks, followed by
d) administering the target drug at a dose of about 6.0 mg once a week for at least about 4 weeks, such as about 12 weeks, followed by
e) administering the target drug at a dose of about 6.0 mg once a week for at least about 4 weeks, such as about 8 weeks, followed by
f) administering the target drug at a dose of about 6.0 mg once every two weeks for at least about 4 weeks, such as about 20 weeks;
   or,
a) administering the target drug at a dose of about 1.5 mg once a week for at least about 4 weeks, followed by
b) administering the target drug at a dose of about 3.0 mg once a week for at least about 4 weeks, followed by
c) administering the target drug at a dose of about 4.5 mg once a week for at least about 4 weeks, followed by
d) administering the target drug at a dose of about 6.0 mg once a week for at least about 4 weeks, such as about 12 weeks, followed by
e) administering the target drug at a dose of about 6.0 mg once a week for at least about 4 weeks, such as about 28 weeks;
   or
a) administering the target drug at a dose of about 1.0 mg once a week for at least about 4 weeks, followed by
d) administering the target drug at a dose of about 1.0 mg once a week for at least about 4 weeks, such as about 20 weeks, followed by
e) administering the target drug at a dose of about 1.0 mg once a week for at least about 4 weeks, such as about 8 weeks, followed by;
   or
a) administering the target drug at a dose of about 1.0 mg once a week for at least about 4 weeks, followed by
d) administering the target drug at a dose of about 2.0 mg once a week for at least about 4 weeks, such as about 20 weeks, followed by
e) administering the target drug at a dose of about 2.0 mg once a week for at least about 4 weeks, such as about 8 weeks, about 24 weeks, or about 28 weeks;
   or
a) administering the target drug at a dose of about 1.5 mg once a week for at least about 4 weeks, followed by
b) administering the target drug at a dose of about 3.0 mg once a week for at least about 4 weeks, followed by
d) administering the target drug at a dose of about 4.5 mg once a week for at least about 4 weeks, such as about 16 weeks, followed by
e) administering the target drug at a dose of about 4.5 mg once a week for at least about 4 weeks, such as about 8 weeks;
   or,
a) administering the target drug at a dose of about 1.0 mg once a week for at least about 4 weeks, followed by
b) administering the target drug at a dose of about 2.0 mg once a week for at least about 4 weeks, followed by
c) administering the target drug at a dose of about 3.0 mg once a week for at least about 4 weeks, followed by
   administering the target drug at a dose of about 4.0 mg once a week for at least about 4 weeks, followed by
   administering the target drug at a dose of about 5.0 mg once a week for at least about 4 weeks, followed by
d) administering the target drug at a dose of about 6.0 mg once a week for at least about 4 weeks, followed by
e) administering the target drug at a dose of about 6.0 mg once a week for at least about 4 weeks, such as about 24 weeks.

In some embodiments, the present disclosure provides another method of administration, wherein a maintenance dose of the target drug is 0.5 mg-10.0 mg, 1.0 mg-8.0 mg, 1.0 mg-6.0 mg, or 1.0 mg-5.0 mg; for example, about 1.0 mg, about 2.0 mg, about 3.0 mg, about 4.0 mg, about 4.5 mg, about 6.0 mg, or about 8.0 mg.

In some embodiments, the target drug is administered in a dose-escalating manner or via dose titration.

In some embodiments, the target drug is administered once a week.

In some embodiments, the target drug is initiated at an initial dose, with dose escalation approximately every 4 weeks at an increment of 1 mg to 2 mg (e.g., one or more selected from about 0.5 mg, about 1 mg, about 1.5 mg, and about 2.0 mg), and gradually titrated to the maintenance dose of the target drug.

In some embodiments, the initial dose is about 0.1 mg to about 2.5 mg, for example, the initial dose is about 0.5 mg, about 1.0 mg or about 1.5 mg.

In some embodiments, the initial dose of the target drug is about 0.5 mg, about 1.0 mg, or about 1.5 mg, which is administered for at least about 4 weeks. Thereafter, the dose is escalated about every 4 weeks, with the dose escalation magnitude selected from one or more of about 0.5 mg, about 1 mg, about 1.5 mg, and about 2.0 mg, so as to gradually titrate to the maintenance dose of the target drug ranging from 1.0 mg to 8.0 mg (e.g., about 1.0 mg, about 2.0 mg, about 3.0 mg, about 4.0 mg, about 4.5 mg, about 6.0 mg, or about 8.0 mg).

In some embodiments, the dose of the target drug is escalated approximately every 4 weeks. Each dose increment is based on the prior dose, for example, by 1 mg to 2 mg on the basis of the prior dose, such as an increase of about 0.5 mg, about 1 mg, about 1.5 mg, or about 2.0 mg relative to the prior dose. In some embodiments, the magnitude of each dose escalation is independently the same or different. For example, the initial dose is about 1 mg, the second dose is about 2 mg with a dose escalation of about 1 mg; the third dose is about 3 mg with a dose escalation of about 1 mg; and the maintenance dose is 4.5 mg with a dose escalation of about 1.5 mg. Exemplarily, the initial dose is about 1 mg, the second dose is about 2 mg with a dose escalation of about 1 mg; the third dose is about 3 mg with a dose escalation of about 1 mg; and the maintenance dose is 4 mg with a dose escalation of about 1 mg.

In some embodiments, the maintenance dose is administered once a week.

In some embodiments, the maintenance dose is administered once every two weeks.

In some embodiments, the maintenance dose is administered once a week, then once every two weeks, for example, the maintenance dose is administered once a week for at least about 4 weeks, at least about 8 weeks, at least about 12 weeks, at least about 16 weeks, at least about 20 weeks, or at least about 24 weeks, and then the maintenance dose is administered once every two weeks.

In any of the dosing regimens disclosed herein, the dosage and duration of administration may be adjusted based on the subject's tolerance, the intended prophylactic or therapeutic effect, and other medical conditions determined by a physician or healthcare provider.

### <Target drug>

In some embodiments, the target drug is selected from GLP-1 analogues or pharmaceutically acceptable salts thereof.

In some embodiments, the GLP-1 analogue has agonist activity against at least one of the following receptors: the GLP-1 receptor and the GIP receptor. In some embodiments, the GLP-1 analogue has agonist activity at both the GLP-1 receptor and the GIP receptor.

In some embodiments, the GLP-1 analogue is a dual agonist of GLP-1 receptor and GIP receptor.

In some embodiments, the GLP-1 analogue comprises or is a GLP-1 analogue represented by the structure below, or a pharmaceutically acceptable salt thereof:
H-YAibEGTFTSDYSIYK(OEG-OEG-γGlu-C20-OH)EKIAAQEFVNWLLAGGPSSGAPPP S-NH₂.

In some embodiments, the K(OEG-OEG-γGlu-C20-OH) has the following structure:

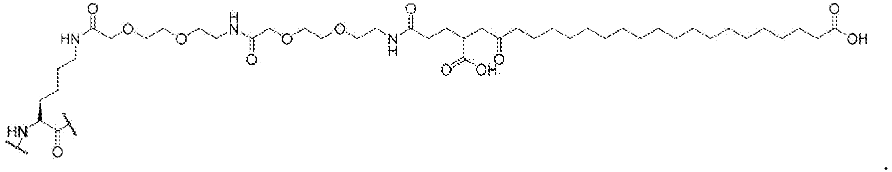

In some embodiments, the K(OEG-OEG-γGlu-C20-OH) has the following structure:

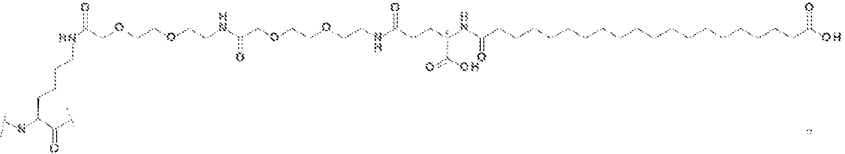

In some embodiments, the target drug is a GLP-1 analogue comprising the structure shown below, or a pharmaceutically acceptable salt thereof,

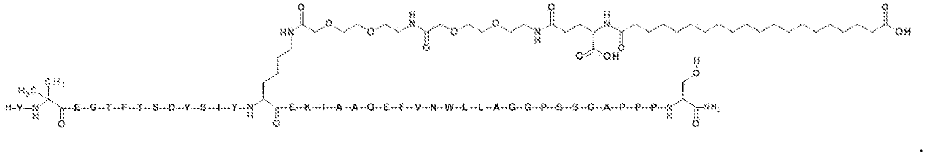

In some embodiments, the target drug is selected from any GLP-1 analogue or pharmaceutically acceptable salts or compositions thereof disclosed in WO2021239082A1. The structures, preparation methods and other relevant contents of the GLP-1 analogues and pharmaceutically acceptable salts thereof disclosed in the foregoing patent are incorporated into the present disclosure by reference.

In some embodiments, the target drug is an acetate or sodium salt of a GLP-1 analogue comprising the structure shown below,

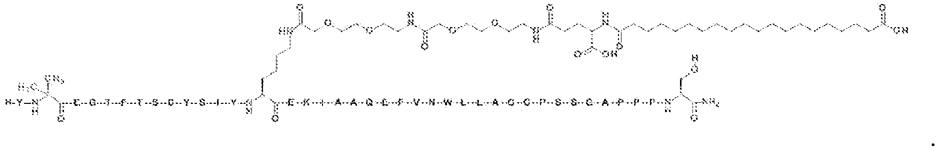

In some embodiments, the GLP-1 analogue is tirzepatide or a pharmaceutically acceptable salt thereof.

In some embodiments, the GLP-1 analogue is a compound represented by any one of the structures below, or a pharmaceutically acceptable salt thereof,

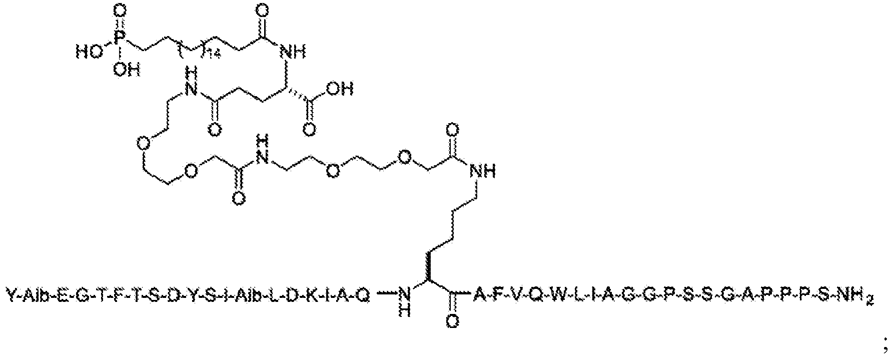

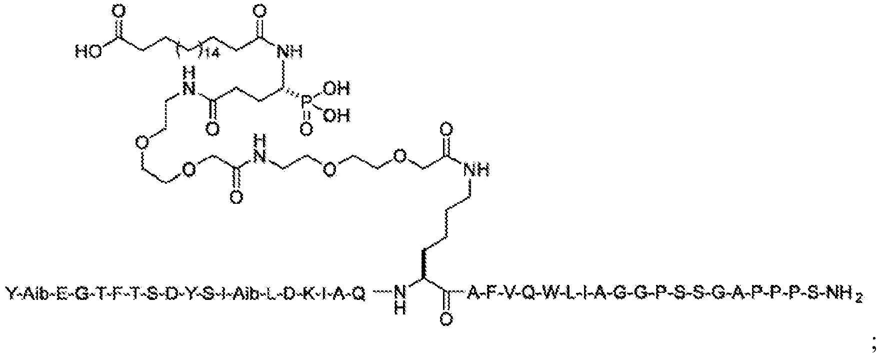

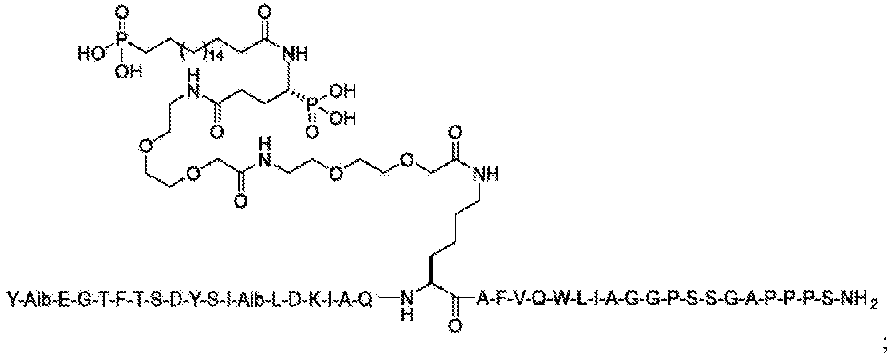

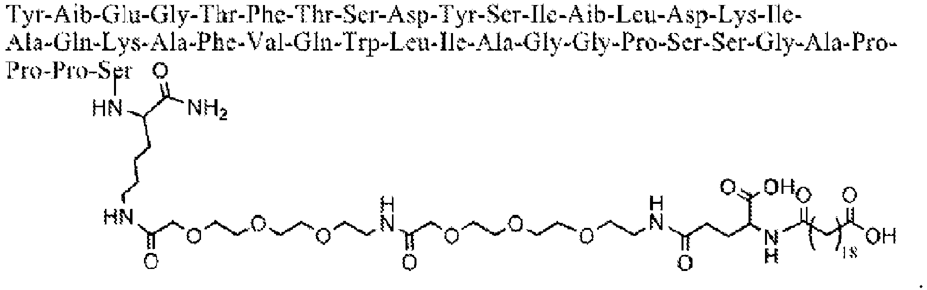

### <Obesity or overweight>

In some embodiments, the present disclosure provides a method for improving weight control in a subject in need thereof, comprising administering a target drug to the subject in need thereof according to the dosing regimen of the present disclosure. For example, the target drug comprises or is a GLP-1 analogue represented by the following structure, or a pharmaceutically acceptable salt thereof:
H-YAibEGTFTSDYSIYK(OEG-OEG-γGlu-C20-OH)EKIAAQEFVNWLLAGGPSSGAPPP S-NH₂.

In some embodiments, the present disclosure provides a method for treating obesity, comprising administering a target drug to a subject in need thereof according to the dosing regimen of the present disclosure. For example, the target drug comprises or is a GLP-1 analogue represented by the following structure, or a pharmaceutically acceptable salt thereof:
H-YAibEGTFTSDYSIYK(OEG-OEG-γGlu-C20-OH)EKIAAQEFVNWLLAGGPSSGAPPP S-NH₂.

In some embodiments, the present disclosure provides a target drug for use in the treatment of obesity, wherein the target drug is administered to a subject in need thereof according to the dosing regimen of the present disclosure. For example, the target drug comprises or is a GLP-1 analogue represented by the following structure, or a pharmaceutically acceptable salt thereof:
H-YAibEGTFTSDYSIYK(OEG-OEG-γGlu-C20-OH)EKIAAQEFVNWLLAGGPSSGAPPP S-NH₂.

In some embodiments, the K(OEG-OEG-γGlu-C20-OH) has the following structure:

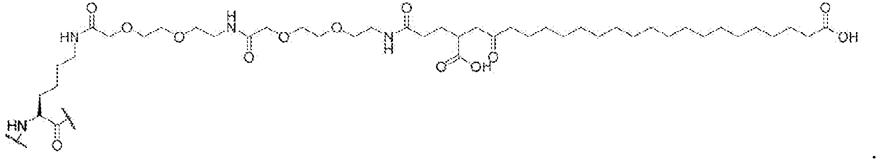

In some embodiments, the K(OEG-OEG-γGlu-C20-OH) has the following structure:

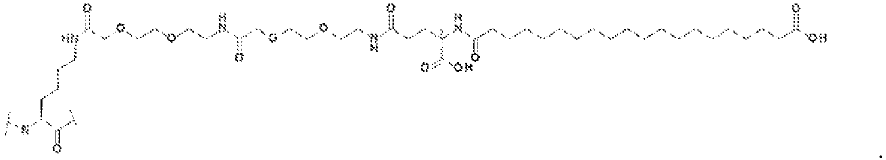

In some embodiments, the target drug is a GLP-1 analogue comprising the structure shown below, or a pharmaceutically acceptable salt thereof,

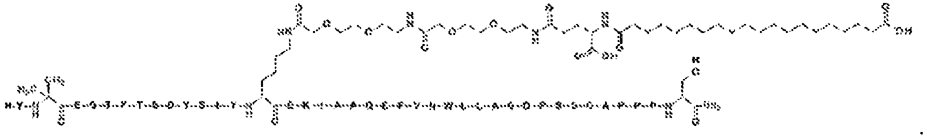

In some embodiments, the target drug is administered to a subject in need thereof in accordance with any one of the aforementioned dosing regimens.

In some embodiments, the subject is obese or overweight.

In some embodiments, the subject is obese or overweight and accompanied by at least one weight-related comorbidity. In some embodiments, the comorbidity includes one or more of prediabetes, hypertension, and dyslipidemia.

In some embodiments, the subject has BMI ≥ 28.0kg/m² and/or BMI ≤ 40.0kg/m².

In some embodiments, the subject has BMI ≥ 24.0kg/m2 and/or BMI < 28.0kg/m², and suffers from at least one weight-related comorbidity. In some embodiments, the comorbidity includes one or more of prediabetes, hypertension, and dyslipidemia.

In some embodiments, the prediabetic subjects include those with impaired fasting glucose, impaired glucose tolerance, and a combination of both.

In some embodiments, the prediabetic subjects are those with a fasting blood glucose level of 6.1 mmol/L < fasting blood glucose < 7.0 mmol/L, or an HbA1c level of 5.7% < HbA1c < 6.5%.

In some embodiments, the method reduces the body weight of the subject.

In some embodiments, the maintenance dose of the target drug is 0.5 mg-10.0 mg, 1.0 mg-8.0 mg or 1.0 mg-5.0 mg; for example, about 1.0 mg, about 2.0 mg, about 3.0 mg, about 4.0 mg, about 4.5 mg, about 6.0 mg, or about 8.0 mg.

In some embodiments, the target drug is administered in a dose-escalating manner or via dose titration.

In some embodiments, the target drug is administered once a week and/or once every two weeks. In some embodiments, the target drug is initiated at an initial dose, with dose escalation approximately every 4 weeks at an increment of 1 mg to 2 mg (e.g., one or more selected from about 0.5 mg, about 1 mg, about 1.5 mg, and about 2.0 mg), and gradually titrated to the maintenance dose of the target drug.

In some embodiments, the initial dose is about 0.1 mg to about 2.5 mg, for example, the initial dose is about 0.5 mg, about 1.0 mg or about 1.5 mg.

In some embodiments, the initial dose of the target drug is about 0.5 mg, about 1.0 mg, or about 1.5 mg, which is administered for at least about 4 weeks. Thereafter, the dose is escalated about every 4 weeks, with the dose escalation magnitude selected from one or more of about 0.5 mg, about 1 mg, about 1.5 mg, and about 2.0 mg, so as to gradually titrate to the maintenance dose of the target drug ranging from 1.0 mg to 8.0 mg (e.g., about 1.0 mg, about 2.0 mg, about 3.0 mg, about 4.0 mg, about 4.5 mg, about 6.0 mg, or about 8.0 mg).

In some embodiments, the dose of the target drug is escalated approximately every 4 weeks. Each dose increment is based on the prior dose, for example, by 1 mg to 2 mg on the basis of the prior dose, such as an increase of about 0.5 mg, about 1 mg, about 1.5 mg, or about 2.0 mg relative to the prior dose. In some embodiments, the magnitude of each dose escalation is independently the same or different. For example, the initial dose is about 1 mg, the second dose is about 2 mg with a dose escalation of about 1 mg; the third dose is about 3 mg with a dose escalation of about 1 mg; and the maintenance dose is 4.5 mg with a dose escalation of about 0.5 mg. Exemplarily, the initial dose is about 1 mg, the second dose is about 2 mg with a dose escalation of about 1 mg; the third dose is about 3 mg with a dose escalation of about 1 mg; and the maintenance dose is 4 mg with a dose escalation of about 1 mg.

In some embodiments, the maintenance dose is administered once a week.

In some embodiments, the maintenance dose is administered once every two weeks.

In some embodiments, the maintenance dose is administered once a week, then once every two weeks, for example, the maintenance dose is administered once a week for at least about 4 weeks, at least about 8 weeks, at least about 12 weeks, at least about 16 weeks, at least about 20 weeks, or at least about 24 weeks, and then the maintenance dose is administered once every two weeks.

In some embodiments, the dosing regimen includes any one of the following regimens:
(1)
   a) administering the target drug at a dose of about 0.5 mg to about 1.5 mg once a week for at least about 1 week, followed by
   d) administering the target drug at a dose of about 1.0 mg to about 6.0 mg once a week for at least about 1 week, followed by
   e) administering the target drug at a dose of about 1.0 mg to about 6.0 mg once a week for at least about 1 week, followed by
   g) administering the target drug at a dose of about 2.0 mg to about 3.0 mg once a week for at least about 1 week, followed by
   h) administering the target drug at a dose of about 2.0 mg to about 3.0 mg once a week for at least about 2 weeks;
(2)
   a) administering the target drug at a dose of about 0.5 mg to about 1.5 mg once a week for at least about 1 week, followed by
   b) administering the target drug at a dose of about 2.0 mg to about 3.0 mg once a week for at least about 1 week, followed by
   d) administering the target drug at a dose of about 1.0 mg to about 6.0 mg once a week for at least about 2 weeks, followed by
   e) administering the target drug at a dose of about 1.0 mg to about 6.0 mg once a week for at least about 1 week, followed by
   f) administering the target drug at a dose of about 1.0 mg to about 6.0 mg once every two weeks for at least about 2 weeks;
(3)
   a) administering the target drug at a dose of about 0.5 mg to about 1.5 mg once a week for at least about 1 week, followed by
   b) administering the target drug at a dose of about 2.0 mg to about 3.0 mg once a week for at least about 1 week, followed by
   d) administering the target drug at a dose of about 1.0 mg to about 6.0 mg once a week for at least about 2 weeks, followed by
   e) administering the target drug at a dose of about 1.0 mg to about 6.0 mg once a week for at least about 2 weeks;
(4)
   a) administering the target drug at a dose of about 0.5 mg to about 1.5 mg once a week for at least about 1 week, followed by
   b) administering the target drug at a dose of about 2.0 mg to about 3.0 mg once a week for at least about 1 week, followed by
   c) administering the target drug at a dose of about 3.0 mg to about 4.5 mg once a week for at least about 1 week, followed by
   d) administering the target drug at a dose of about 1.0 mg to about 6.0 mg once a week for at least about 2 weeks, followed by
   e) administering the target drug at a dose of about 1.0 mg to about 6.0 mg once a week for at least about 1 week, followed by
   f) administering the target drug at a dose of about 1.0 mg to about 6.0 mg once every two weeks for at least about 2 weeks;
(5)
   a) administering the target drug at a dose of about 0.5 mg to about 1.5 mg once a week for at least about 1 week, followed by
   b) administering the target drug at a dose of about 2.0 mg to about 3.0 mg once a week for at least about 1 week, followed by
   c) administering the target drug at a dose of about 3.0 mg to about 4.5 mg once a week for at least about 1 week, followed by
   d) administering the target drug at a dose of about 1.0 mg to about 6.0 mg once a week for at least about 2 weeks, followed by
   e) administering the target drug at a dose of about 1.0 mg to about 6.0 mg once a week for at least about 2 weeks;
(6)
   a) administering the target drug at a dose of about 0.5 mg to about 1.5 mg once a week for at least about 1 week, followed by
   d) administering the target drug at a dose of about 1.0 mg to about 6.0 mg once a week for at least about 2 weeks, followed by
   e) administering the target drug at a dose of about 1.0 mg to about 6.0 mg once a week for at least about 2 weeks;
(7)
   a) administering the target drug at a dose of about 0.5 mg to about 1.5 mg once a week for at least about 1 week, followed by
   b) administering the target drug at a dose of about 2.0 mg to about 3.0 mg once a week for at least about 1 week, followed by
   c) administering the target drug at a dose of about 3.0 mg to about 4.5 mg once a week for at least about 1 week, followed by
      administering the target drug at a dose of about 4.0 mg to about 5.0 mg once a week for at least about 1 week, followed by
      administering the target drug at a dose of about 5.0 mg to about 6.0 mg once a week for at least about 1 week, followed by
   d) administering the target drug at a dose of about 1.0 mg to about 6.0 mg once a week for at least about 2 weeks, followed by
   e) administering the target drug at a dose of about 1.0 mg to about 6.0 mg once a week for at least about 2 weeks; or
(8)
   a) administering the target drug at a dose of about 0.5 mg to about 1.5 mg once a week for at least about 1 week, followed by
   b) administering the target drug at a dose of about 2.0 mg to about 3.0 mg once a week for at least about 1 week, followed by
   c) administering the target drug at a dose of about 3.0 mg to about 4.5 mg once a week for at least about 1 week, followed by
      administering the target drug at a dose of about 4.0 mg to about 5.0 mg once a week for at least about 1 week, followed by
      administering the target drug at a dose of about 5.0 mg to about 6.0 mg once a week for at least about 1 week, followed by
      administering the target drug at a dose of about 6.0 mg to about 7.0 mg once a week for at least about 2 weeks, followed by
   d) administering the target drug at a dose of about 1.0 mg to about 8.0 mg once a week for at least about 4 weeks.

In some embodiments, the dosing regimen comprises any of the following regimens:
(1) administering the target drug at a dose of about 0.5 mg once a week for at least about 4 weeks, followed by
   administering the target drug at a dose of about 1.0 mg once a week for at least about 4 weeks, followed by
   administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks;
(2) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, followed by
   administering the target drug at a dose of about 3.0 mg once a week for at least about 4 weeks, followed by
   administering the target drug at a dose of about 3.0 mg once every two weeks for at least about 4 weeks;
(3) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, followed by
   administering the target drug at a dose of about 3.0 mg once a week for at least about 4 weeks;
(4) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, followed by
   administering the target drug at a dose of about 4.5 mg once a week for at least about 4 weeks, followed by
   administering the target drug at a dose of about 4.5 mg once a week for at least about 4 weeks, followed by
   administering the target drug at a dose of about 4.5 mg once every two weeks for at least about 4 weeks;
(5) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, followed by
   administering the target drug at a dose of about 4.5 mg once a week for at least about 4 weeks, followed by
   administering the target drug at a dose of about 4.5 mg once a week for at least about 4 weeks;
(6) administering the target drug at a dose of about 1.5 mg once a week for at least about 4 weeks, followed by
   administering the target drug at a dose of about 3.0 mg once a week for at least about 4 weeks, followed by
   administering the target drug at a dose of about 4.5 mg once a week for at least about 4 weeks, followed by
   administering the target drug at a dose of about 6.0 mg once a week for at least about 4 weeks, followed by
   administering the target drug at a dose of about 6.0 mg once a week for at least about 4 weeks, followed by
   administering the target drug at a dose of about 6.0 mg once every two weeks for at least about 4 weeks;
(7) administering the target drug at a dose of about 1.5 mg once a week for at least about 4 weeks, followed by
   administering the target drug at a dose of about 3.0 mg once a week for at least about 4 weeks, followed by
   administering the target drug at a dose of about 4.5 mg once a week for at least about 4 weeks, followed by
   administering the target drug at a dose of about 6.0 mg once a week for at least about 4 weeks, followed by
   administering the target drug at a dose of about 6.0 mg once a week for at least about 4 weeks;
(8) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 2.0 mg once a week for at least about 4 weeks;
(9) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, followed by
   administering the target drug at a dose of about 4.0 mg once a week for at least about 4 weeks, followed by
   administering the target drug at a dose of about 4.0 mg once a week for at least about 4 weeks;
(10) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, followed by
   administering the target drug at a dose of about 4.0 mg once a week for at least about 4 weeks, followed by
   administering the target drug at a dose of about 5.0 mg once a week for at least about 4 weeks, followed by
   administering the target drug at a dose of about 6.0 mg once a week for at least about 4 weeks, followed by
   administering the target drug at a dose of about 6.0 mg once a week for at least about 4 weeks; or,
(11) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, followed by
   administering the target drug at a dose of about 4.0 mg once a week for at least about 4 weeks, followed by
   administering the target drug at a dose of about 5.0 mg once a week for at least about 4 weeks, followed by
   administering the target drug at a dose of about 6.0 mg once a week for at least about 4 weeks, followed by
   administering the target drug at a dose of about 8.0 mg once a week for at least about 4 weeks.

In some embodiments, the dosing regimen comprises any of the following regimens:
(1) administering the target drug at a dose of about 0.1 mg to about 2.5 mg once a week for at least about 4 weeks, followed by
   administering the target drug at a dose of about 0.5 mg to about 10.0 mg once a week for at least about 4 weeks, such as about 28 weeks;
   optionally, administering the target drug at a dose of about 2.0 mg to about 3.0 mg once a week for at least about 4 weeks, followed by
   administering the target drug at a dose of about 2.0 mg to about 3.0 mg once a week for at least about 4 weeks, such as about 16 weeks;
(2) administering the target drug at a dose of about 0.1 mg to about 2.5 mg once a week for at least about 4 weeks, followed by
   administering the target drug at a dose of about 1.0 mg to about 5.0 mg once a week for at least about 4 weeks, followed by
   administering the target drug at a dose of about 0.5 mg to about 10.0 mg once a week for at least about 4 weeks, such as about 24 weeks;
   optionally, followed by administering the target drug at a dose of about 0.5 mg to about 10.0 mg once every two weeks for at least about 4 weeks, such as about 20 weeks;
(3) administering the target drug at a dose of about 0.1 mg to about 2.5 mg once a week for at least about 4 weeks, followed by
   administering the target drug at a dose of about 1.0 mg to about 5.0 mg once a week for at least about 4 weeks, followed by
   administering the target drug at a dose of about 1.0 mg to about 7.5 mg once a week for at least about 4 weeks, followed by
   administering the target drug at a dose of about 0.5 mg to about 10.0 mg once a week for at least about 4 weeks, such as about 20 weeks;
   optionally, followed by administering the target drug at a dose of about 0.5 mg to about 10.0 mg once every two weeks for at least about 4 weeks, such as about 20 weeks;
(4) administering the target drug at a dose of about 0.1 mg to about 2.5 mg once a week for at least about 4 weeks, followed by
   administering the target drug at a dose of about 1.0 mg to about 5.0 mg once a week for at least about 4 weeks, followed by
   administering the target drug at a dose of about 1.0 mg to about 7.5 mg once a week for at least about 4 weeks, followed by
   administering the target drug at a dose of about 1.0 mg to about 9 mg once a week for at least about 4 weeks, followed by
   administering the target drug at a dose of about 1.0 mg to about 11 mg once a week for at least about 4 weeks, followed by
   administering the target drug at a dose of about 0.5 mg to about 10.0 mg once a week for at least about 4 weeks, such as about 24 weeks; or,
(5) administering the target drug at a dose of about 0.1 mg to about 2.5 mg once a week for at least about 4 weeks, followed by
   administering the target drug at a dose of about 1.0 mg to about 5.0 mg once a week for at least about 4 weeks, followed by
   administering the target drug at a dose of about 1.0 mg to about 7.5 mg once a week for at least about 4 weeks, followed by
   administering the target drug at a dose of about 1.0 mg to about 9 mg once a week for at least about 4 weeks, followed by
   administering the target drug at a dose of about 1.0 mg to about 11 mg once a week for at least about 4 weeks, followed by
   administering the target drug at a dose of about 1.0 mg to about 11 mg once a week for at least about 4 weeks, followed by
   administering the target drug at a dose of about 0.5 mg to about 10.0 mg once a week for at least about 4 weeks, such as about 12 weeks;
(6) administering the target drug at a dose of about 0.5 mg once a week for at least about 4 weeks, followed by
   administering the target drug at a dose of about 1.0 mg once a week for at least about 4 weeks, such as about 28 weeks;
   optionally, administering the target drug at a dose of about 2.0 mg once a week for at least about 4 weeks, followed by
   administering the target drug at a dose of about 3.0 mg once a week for at least about 4 weeks, such as about 16 weeks;
(7) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, such as about 24 weeks;
   optionally, followed by administering the target drug at a dose of about 3.0 mg once every two weeks for at least about 4 weeks, such as about 20 weeks;
(8) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, followed by
   administering the target drug at a dose of about 4.5 mg once a week for at least about 4 weeks, such as about 20 weeks,
   optionally, followed by administering the target drug at a dose of about 4.5 mg once every two weeks for at least about 4 weeks, such as about 20 weeks;
(9) administering the target drug at a dose of about 1.5 mg once a week for at least about 4 weeks, followed by
   administering the target drug at a dose of about 3.0 mg once a week for at least about 4 weeks, followed by
   administering the target drug at a dose of about 4.5 mg once a week for at least about 4 weeks, followed by
   administering the target drug at a dose of about 6.0 mg once a week for at least about 4 weeks, such as about 20 weeks,
   optionally, followed by administering the target drug at a dose of about 6.0 mg once every two weeks for at least about 4 weeks, such as about 20 weeks;
(10) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, followed by
   administering the target drug at a dose of about 4.0 mg once a week for at least about 4 weeks, such as about 32 weeks;
(11) administering the target drug at a dose of about 1.0 mg once a week for about 4 weeks, followed by
   administering the target drug at a dose of about 2.0 mg once a week for at least about 4 weeks, such as about 44 weeks;
(12) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, followed by
   administering the target drug at a dose of about 4.0 mg once a week for at least about 4 weeks, followed by
   administering the target drug at a dose of about 5.0 mg once a week for at least about 4 weeks, followed by
   administering the target drug at a dose of about 6.0 mg once a week for at least about 4 weeks, such as about 28 weeks; or,
(13) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, followed by
   administering the target drug at a dose of about 4.0 mg once a week for at least about 4 weeks, followed by
   administering the target drug at a dose of about 5.0 mg once a week for at least about 4 weeks, followed by
   administering the target drug at a dose of about 6.0 mg once a week for at least about 4 weeks, followed by
   administering the target drug at a dose of about 8.0 mg once a week for at least about 4 weeks, such as about 12 weeks.

In some embodiments, the method ameliorates or reduces any of the following indicators in a subject:
1) glucose metabolism, such as fasting plasma glucose, fasting serum insulin, insulin resistance (HOMA model), and glycated hemoglobin (HbA1c);
2) blood lipids, such as total cholesterol (TC), low-density lipoprotein cholesterol (LDL-C), high-density lipoprotein cholesterol (HDL-C), and triglycerides (TG).

In some embodiments, "the method ameliorates or reduces" refers to comparison against baseline.

In some embodiments, following administration according to the administration method of the present disclosure, the subject's body weight is reduced by at least 5%, at least 10%, at least 15%, at least 20%, or at least 25%, as compared with baseline.

In some embodiments, the subject's systolic blood pressure is reduced by at least 4 mmHg or at least 8 mmHg after administration in accordance with the administration method of the present disclosure, relative to baseline.

In some embodiments, the subject's waist circumference is decreased by at least 5 cm, at least 10 cm, or at least 15 cm compared with baseline.

### <Diabetes>

In some embodiments, the present disclosure provides a method for treating type 2 diabetes, comprising administering a target drug to a subject in need thereof in accordance with the dosing regimen of the present disclosure. For example, the target drug comprises or is a GLP-1 analogue represented by the following structure, or a pharmaceutically acceptable salt thereof: H-YAibEGTFTSDYSIYK(OEG-OEG-γGlu-C20-OH)EKIAAQEFVNWLLAGGPSSGAPPPS-NH₂.

In some embodiments, the present disclosure provides a method for improving weight control in a subject with type 2 diabetes, comprising administering a target drug to a subject in need thereof in accordance with the above dosing regimen. For example, the target drug comprises or is a GLP-1 analogue represented by the following structure, or a pharmaceutically acceptable salt thereof: H-YAibEGTFTSDYSIYK(OEG-OEG-γGlu-C20-OH)EKIAAQEFVNWLLAGGPSSGAPPPS-NH₂.

In some embodiments, the present disclosure provides a method for improving blood glucose control in a subject with type 2 diabetes, comprising administering a target drug to a subject in need thereof in accordance with the above dosing regimen. For example, the target drug comprises or is a GLP-1 analogue represented by the following structure, or a pharmaceutically acceptable salt thereof: H-YAibEGTFTSDYSIYK(OEG-OEG-γGlu-C20-OH)EKIAAQEFVNWLLAGG PSSGAPPPS-NH₂.

In some embodiments, the present disclosure provides a method for improving blood lipid control in a subject with type 2 diabetes, comprising administering a target drug to a subject in need thereof in accordance with the above dosing regimen. For example, the target drug comprises or is a GLP-1 analogue represented by the following structure, or a pharmaceutically acceptable salt thereof: H-YAibEGTFTSDYSIYK(OEG-OEG-γGlu-C20-OH) -NH₂.

In some embodiments, the present disclosure provides a method for improving blood pressure (e.g., hypertension) in a subject with type 2 diabetes, comprising administering a target drug to a subject in need thereof in accordance with the above dosing regimen. For example, the target drug comprises or is a GLP-1 analogue represented by the following structure, or a pharmaceutically acceptable salt thereof: H-YAibEGTFTSDYSIYK(OEG-OEG-γGlu-C20-OH)EKIAAQEFVNW LLAGGPSSGAPPPS-NH₂.

In some embodiments, the present disclosure provides use of a target drug in combination with other hypoglycemic agents in the manufacture of a medicament for treating type 2 diabetes, wherein the other hypoglycemic agents are metformin, or metformin and an SGLT2 inhibitor. For example, the target drug comprises or is a GLP-1 analogue represented by the following structure, or a pharmaceutically acceptable salt thereof: H-YAibEGTFTSDYSIYK(OEG-OEG-γGlu-C20-OH)EKIAAQEFVNWLLAGGPSSGAPPPS-NH₂.

In some embodiments, the present disclosure provides a target drug for use in treating a subject suffering from type 2 diabetes; wherein the subject is further treated with other hypoglycemic agents, and the other hypoglycemic agents are metformin, or metformin and an SGLT2 inhibitor. For example, the target drug comprises or is a GLP-1 analogue represented by the following structure, or a pharmaceutically acceptable salt thereof: H-YAibEGTFTSDYSIYK(OEG-OEG-γGlu-C20-OH)EKIAAQEFVNWLLAGGPSSGAPPPS-NH₂.

In some embodiments, the present disclosure provides a target drug in combination with other hypoglycemic agents for treating type 2 diabetes, wherein the other hypoglycemic agents are metformin, or metformin and an SGLT2 inhibitor. For example, the target drug comprises or is a GLP-1 analogue represented by the following structure, or a pharmaceutically acceptable salt thereof: H-YAibEGTFTSDYSIYK(OEG-OEG-γGlu-C20-OH)EKIAAQEFVNWLLAGGPSSGA PPPS-NH₂.

In some embodiments, the present disclosure provides a pharmaceutical composition for treating type 2 diabetes, comprising a therapeutically effective amount of the above target drug and other hypoglycemic agents, wherein the other hypoglycemic agents are metformin, or metformin and an SGLT2 inhibitor. For example, the target drug comprises or is a GLP-1 analogue represented by the following structure, or a pharmaceutically acceptable salt thereof: H-YAibEGTFTSDYSIYK(OEG-OEG-γGlu-C20-OH)EKIAAQEFVNWLLAGGPSSGAPPPS-NH₂.

In some embodiments, the present disclosure provides a kit or product for treating type 2 diabetes, comprising a therapeutically effective amount of the above target drug and other hypoglycemic agents, wherein the other hypoglycemic agents are metformin, or metformin and an SGLT2 inhibitor. For example, the target drug comprises or is a GLP-1 analogue represented by the following structure, or a pharmaceutically acceptable salt thereof: H-YAibEGTFTSDYSI YK(OEG-OEG-γGlu-C20-OH)EKIAAQEFVNWLLAGGPSSGAPPPS-NH₂.

In some embodiments, the K(OEG-OEG-γGlu-C20-OH) has the following structure:

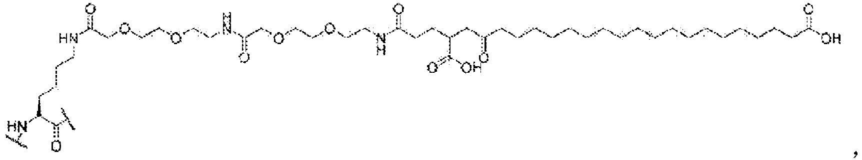

In some embodiments, the K(OEG-OEG-γGlu-C20-OH) has the following structure:

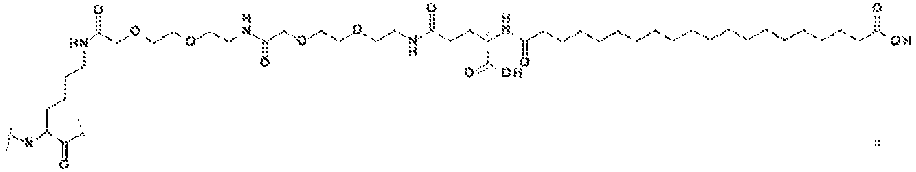

In some embodiments, the target drug is a GLP-1 analogue comprising the structure shown below, or a pharmaceutically acceptable salt thereof,

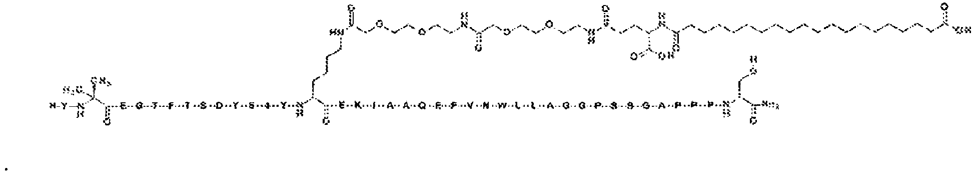

In some embodiments, the target drug is an acetate or sodium salt of a GLP-1 analogue comprising the structure shown below,

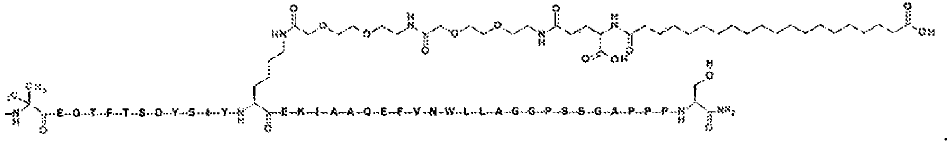

In some embodiments, the subject with type 2 diabetes has HbA1c ≥ 7.5% and/or HbA1c ≤ 11%.

In some embodiments, the subjects with type 2 diabetes fail to achieve satisfactory outcomes with lifestyle intervention alone (e.g., dietary and/or exercise management), such as poor blood glucose control following exclusive lifestyle intervention, or failure to reach the target HbA1c level after lifestyle intervention alone. In some embodiments, the type 2 diabetes subject is one whose HbA1c is still greater than 7% after lifestyle intervention alone, for example, HbA1c ≥ 7.5%.

In some embodiments, the subjects with type 2 diabetes exhibit an inadequate response to metformin treatment, such as poor blood glucose control despite metformin therapy, or failure to achieve the target HbA1c level with metformin treatment. In some embodiments, the subject has HbA1c>7% after receiving metformin treatment, for example, HbA1c≥7.5%.

In some embodiments, the subjects with type 2 diabetes have an insufficient response to combination therapy of metformin and an SGLT2 inhibitor, such as unsatisfactory blood glucose control after treatment with metformin combined with an SGLT2 inhibitor, or failure to achieve the target HbA1c level after treatment with metformin combined with an SGLT2 inhibitor. In some embodiments, the subjects have HbA1c>7%, such as HbA1c≥7.5%, after treatment with metformin combined with an SGLT2 inhibitor.

In some embodiments, the subjects receiving the target drug are further administered metformin.

In some embodiments, the subjects receiving the target drug are additionally administered metformin as the original stable treatment.

In some embodiments, the subjects receiving the target drug are further administered metformin and an SGLT2 inhibitor.

In some embodiments, the subjects receiving the target drug are further administered metformin and an SGLT2 inhibitor as the original stable treatment.

In some embodiments, stable treatment is defined as no changes in the baseline medications (metformin and SGLT2 inhibitor) and total daily doses prior to administration of the target drug. For example, the metformin dose is ≥ 1500 mg per day or the maximum tolerated dose (≥ 1000 mg per day); the SGLT2 inhibitor dose is the daily dose recommended in the product instructions.

In some implementations, the subject has BMI ≥ 22kg/m² and/or BMI ≤ 40.0kg/m².

In some embodiments, the subjects are obese.

In some embodiments, the subjects are non-obese.

In some embodiments, the subjects with type 2 diabetes satisfy at least one of the following conditions:
1) poor control with lifestyle intervention alone (e.g., dietary and/or exercise management) or metformin monotherapy;
2) HbA1c ≥ 7.5% and/or HbA1c ≤ 10.5%;
3) BMI≥22kg/m² and/or BMI≤40.0kg/m².

In some embodiments, the subjects with type 2 diabetes satisfy at least one of the following conditions:
1) poor glucose control despite treatment with metformin or metformin combined with an SGLT2 inhibitor;
2) HbA1c ≥ 7.5% and/or HbA1c ≤ 11%;
3) BMI≥22kg/m² and/or BMI≤40.0kg/m².

In some embodiments, the subjects with diabetes satisfy at least one of the following conditions:
1) being receiving treatment with metformin or metformin combined with an SGLT2 inhibitor;
2) HbA1c ≥ 7.5% and/or HbA1c ≤ 11%;
3) BMI≥22kg/m² and/or BMI≤40.0kg/m².

In some embodiments, the maintenance dose of the target drug is 0.5 mg-10.0 mg, 1.0 mg-6.0 mg, or 1.0 mg-5.0 mg; for example, about 1.0 mg, about 2.0 mg, about 3.0 mg, about 4.0 mg, or about 4.5 mg. In some embodiments, the target drug is administered in a dose-escalating manner or via dose titration.

In some embodiments, the target drug is administered once a week.

In some embodiments, the target drug is initiated at an initial dose, with dose escalation approximately every 4 weeks at an increment of 1 mg to 2 mg (e.g., one or more selected from about 1 mg and about 1.5 mg), and gradually titrated to the maintenance dose of the target drug.

In some embodiments, the initial dose is about 0.1 mg to about 2.5 mg, for example, the initial dose is about 1.0 mg.

In some embodiments, the initial dose of the target drug is about 1.0 mg administered for at least 4 weeks; thereafter, the dose is escalated approximately every 4 weeks, and the magnitude of dose escalation is selected from one or more of about 1 mg and about 1.5 mg, to gradually titrate up to the maintenance dose of the target drug ranging from 1.0 mg to 5.0 mg (e.g., about 1.0 mg, about 2.0 mg, about 3.0 mg, about 4.0 mg, or about 4.5 mg).

In some embodiments, the dose of the target drug is escalated approximately every 4 weeks. Each dose increment is based on the prior dose, for example, by 1 mg to 2 mg on the basis of the prior dose, such as an increase of about 1 mg or about 1.5 mg relative to the prior dose. In some embodiments, the magnitude of each dose escalation is independently the same or different. For example, the initial dose is about 1 mg, the second dose is about 2 mg with a dose escalation of about 1 mg; the third dose is about 3 mg with a dose escalation of about 1 mg; and the maintenance dose is 4.5 mg with a dose escalation of about 1.5 mg. Exemplarily, the initial dose is about 1 mg, the second dose is about 2 mg with a dose escalation of about 1 mg; the third dose is about 3 mg with a dose escalation of about 1 mg; and the maintenance dose is 4 mg with a dose escalation of about 1 mg.

In some embodiments, the maintenance dose is administered once a week.

In some embodiments, the dosing regimen includes any one of the following regimens:
(1)
   a) administering the target drug at a dose of about 0.5 mg to about 1.5 mg once a week for at least about 1 week, followed by
   d) administering the target drug at a dose of about 1.0 mg to about 6.0 mg once a week for at least about 2 weeks, followed by
   e) administering the target drug at a dose of about 1.0 mg to about 6.0 mg once a week, for example, for at least about 2 weeks;
(2)
   a) administering the target drug at a dose of about 0.5 mg to about 1.5 mg once a week for at least about 1 week, followed by
   b) administering the target drug at a dose of about 2.0 mg to about 3.0 mg once a week for at least about 1 week, followed by
   d) administering the target drug at a dose of about 1.0 mg to about 6.0 mg once a week for at least about 2 weeks, followed by
   e) administering the target drug at a dose of about 1.0 mg to about 6.0 mg once a week, for example, for at least about 2 weeks;
(3)
   a) administering the target drug at a dose of about 0.5 mg to about 1.5 mg once a week for at least about 1 week, followed by
   b) administering the target drug at a dose of about 2.0 mg to about 3.0 mg once a week for at least about 1 week, followed by
   c) administering the target drug at a dose of about 3.0 mg to about 4.5 mg once a week for at least about 2 weeks, followed by
   d) administering the target drug at a dose of about 1.0 mg to about 6.0 mg once a week for at least about 2 weeks, followed by
   e) administering the target drug at a dose of about 1.0 mg to about 6.0 mg once a week, for example, for at least about 2 weeks.

In some embodiments, the dosing regimen comprises any of the following regimens:
(1) administering the target drug at a dose of about 1.0 mg once a week for at least about 4 weeks, followed by
   administering the target drug at a dose of about 1.0 mg once a week for at least about 4 weeks, followed by
   administering the target drug at a dose of about 1.0 mg once a week for at least about 4 weeks;
(2) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 2.0 mg once a week for at least about 4 weeks;
(3) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, followed by
   administering the target drug at a dose of about 3.0 mg once a week for at least about 4 weeks;
(4) administering the target drug at a dose of about 1.5 mg once a week for at least about 4 weeks, followed by
   administering the target drug at a dose of about 3.0 mg once a week for at least about 4 weeks, followed by
   administering the target drug at a dose of about 4.5 mg once a week for at least about 4 weeks, followed by
   administering the target drug at a dose of about 4.5 mg once a week for at least about 4 weeks;
(5) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, followed by
   administering the target drug at a dose of about 4.0 mg once a week for at least about 4 weeks, followed by
   administering the target drug at a dose of about 4.0 mg once a week for at least about 4 weeks.

In some embodiments, the maintenance dose is administered once a week.

In some embodiments, the dosing regimen comprises any of the following regimens:
(1) administering the target drug at a dose of about 0.1 mg to about 2.5 mg once a week for at least about 4 weeks, followed by
   administering the target drug at a dose of about 0.5 mg to about 10.0 mg once a week for at least about 4 weeks, such as about 28 weeks;
(2) administering the target drug at a dose of about 0.1 mg to about 2.5 mg once a week for at least about 4 weeks, followed by
   administering the target drug at a dose of about 1.0 mg to about 5.0 mg once a week for at least about 4 weeks, followed by
   administering the target drug at a dose of about 0.5 mg to about 10.0 mg once a week for at least about 4 weeks, such as about 24 weeks; or,
(3) administering the target drug at a dose of about 0.1 mg to about 2.5 mg once a week for at least about 4 weeks, followed by
   administering the target drug at a dose of about 1.0 mg to about 5.0 mg once a week for at least about 4 weeks, followed by
   administering the target drug at a dose of about 1.0 mg to about 7.5 mg once a week for at least about 4 weeks, followed by
   administering the target drug at a dose of about 0.5 mg to about 10.0 mg once a week for at least about 4 weeks, such as about 36 weeks.

In some embodiments, the dosing regimen comprises any of the following regimens:
(1) administering the target drug at a dose of about 0.5 mg to about 1.5 mg once a week for at least about 4 weeks, followed by
   administering the target drug at a dose of about 1.0 mg to about 6.0 mg once a week for at least about 4 weeks, such as about 28 weeks;
(2) administering the target drug at a dose of about 0.5 mg to about 1.5 mg once a week for at least about 4 weeks, followed by
   administering the target drug at a dose of about 2.0 mg to about 3.0 mg once a week for at least about 4 weeks, followed by
   administering the target drug at a dose of about 1.0 mg to about 6.0 mg once a week for at least about 4 weeks, such as about 24 weeks;
(3) administering the target drug at a dose of 0.5 mg to about 1.5 mg once a week for at least about 4 weeks, followed by
   administering the target drug at a dose of about 2.0 mg to about 3.0 mg once a week for at least about 4 weeks, followed by
   administering the target drug at a dose of about 3.0 mg to about 4.5 mg once a week for at least about 4 weeks, followed by
   administering the target drug at a dose of about 1.0 mg to about 6.0 mg once a week for at least about 4 weeks, such as about 36 weeks; or,

In some embodiments, the dosing regimen comprises any of the following regimens:
(1) administering the target drug at a dose of about 1.0 mg once a week for at least about 4 weeks, followed by
   administering the target drug at a dose of about 1.0 mg once a week for at least about 4 weeks, such as about 28 weeks;
(2) administering the target drug at a dose of about 1.0 mg once a week for at least about 4 weeks, followed by
   administering the target drug at a dose of about 2.0 mg once a week for at least about 4 weeks, such as about 28 weeks;
(3) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, such as about 24 weeks;
(4) administering the target drug at a dose of about 1.5 mg once a week for at least about 4 weeks, followed by
   administering the target drug at a dose of about 3.0 mg once a week for at least about 4 weeks, followed by
   administering the target drug at a dose of about 4.5 mg once a week for at least about 4 weeks, such as about 24 weeks; or,
(5) administering the target drug at a dose of about 1.0 mg once a week for at least about 4 weeks, followed by
   administering the target drug at a dose of about 2.0 mg once a week for at least about 4 weeks, followed by
   administering the target drug at a dose of about 3.0 mg once a week for at least about 4 weeks, followed by
   administering the target drug at a dose of about 4.0 mg once a week for at least about 4 weeks, such as about 36 weeks.

In some embodiments, the method reduces the body weight of the subject.

In some embodiments, the method ameliorates or reduces at least one of the following indicators in a subject:
1) HbA1c;
2) the proportions of subjects with HbA1c < 7.0%, HbA1c ≤ 6.5%, or HbA1c < 5.7%;
3) fasting plasma glucose, serum insulin, and C-peptide;
4) 2h post-mixed-meal plasma glucose, serum insulin, and C-peptide after a mixed meal;
5) pancreatic β-cell function and insulin resistance (HOMA model, such as HOMA-β and HOMA-IR);
6) average 7-point self-monitored blood glucose (SMBG);
7) body weight, waist circumference, and BMI;
8) the proportions of subjects with a body weight reduction of ≥ 5%, ≥ 10%, or ≥ 15%;
9) the proportion of subjects with HbA1c < 7.0% and a body weight reduction of ≥ 5%;
10) the proportion of subjects with HbA1c ≤ 6.5% and a body weight reduction of ≥ 10%;
11) blood lipids, such as one or more selected from TG, TC, LDL-C, non-HDL-C, and HDL-C;
12) blood pressure, such as systolic blood pressure and/or diastolic blood pressure.

In some embodiments, "the method ameliorates or reduces" refers to comparison against baseline.

In some embodiments, the subject exhibits an HbA_{1C} reduction of at least 2%, at least 2.5%, or at least 3% after administration according to the dosing regimen of the present disclosure, compared with baseline.

In some embodiments, the subject exhibits a body weight reduction of at least 3%, at least 5%, or at least 10% after administration according to the dosing regimen of the present disclosure, compared with baseline.

In some embodiments, the subject exhibits a reduction in urine albumin-to-creatinine ratio of at least 15%, at least 30%, at least 45%, or at least 50% after administration according to the dosing regimen of the present disclosure, compared with baseline.

In some embodiments, the subject exhibits a reduction in triglycerides of at least 10%, at least 20%, at least 25%, or at least 30% after administration according to the dosing regimen of the present disclosure, compared with baseline.

In some embodiments, the subject exhibits a reduction in systolic blood pressure of at least 5% or at least 10% after administration according to the dosing regimen of the present disclosure, compared with baseline.

The present disclosure further provides another exemplary embodiment:
Embodiment 1. A method for preventing or treating metabolic diseases, comprising administering a target drug to a subject in need thereof approximately once a week, wherein the target drug comprises a GLP-1 analogue having the structure shown below, or a pharmaceutically acceptable salt thereof:
   H-YAibEGTFTSDYSIYK(OEG-OEG-γGlu-C20-OH)EKIAAQEFVNWLLAGGPSSGAPPP S-NH₂.
Embodiment 2. The method according to Embodiment 1, wherein a maintenance dose of the target drug is 0.5 mg-10.0 mg or 1.0 mg-8.0 mg; preferably, the maintenance dose is about 1.0 mg, about 2.0 mg, about 3.0 mg, about 4.0 mg, about 4.5 mg, about 6.0 mg, or about 8.0 mg.
Embodiment 3. The method according to Embodiment 1 or 2, wherein the target drug is administered in a dose-escalating manner.
Embodiment 4. The method according to any one of Embodiments 1 to 3, wherein the target drug is administered starting from an initial dose, the dose is escalated approximately every 4 weeks at a dose increment of 1 mg to 2 mg, and the dose is gradually escalated to the maintenance dose of the target drug,
   preferably, the dose increment is selected from one or more of about 0.5 mg, about 1 mg, about 1.5 mg, and about 2.0 mg,
   preferably, the initial dose is from about 0.1 mg to about 2.5 mg, more preferably, the initial dose is from about 0.5 mg, about 1.0 mg or about 1.5 mg.
Embodiment 5. The method according to any one of Embodiments 1 to 4, wherein the initial dose of the target drug is about 0.5 mg, about 1.0 mg, or about 1.5 mg, administered for at least about 4 weeks;
   then, the dose is escalated approximately every 4 weeks, and the dose increment is selected from one or more of about 0.5 mg, about 1 mg, about 1.5 mg, and about 2.0 mg, to gradually titrate to the maintenance dose of 1.0 mg to 8.0 mg for the target drug;
   preferably, the maintenance dose is about 1.0 mg, about 2.0 mg, about 3.0 mg, about 4.0 mg, about 4.5 mg, about 6.0 mg, or about 8.0 mg.
Embodiment 6. The method according to any one of Embodiments 1 to 5, wherein each dose increment of the target drug is independently the same or different.
Embodiment 7. The method according to any one of Embodiments 1 to 6, wherein the maintenance dose is administered once a week initially, followed by once every two weeks; preferably, the maintenance dose of the target drug is administered once a week for at least about 4 weeks, and then the maintenance dose is administered once every two weeks.
Embodiment 8. The method according to any one of Embodiments 1 to 7, wherein the dosing regimen comprises any one of the following regimens:
   (1) administering the target drug at a dose of about 0.1 mg to about 2.5 mg once a week for at least about 4 weeks, followed by
      administering the target drug at a dose of about 0.5 mg to about 10.0 mg once a week for at least about 4 weeks;
      optionally, followed by administering the target drug at a dose of about 2.0 mg to about 3.0 mg once a week for at least about 4 weeks, followed by
      administering the target drug at a dose of about 2.0 mg to about 3.0 mg once a week for at least about 4 weeks;
   (2) administering the target drug at a dose of about 0.1 mg to about 2.5 mg once a week for at least about 4 weeks, followed by
      administering the target drug at a dose of about 1.0 mg to about 5.0 mg once a week for at least about 4 weeks, followed by
      administering the target drug at a dose of about 0.5 mg to about 10.0 mg once a week for at least about 4 weeks; followed by
      optionally, followed by administering the target drug at a dose of about 0.5 mg to about 10.0 mg once every two weeks for at least about 4 weeks;
   (3) administering the target drug at a dose of about 0.1 mg to about 2.5 mg once a week for at least about 4 weeks, followed by
      administering the target drug at a dose of about 1.0 mg to about 5.0 mg once a week for at least about 4 weeks, followed by
      administering the target drug at a dose of about 1.0 mg to about 7.5 mg once a week for at least about 4 weeks, followed by
      administering the target drug at a dose of about 0.5 mg to about 10.0 mg once a week for at least about 4 weeks;
      optionally, followed by administering the target drug at a dose of about 0.5 mg to about 10.0 mg once every two weeks for at least about 4 weeks;
   (4) administering the target drug at a dose of about 0.1 mg to about 2.5 mg once a week for at least about 4 weeks, followed by
      administering the target drug at a dose of about 1.0 mg to about 5.0 mg once a week for at least about 4 weeks, followed by
      administering the target drug at a dose of about 1.0 mg to about 7.5 mg once a week for at least about 4 weeks, followed by
      administering the target drug at a dose of about 1.0 mg to about 9 mg once a week for at least about 4 weeks, followed by
      administering the target drug at a dose of about 1.0 mg to about 11 mg once a week for at least about 4 weeks, followed by
      administering the target drug at a dose of about 0.5 mg to about 10.0 mg once a week for at least about 4 weeks; or,
   (5) administering the target drug at a dose of about 0.1 mg to about 2.5 mg once a week for at least about 4 weeks, followed by
      administering the target drug at a dose of about 1.0 mg to about 5.0 mg once a week for at least about 4 weeks, followed by
      administering the target drug at a dose of about 1.0 mg to about 7.5 mg once a week for at least about 4 weeks, followed by
      administering the target drug at a dose of about 1.0 mg to about 9 mg once a week for at least about 4 weeks, followed by
      administering the target drug at a dose of about 1.0 mg to about 11 mg once a week for at least about 4 weeks, followed by
      administering the target drug at a dose of about 1.0 mg to about 11 mg once a week for at least about 4 weeks, followed by
      administering the target drug at a dose of about 0.5 mg to about 10.0 mg once a week for at least about 4 weeks;
   (6) administering the target drug at a dose of about 0.5 mg once a week for at least about 4 weeks, followed by
      administering the target drug at a dose of about 1.0 mg once a week for at least about 4 weeks;
      optionally, followed by administering the target drug at a dose of about 2.0 mg once a week for at least about 4 weeks, followed by
      administering the target drug at a dose of about 3.0 mg once a week for at least about 4 weeks;
   (7) administering the target drug at a dose of about 1.0 mg once a week for at least about 4 weeks, followed by
      administering the target drug at a dose of about 2.0 mg once a week for at least about 4 weeks, followed by
      administering the target drug at a dose of about 3.0 mg once a week for at least about 4 weeks;
      optionally, followed by administering the target drug at a dose of about 3.0 mg once every two weeks for at least about 4 weeks;
   (8) administering the target drug at a dose of about 1.0 mg once a week for at least about 4 weeks, followed by
      administering the target drug at a dose of about 2.0 mg once a week for at least about 4 weeks, followed by
      administering the target drug at a dose of about 3.0 mg once a week for at least about 4 weeks, followed by
      administering the target drug at a dose of about 4.5 mg once a week for at least about 4 weeks,
      optionally, followed by administering the target drug at a dose of about 4.5 mg once every two weeks for at least about 4 weeks;
   (9) administering the target drug at a dose of about 1.5 mg once a week for at least about 4 weeks, followed by
      administering the target drug at a dose of about 3.0 mg once a week for at least about 4 weeks, followed by
      administering the target drug at a dose of about 4.5 mg once a week for at least about 4 weeks, followed by
      administering the target drug at a dose of about 6.0 mg once a week for at least about 4 weeks,
      optionally, followed by administering the target drug at a dose of about 6.0 mg once every two weeks for at least about 4 weeks;
   (10) administering the target drug at a dose of about 1.0 mg once a week for at least about 4 weeks, followed by
      administering the target drug at a dose of about 2.0 mg once a week for at least about 4 weeks, followed by
      administering the target drug at a dose of about 3.0 mg once a week for at least about 4 weeks, followed by
      administering the target drug at a dose of about 4.0 mg once a week for at least about 4 weeks;
   (11) administering the target drug at a dose of about 1.0 mg once a week for about 4 weeks, followed by
      administering the target drug at a dose of about 2.0 mg once a week for at least about 4 weeks;
   (12) administering the target drug at a dose of about 1.0 mg once a week for at least about 4 weeks, followed by
      administering the target drug at a dose of about 2.0 mg once a week for at least about 4 weeks, followed by
      administering the target drug at a dose of about 3.0 mg once a week for at least about 4 weeks, followed by
      administering the target drug at a dose of about 4.0 mg once a week for at least about 4 weeks, followed by
      administering the target drug at a dose of about 5.0 mg once a week for at least about 4 weeks, followed by
      administering the target drug at a dose of about 6.0 mg once a week for at least about 4 weeks; or,
   (13) administering the target drug at a dose of about 1.0 mg once a week for at least about 4 weeks, followed by
      administering the target drug at a dose of about 2.0 mg once a week for at least about 4 weeks, followed by
      administering the target drug at a dose of about 3.0 mg once a week for at least about 4 weeks, followed by
      administering the target drug at a dose of about 4.0 mg once a week for at least about 4 weeks, followed by
      administering the target drug at a dose of about 5.0 mg once a week for at least about 4 weeks, followed by
      administering the target drug at a dose of about 6.0 mg once a week for at least about 4 weeks, followed by
      administering the target drug at a dose of about 8.0 mg once a week for at least about 4 weeks;
   (14) administering the target drug at a dose of about 1.0 mg once a week for about 4 weeks, followed by
      administering the target drug at a dose of about 1.0 mg once a week for at least about 4 weeks;
   (15) administering the target drug at a dose of about 1.5 mg once a week for at least about 4 weeks, followed by
      administering the target drug at a dose of about 3.0 mg once a week for at least about 4 weeks, followed by
      administering the target drug at a dose of about 4.5 mg once a week for at least about 4 weeks.
Embodiment 9. The method according to any one of Embodiments 1 to 8, wherein the metabolic disease is obesity or overweight.
Embodiment 10. A method for improving weight control in a subject in need thereof, comprising administering a target drug to the subject according to the method of any one of Embodiments 1 to 8,
   wherein the target drug is a GLP-1 analogue comprising the structure shown below, or a pharmaceutically acceptable salt thereof: H-YAibEGTFTSDYSIYK(OEG-OEG-γGlu-C20-OH)EKIAAQEFVNWLLAGGPSSGAPPPS-NH₂.
Embodiment 11. A method for improving blood glucose control in a subject in need thereof, comprising administering a target drug to the subject according to the method of any one of Embodiments 1 to 8,
   wherein the target drug is a GLP-1 analogue comprising the structure shown below, or a pharmaceutically acceptable salt thereof: H-YAibEGTFTSDYSIYK(OEG-OEG-γGlu-C20-OH)EKIAAQEFVNWLLAGGPSSGAPPPS-NH₂.
Embodiment 12. A method for improving blood lipid control in a subject in need thereof, comprising administering a target drug to the subject according to the method of any one of Embodiments 1 to 8,
   wherein the target drug is a GLP-1 analogue comprising the structure shown below, or a pharmaceutically acceptable salt thereof: H-YAibEGTFTSDYSIYK(OEG-OEG-γGlu-C20-OH)EKIAAQEFVNWLLAGGPSSGAPPPS-NH₂.
Embodiment 13. The method according to any one of Embodiments 1 to 12, wherein the metabolic disease is diabetes, preferably type 2 diabetes.
Embodiment 14. A method for treating type 2 diabetes in a subject in need thereof, comprising administering a target drug to the subject according to the method of Embodiment 13,
   wherein the target drug is a GLP-1 analogue comprising the structure shown below, or a pharmaceutically acceptable salt thereof: H-YAibEGTFTSDYSIYK(OEG-OEG-γGlu-C20-OH)EKIAAQEFVNWLLAGGPSSGAPPPS-NH₂,
   wherein the subject with type 2 diabetes satisfies at least one of the following conditions:
      1) poor efficacy with lifestyle intervention alone;
      2) poor efficacy after treatment with metformin, or metformin in combination with an SGLT2 inhibitor;
      3) HbA1c ≥ 7.5% and/or HbA1c ≤ 11%;
      4) BMI≥22kg/m² and/or BMI≤40.0kg/m².
Embodiment 15. The method according to Embodiment 13 or 14, wherein the subject is further administered metformin, or metformin and an SGLT2 inhibitor.
Embodiment 16. The method according to any one of Embodiments 13 to 15, wherein the method ameliorates or reduces at least one of the following indicators in the subject:
   1) HbA1c;
   2) the proportions of subjects with HbA1c < 7.0%, HbA1c ≤ 6.5%, or HbA1c < 5.7%;
   3) fasting plasma glucose, serum insulin, and C-peptide;
   4) 2h post-mixed-meal plasma glucose, serum insulin, and C-peptide after a mixed meal;
   5) pancreatic β-cell function and insulin resistance;
   6) average 7-point self-monitored blood glucose;
   7) body weight, waist circumference, and BMI;
   8) the proportions of subjects with a body weight reduction of ≥ 5%, ≥ 10%, or ≥ 15%;
   9) the proportion of subjects with HbA1c < 7.0% and a body weight reduction of ≥ 5%;
   10) blood lipids, preferably including one or more of TC, LDL-C, HDL-C and TG;
   11) blood pressure, preferably including systolic and/or diastolic blood pressure.
Embodiment 17. The method according to any one of Embodiments 1 to 18, wherein the target drug is a GLP-1 analogue comprising the structure shown below, or a pharmaceutically acceptable salt thereof,

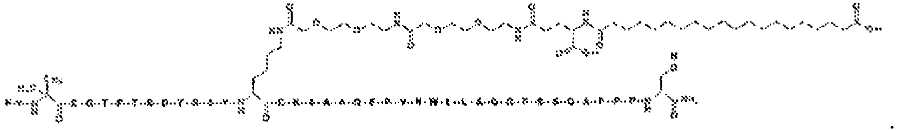

In some embodiments, the target drug is administered via parenteral administration, which includes, but is not limited to, intravenous injection, subcutaneous injection, and intramuscular injection. In some embodiments, the target drug is administered via subcutaneous injection into the abdomen.

In some embodiments, the target drug is formulated in an injectable form; for example, the pharmaceutical composition containing the aforementioned target drug further comprises at least one of a buffer, an osmotic pressure regulator, a pH regulator, and a bacteriostatic agent.

In some embodiments, the buffer is selected from one or more of acetate buffer, histidine buffer, phosphate buffer, succinate buffer and citrate buffer. In some embodiments, the buffer is a phosphate buffer, such as disodium hydrogen phosphate.

In some embodiments, the pharmaceutical composition further comprises an osmotic pressure regulator. The osmotic pressure regulator includes, but is not limited to: salts (e.g., sodium chloride, phosphate, sodium citrate, boric acid, and sodium tartrate), sugars or sugar alcohols (lactose, trehalose, sucrose, glucose, mannitol, sorbitol, xylitol), amino acids (e.g., L-glycine, L-histidine, arginine, lysine, isoleucine, aspartic acid, tryptophan, threonine), polyhydroxy alcohols [e.g., glycerol, 1,2-propylene glycol (also referred to as propylene glycol in the present disclosure), 1,3-propylene glycol, 1,3-butanediol], polyethylene glycol (e.g., PEG 400), or mixtures thereof. In some embodiments, the osmotic regulator is selected from one or more of propylene glycol, mannitol, sorbitol, xylitol, glycerol, lactose, trehalose, sucrose, glucose, sodium chloride, phosphate, sodium citrate, boric acid, and sodium tartrate. In some embodiments, the osmotic pressure regulator is sodium chloride. In some embodiments, the osmotic pressure regulator is propylene glycol or mannitol.

In some embodiments, the pharmaceutical composition further comprises a pharmaceutically acceptable bacteriostatic agent. In some embodiments, the pharmaceutically acceptable bacteriostatic agent includes, but is not limited to: phenol, o-cresol, m-cresol, p-cresol, methylparaben, propylparaben, 2-phenoxyethanol, butylparaben, 2-phenethyl alcohol, benzyl alcohol, ethanol, chlorobutanol, thimerosal, bronopol, benzoic acid, imidurea, chlorhexidine, sodium dehydroacetate, chlorocresol, ethylparaben, benzethonium chloride, or mixtures thereof. In some embodiments, the pharmaceutically acceptable bacteriostatic agent is phenol.

In some embodiments, the pharmaceutical composition is any pharmaceutical composition with components and concentrations disclosed in WO2023083301. The contents such as the compositions and preparation methods in the aforementioned patent are incorporated into the present disclosure by reference.

In some embodiments, administration of the target drug according to the dosing regimen provided herein can significantly reduce body weight in obese subjects and exert beneficial effects on glucose and lipid metabolism.

In some embodiments, compared with other GLP-1 analogues (e.g., dual agonists of GLP-1 receptor and GIP receptor, such as tirzepatide), administration of the aforementioned target drug in accordance with the dosing regimen of the present disclosure achieves superior weight control and/or blood glucose control.

In some embodiments, administration of the aforementioned target drug with the dosing regimen disclosed herein provides a superior dosage advantage relative to other GLP-1 analogues (e.g., dual agonists of GLP-1 receptor and GIP receptor, such as tirzepatide).

In some embodiments, administration of the aforementioned target drug using the dosing regimen provided herein results in reduced adverse reactions compared with other GLP-1 analogues (e.g., dual agonists of GLP-1 receptor and GIP receptor, such as tirzepatide), for example, a lowered risk of hypoglycemia, such as a lowered risk of severe hypoglycemia.

In some embodiments, the present disclosure provides a method for reducing the risk of hypoglycemia (e.g., severe hypoglycemia) in a subject, comprising administering the aforementioned target drug according to the dosing regimen described herein. For instance, the subject suffers from metabolic diseases, such as type 2 diabetes.

### Terms

To facilitate the understanding of the present disclosure, some technical and scientific terms are specifically defined below. Unless specifically defined otherwise herein, all other technical and scientific terms used herein have the same meaning as commonly understood by one of ordinary skill in the art to which the present disclosure belongs.

Unless the context clearly requires otherwise, throughout the specification and claims, the words "comprising," "having," "including," etc., should be understood as having an inclusive meaning, rather than an exclusive or exhaustive meaning; that is, the meaning of "including but not limited to".

"About" or "approximately" refer to a value falling within an acceptable error range of the specific value as determined by a person of ordinary skill in the art, which depends in part on the manner of measurement or determination (i.e., the limitations of the measurement system). For example, "about" can mean within 1 or more than 1 standard deviation. Alternatively, "about" or "substantially comprising" may mean a variation range of up to 20%, such as 1% to 15%, 1% to 10%, 1% to 5%, 0.5% to 5%, or 0.5% to 1%. In the present disclosure, every instance where the term "about" precedes a number or numerical range also encompasses embodiments directed to the exact given value. Unless otherwise specified, when specific values appear in the present application and the claims, "about" or "substantially comprising" shall be deemed to cover values within the acceptable error range of such specific values.

The term "and/or", such as "X and/or Y", should be understood to mean "X and Y" or "X or Y" and should be used to provide explicit support for both meanings or either meaning.

"Optional" or "optionally" means that the subsequently described event or circumstance may, but not necessarily, occur, and that the description includes instances where the event or circumstance occurs and does not occur.

The term "native GLP-1" refers to naturally occurring molecules of the glucagon family or the exendin family of peptides, wherein: the glucagon family of peptides is encoded by the preproglucagon gene and includes three small peptides with high homology, namely glucagon (1-29), GLP-1 (1-37) and GLP-2 (1-33); exendins are peptides expressed in lizards and, like GLP-1, are insulinotropic. In some embodiments, the term "native GLP-1" also refers to human GLP-1 (7-37) and human GLP-1 (7-36). The term "native GIP" refers to a peptide comprising the human GIP (1-42) sequence.

The term "GLP-1 analogue" refers to one having at most 25, at most 24, at most 23, at most 22, at most 21, at most 20, at most 19, at most 18, at most 17, at most 16, at most 15, at most 14, at most 13, at most 12, at most 11, at most 10, at most 9, at most 8, at most 7, at most 6, at most 5, at most 4, at most 3, at most 2, or 1 amino acid modification or chemical modification compared to native GLP-1(in particular human GLP-1 (7-37) and human GLP-1 (7-36)), wherein the amino acid modification may be an amino acid substitution, addition and/or deletion; and the chemical modification may be a chemical modification with a group selected from: amide, carbohydrate, alkyl, acyl, ester, a polyethylene glycol (PEG) group, a sialylation group, a glycosylation group, and the like.

In some embodiments, the GLP-1 analogue is a GLP-1 receptor agonist. In some embodiments, the GLP-1 analogue is a dual agonist of GLP-1 receptor and GIP receptor.

The term agonist is defined as a substance that activates the referenced receptor type. Terms such as "activity", "activation" and "activate" are measured by assays known in the art.

An amino acid "substitution" as described in the present disclosure means the replacement of one amino acid residue with a different amino acid residue.

As used herein, the term "peptide" encompasses peptides with modified amino termini and carboxyl termini. For example, an amino acid chain containing a terminal carboxylic acid replaced with an amide group is also included in the amino acid sequences designated as natural amino acids.

All hydrogen atoms described in the present disclosure may be substituted with their isotopes (protium, deuterium and tritium), and any hydrogen atom in the compounds involved in the present disclosure may also be replaced by an isotope atom.

"Optional" or "optionally" means that the subsequently described event or circumstance may, but not necessarily, occur, and that the description includes instances where the event or circumstance occurs and does not occur. For example, "a heterocyclic group optionally substituted with an alkyl group" means that the alkyl group may be, but not necessarily, present, and the description includes both the case where the heterocyclic group is substituted with the alkyl group and the case where the heterocyclic group is not substituted with the alkyl group.

"Substituted" means that one or more hydrogen atoms in a group, preferably up to 5, more preferably 1 to 3 hydrogen atoms, are independently replaced by substituents. Substituents shall only be located at chemically feasible positions. A person skilled in the art can determine (either experimentally or theoretically) feasible or non-feasible substitution modes without undue effort. For example, an amino group or hydroxyl group bearing free hydrogen may be unstable when bound to a carbon atom with an unsaturated bond (e.g., an alkene bond).

"Pharmaceutical composition" refers to a mixture containing one or more compounds described herein, or a physiological salt/pharmaceutically acceptable salt or prodrug thereof
in combination with other chemical components; such other components include physiological/pharmaceutically acceptable carriers and excipients. The purpose of the pharmaceutical composition is to promote administration to an organism, which facilitates the absorption of the active ingredients, thereby exerting biological activities.

"Buffer" refers to a buffer that is resistant to pH changes through the action of its acid-base conjugate components. Examples of buffers for maintaining pH within an appropriate range include acetate, succinate, citrate, phosphate, gluconate, histidine, oxalate, lactate, tartrate, fumarate, glycylglycine, and other organic acid buffers.

The three-letter and single-letter amino acid codes used in the present disclosure are as described in J. biol. chem, 243, p3558 (1968).

When applied to animals, humans, experimental subjects, cells, tissues, organs, or biological fluids, "administration" and "treatment" refer to the contact between an exogenous drug, therapeutic agent, diagnostic agent, or composition and the animal, human, subject, cell, tissue, organ, or biological fluid. "Administration" and "treatment" may include, for example, therapeutic, pharmacokinetic, diagnostic, research and experimental approaches. Cell processing includes contact between a reagent and a cell, and contact between a reagent and a fluid, wherein the fluid is in contact with the cell. "Administration" and "treatment" also refer to in vitro and ex vivo processing of cells, such as by means of reagents, diagnostics, binding compositions, or another cell type. When applied to human, veterinary or research subjects, "treatment" refers to therapeutic intervention, prophylactic or preventive measures, as well as research and diagnostic applications.

"Treatment" means the administration to a patient of an internal or topical therapeutic agent, such as a composition comprising any binding compound of the present disclosure, wherein the patient presents with one or more disease symptoms for which such therapeutic agents are known to exert a therapeutic effect. Typically, the therapeutic agent is administered to a treated patient or patient population in an amount effective to alleviate one or more disease symptoms, so as to induce regression of such symptoms or inhibit their progression to any clinically measurable extent. The amount of a therapeutic agent that is effective in alleviating the symptoms of any specific disease (also referred to as the "therapeutically effective amount") can vary depending on a variety of factors, such as the disease state, age and body weight of a patient, and the ability of a drug to produce the desired therapeutic effect in the patient. Whether the symptoms of a disease have been relieved can be assessed using any clinical test that a doctor or other healthcare professional typically uses to assess the severity or progression of the symptoms. Although the embodiments of the present disclosure (e.g., treatment methods or products) may be ineffective in alleviating the symptoms of each target disease, they should reduce the symptoms of the target disease in a statistically significant number of patients, as determined by any statistical test known in the art, such as the Student t-test, chi-square test, U-test according to Mann and Whitney, Kruskal-Wallis test (H-test), Jonckheere-Terpstra test, and Wilcoxon test.

An "effective amount" refers to an amount sufficient to improve or prevent the symptoms or condition of a medical disease. An effective amount also refers to an amount sufficient to allow or facilitate a diagnosis. The effective amount for a specific human or veterinary subject may vary based on factors including the condition to be treated, the overall health status of the patient, the route and frequency of administration, and the severity of side effects. An effective amount can be the maximum dose or dosing regimen that avoids significant side effects or toxicity.

As used herein, "blood glucose control" refers to the regulation of glucose metabolism indicators. Exemplarily, it refers to maintaining or reducing fasting plasma glucose, fasting serum insulin, glycated hemoglobin (HbA1c), insulin resistance (HOMA model), and the like.

As used herein, "weight control" or "weight management" refers to the management of obesity or overweight in an individual; "improved" weight management means body weight reduction.

As used herein, "titrated dose" or "escalating dose" refers to a dose lower than the maximum effective dose required for a patient. As used herein, the present disclosure encompasses that a "titrated dose" or "escalating dose" may be converted to the required maximum effective dose, or may be converted to a "maintenance dose" if such dose is observed to be effective for the patient.

As used herein, a "maintenance dose" refers to a dose serving as the maximum effective dose required for a patient. Where a maintenance dose is lower than the required maximum effective dose, such maintenance dose may be converted to an escalating dose. That is, if a maintenance dose of "about 4.5 mg" encompassed by the present disclosure is not the maximum effective dose needed for a specific patient, the 4.5 mg maintenance dose shall instead serve as an escalating dose, and the dosage for that specific patient will be increased until the next higher maintenance dose encompassed herein, for example, 6.0 mg, is maintained for at least about 2 weeks.

As used herein, "pharmaceutically acceptable salts" are well known to persons skilled in the art. In one embodiment, the GLP-1 analogue is in the form of a pharmaceutically acceptable salt. In one embodiment, the GLP-1 analogue is in a non-salt form.

The terms "subject" and "patient" refer to mammals, especially primates, and particularly humans.

### Detailed Description of the Embodiments

Hereinafter, the present disclosure is further described in conjunction with examples, which are not intended to limit the scope of the present disclosure. Unless otherwise specified, the experimental methods in the examples of the present disclosure are generally carried out under conventional conditions, for example, with reference to Antibodies: A Laboratory Manual, and Molecular Cloning: A Laboratory Manual published by Cold Spring Harbor Laboratory; or under the conditions recommended by the manufacturers of raw materials or commercial products. Reagents without specific sources indicated are commercially available conventional reagents.

### Example 1. Preparation of GLP-1 analogues

The GLP-1 analogue is compound 18# prepared in Example 1 of WO2023083301 (which is incorporated herein by reference in its entirety).

The molecular structure of compound 18# is: H-YAibEGTFTSDYSIYK(OEG-OEG-γGlu-C20-OH)EKIAAQEFVNWLLAGGPSSGAPPPS-NH₂

The structural formula is shown below:

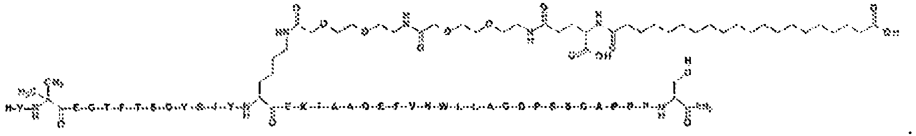

### Example 2. Study on safety, tolerability, pharmacokinetics and pharmacodynamics of GLP-1 analogue injections

A randomized, double-blind, dose-escalating, placebo-controlled Phase I clinical study was conducted in healthy subjects to evaluate the safety, tolerability, pharmacokinetic and pharmacodynamic profiles of single and multiple subcutaneous administrations of GLP-1 analogue injections

Following single subcutaneous injection of GLP-1 analogue injection in healthy subjects, the drug was slowly absorbed within the dose range of 0.1 to 8.1 mg, with a median time to peak of 48-72 h across all dose groups. After reaching the peak concentration, drug concentration declined in a mono-exponential elimination pattern. The arithmetic mean of the terminal elimination half-life (t_{1/2}) was 156-182 h, and the geometric mean of the mean residence time (MRT) was 251-288 h across all dose groups. On D22, after the fourth subcutaneous administration of 0.9 mg, 2.7 mg and 5.4 mg GLP-1 analogue injection in healthy subjects, slow drug absorption was observed, with a median time to peak of 48-72 h for all dose groups. Post-peak drug concentration exhibited mono-exponential elimination. The arithmetic mean of t_{1/2} was 169-192 h, and the geometric mean of MRT was 279-303 h. The geometric mean of steady-state apparent clearance (CLₛₛ/F) was 27.7-41.1 ml/h, and the geometric mean of steady-state apparent volume of distribution (Vₛₛ/F) was 7.61-10.2 L. The exposure accumulation ratio on D22 was approximately 2-fold for the 0.9 mg and 2.7 mg dose groups.

After weekly subcutaneous injection of the GLP-1 analogue for 4 consecutive weeks (D29, QW) in healthy subjects, the relative changes in body weight from baseline in the placebo group and the 0.9 mg, 2.7 mg, and 5.4 mg GLP-1 analogue injection groups (2.7/2.7/4.0/5.4 mg, titrated dosing) were -1.15 kg (-1.35%), -4.29 kg (-6.72%), -5.94 kg (-7.38%), and -7.73 kg (-9.63%), respectively. The weight-loss effect of each GLP-1 analogue dose group was significantly superior to that of the placebo group.

A total of 72 subjects received single or multiple administrations of the GLP-1 analogue at doses ranging from 0.1 to 8.1 mg in this Phase I study. Among the 72 subjects, the incidence of GLP-1 analogue-related adverse events (AEs) was 65.3% (47/72). The most common adverse events included abdominal distension and nausea during the single dose escalation stage, as well as urine ketone bodies and nausea during the multiple dose escalation stage. All adverse events were mild or moderate (predominantly mild). No severe hypoglycemia or serious adverse events occurred.

### Example 3. A multi-center, randomized, double-blind, placebo-controlled parallel Phase II clinical study to evaluate the efficacy and safety of GLP-1 analogue injection in obese, non-diabetic subjects

### 1. Investigational drug

1) The GLP-1 analogue prepared in Example 1, presented as an injection with a specification of 3ml : 30 mg.

### 2. Enrolled subjects

Subjects must meet all of the following criteria to be eligible for enrollment in this study:
1) Aged 18-65 years (inclusive), without restriction on gender;
2) Body mass index (BMI) at screening: 28.0-40.0 kg/m² (inclusive);
3) Self-reported diet and exercise intervention for 3 months or more prior to screening, with a body weight fluctuation (the difference between the maximum and minimum body weight within 3 months) not exceeding 5 kg in the latest 3 months.

### 3. Dosing regimen

The GLP-1 analogue or placebo was administered via subcutaneous injection into the abdomen, either before or after meals. The detailed dosing regimen is as follows:

**Table 1. Dosing regimens for the first treatment period (weeks 0-23) and the second treatment period (weeks 24-31)**

| Dosing week | 1.0 mg QW group | 3.0 mg QW group | 4.5 mg QW group | 6.0 mg QW group |
|---|---|---|---|---|
| 0-3 weeks | 0.5 mg | 1.0 mg | 1.0 mg | 1.5 mg |
| 4-7 weeks | 1.0 mg | 2.0 mg | 2.0 mg | 3.0 mg |
| 8-11 weeks | 1.0 mg | 3.0 mg | 3.0 mg | 4.5 mg |
| 12-15 weeks | 1.0 mg | 3.0 mg | 4.5 mg | 6.0 mg |
| 16-19 weeks | 1.0 mg | 3.0 mg | 4.5 mg | 6.0 mg |
| 20-23 weeks | 1.0 mg | 3.0 mg | 4.5 mg | 6.0 mg |
| 24-27 weeks | 1.0 mg | 3.0 mg | 4.5 mg | 6.0 mg |
| 28-31 weeks | 1.0 mg | 3.0 mg | 4.5 mg | 6.0 mg |

**Table 2. Dosing regimen for the second treatment period (weeks 32-51)**

| Dosing week | Placebo/ 3.0 mg QW group | 1.0 mg QW / 3.0 mg QW group | 3.0 mg QW/ 3.0 mg QW group | 3.0 mg QW/3.0 mg Q2W group | 4.5 mg QW/4.5 mg QW group | 4.5 mg QW/4.5 mg Q2W group | 6.0 mg QW/6.0 mg QW group | 6.0 mg QW/6.0 mg Q2W group |
|---|---|---|---|---|---|---|---|---|
| 32-35 weeks | 1.0 mg | 2.0 mg | 3.0 mg | 3.0 mg/2w | 4.5 mg | 4.5 mg/2w | 6.0 mg | 6.0 mg/2w |
| 36-39 weeks | 2.0 mg | 3.0 mg | 3.0 mg | 3.0 mg/2w | 4.5 mg | 4.5 mg/2w | 6.0 mg | 6.0 mg/2w |
| 40-43 weeks | 3.0 mg | 3.0 mg | 3.0 mg | 3.0 mg/2w | 4.5 mg | 4.5 mg/2w | 6.0 mg | 6.0 mg/2w |
| 44-47 weeks | 3.0 mg | 3.0 mg | 3.0 mg | 3.0 mg/2w | 4.5 mg | 4.5 mg/2w | 6.0 mg | 6.0 mg/2w |
| 48-51 weeks | 3.0 mg | 3.0 mg | 3.0 mg | 3.0 mg/2w | 4.5 mg | 4.5 mg/2w | 6.0 mg | 6.0 mg/2w |

### 4. Outcome evaluation

### 4.1 Efficacy evaluation

Efficacy assessment indicators include body weight, waist circumference, BMI, blood pressure (systolic blood pressure and diastolic blood pressure), glucose metabolism (fasting plasma glucose, fasting serum insulin, insulin resistance (HOMA model*), and HbA1c), and lipids (TC, LDLC, HDL-C, and TG).
*Note: Insulin resistance under the Homeostasis Model Assessment (HOMA) (HOMA-IR) is calculated using the following formula: $HOMA - IR = \left[Fasting plasma glucose \left(FPG, mmol/L\right)\times Fasting insulin \left(FINS, mIU/L\right)\right] / 22.5$

### 4.2 Safety evaluation

Safety evaluation indicators include adverse events, laboratory indicators, 12-lead electrocardiogram, vital signs, physical examination, PHQ-9 score, etc.

### 4.3 Results

A total of 249 Chinese adults with a body mass index of 28-40kg/m² received once-weekly subcutaneous injections of a GLP-1 analogue (1.0 mg QW group, 3.0 mg QW group, 4.5 mg QW group, 6.0 mg QW group) or placebo for 24 weeks (24W), in accordance with the dosing regimen for the first treatment period specified in Table 1.

At week 24, the mean percentage change in body weight from baseline was -5.4% (95% CI - 7.3% to -3.5%), -13.4% (-15.2% to -11.5%), -14.0% (-15.9% to -12.1%), and -16.8% (-18.8% to - 14.9%) in the 1.0 mg QW, 3.0 mg QW, 4.5 mg QW, and 6.0 mg QW groups, respectively, compared to -0.1% (-2.1% to 1.8%) in the placebo group (P < 0.0001 in all comparisons with placebo). The percentages of participants who experienced a weight loss of ≥5% in the 1.0 mg QW group, 3.0 mg QW group, 4.5 mg QW group, and 6.0 mg QW group were 52.0%, 88.2%, 92.0%, 91.8%, and 10.2%, respectively. In addition, GLP-1 analogues were more effective than the placebo in lowering blood pressure, regulating blood glucose levels, inhibiting insulin resistance, and reducing triglyceride levels. At W24, the least squares mean change in systolic blood pressure from baseline in the GLP-1 analogue groups was -4.46 to -8.33 mmHg (placebo: -0.41 mmHg), and the least squares mean change in waist circumference from baseline in the GLP-1 analogue groups was -5.14 to -12.73 cm (placebo: -1.82 cm).

The majority of adverse events (AEs) were mild to moderate in severity. The most frequently reported adverse events included nausea, diarrhea, decreased appetite and vomiting, which primarily occurred during the dose escalation phase. No treatment-related serious adverse events occurred, and no participants discontinued treatment due to treatment-related adverse events.

Conclusion: GLP-1 analogues can effectively reduce body weight, blood pressure, blood glucose and triglyceride levels with a favorable safety profile. These data support further clinical development of GLP-1 analogues for the treatment of obesity.

### Example 4. A multi-center, randomized, double-blind, placebo-controlled parallel Phase II clinical study to evaluate the efficacy and safety of GLP-1 analogue injections in subjects with type 2 diabetes

### 1. Investigational drug

1) The GLP-1 analogue prepared in Example 1, presented as an injection.

### 2. Enrolled subjects

Subjects must meet all of the following criteria to be eligible for this study:
1) Aged 18-65 years (inclusive), without restriction on gender;
2) Diagnosed with type 2 diabetes according to the criteria of the "Guidelines for the Prevention and Treatment of Type 2 Diabetes in China (2020 Edition)", with a duration of at least 6 months since meeting the diagnostic criteria;
3) Received lifestyle intervention alone for at least 8 weeks prior to screening, or maintained stable monotherapy with metformin (daily dose ≥ 1000 mg) for at least 8 weeks. Stable treatment is defined as no changes in medication type and daily dosage;
4) 7.5%≤HbA1c≤10.5% at screening;
5) Body mass index (BMI) at screening: 22.0-40.0kg/m² (inclusive);
6) Body weight fluctuation (the difference between the maximum and minimum body weight within 3 months) shall not exceed 5 kg in the 3 months before screening.

### 3. Dosing regimen

The investigational drug was administered via subcutaneous injection into the abdomen once a week, either before or after meals.

First treatment period (weeks 0-19): Subjects received GLP-1 analogue injection at doses of 1.0 mg, 2.0 mg, 3.0 mg and 4.5 mg, as well as placebo, for a 20-week treatment duration.

Second treatment period (weeks 20-31): The doses of GLP-1 analogue injection or placebo administered in the first treatment period were maintained. The original investigational product groups were additionally given placebo, and the original placebo group received an additional 1.0 mg of the investigational drug. The treatment lasted for 12 weeks.

The specific dosing regimen is as follows:

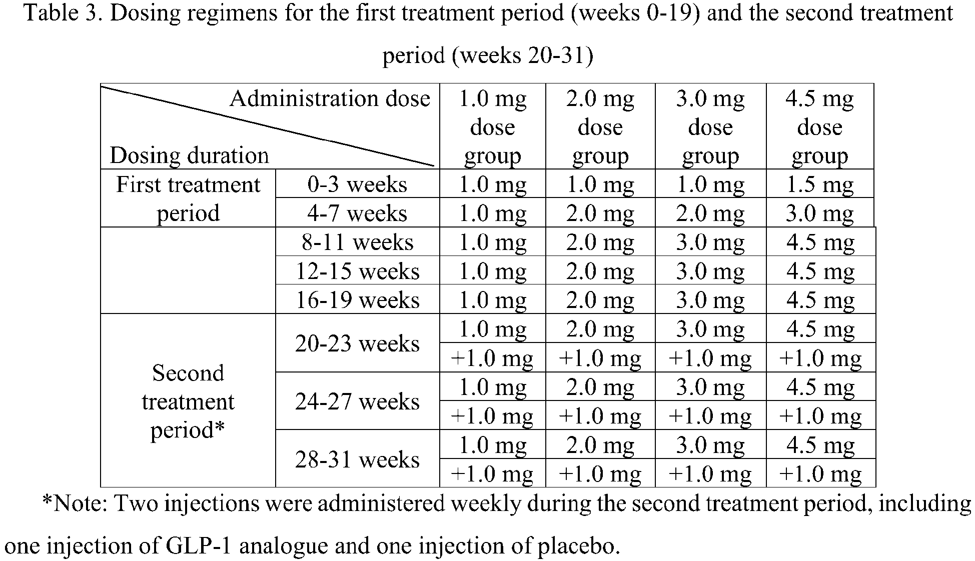

### 4. Outcome evaluation

### 4.1 Efficacy evaluation

Efficacy evaluation indicators include HbA1c, fasting and 2h post-mixed-meal plasma glucose, fasting and 2h post-mixed-meal serum insulin and C-peptide, pancreatic β-cell function, insulin resistance (HOMA model), 7-point SMBG profile, body weight, waist circumference, blood lipids (TC, LDL-C, HDL-C, TG), and blood pressure (systolic blood pressure, diastolic blood pressure), and the like.

### 4.2 Safety evaluation

Adverse events, hypoglycemic events, laboratory indicators, 12-lead electrocardiogram, vital signs, etc.

### 4.3 Results

A total of 199 subjects with type 2 diabetes were randomly assigned in a 1:1:1:1:1 ratio to receive once-weekly subcutaneous injections of GLP-1 analogue (1.0 mg, 2.0 mg, 3.0 mg, and 4.5 mg) or placebo for 20 weeks. The primary endpoint was the change in glycated hemoglobin (HbA_{1C}) relative to baseline after 20 weeks of administration.

The baseline HbA_{1C} levels were comparable across the five study groups, averaging between 8.2% and 8.7%. As of the data cutoff date (May 1, 2024), 187 out of 199 subjects (94.0%) completed the 20-week study treatment. At week 20, the least squares mean (LS Mean) changes in HbA_{1C} from baseline were -2.1% (95% CI -2.4% to -1.9%), -2.6% (95% CI -2.8% to -2.3%), - 2.7% (95% CI -2.9% to -2.4%), and -2.5% (95% CI -2.7% to -2.2%) in the GLP-1 analogue 1.0 mg, 2.0 mg, 3.0 mg, and 4.5 mg groups, respectively, compared to -0.3% (95% CI -0.6% to 0%) in the placebo group (one-sided p < 0.0001 for all comparison groups relative to placebo). At week 20, the percentages of subjects achieving the target HbA1c < 7.0% in the GLP-1 analogue groups (1.0 mg, 2.0 mg, 3.0 mg, and 4.5 mg) were 75.0%, 90.2%, 84.6%, and 90.0%, respectively (placebo: 12.8%); the percentages achieving HbA1c ≤ 6.5% were 62.5%, 80.5%, 69.2%, and 90.0% (placebo: 2.6%); and the percentages achieving HbA1c < 5.7% were 17.5%, 26.8%, 28.2%, and 35.0% (placebo: 0%).

Compared to baseline, the LS Mean changes in body weight at week 20 for the GLP-1 analogue 1.0 mg, 2.0 mg, 3.0 mg, and 4.5 mg groups were -3.1%, -4.9%, -5.5%, and -7.1%, respectively (placebo: -0.6%).

Compared to baseline, the LS Mean changes in urinary protein-to-creatinine ratio (UACR) at week 20 for the GLP-1 analogue groups of 1.0 mg, 2.0 mg, 3.0 mg, and 4.5 mg were -18.1%, - 30.5%, -46.5%, and -49.5% (placebo: 2.5%), respectively.

Compared to baseline, the LS Mean changes in triglycerides (TG) at week 20 in the GLP-1 analogue 1.0 mg, 2.0 mg, 3.0 mg, and 4.5 mg groups were -10.0%, -25.9%, -25.7%, and -27.9% (placebo: -6.9%), respectively.

Relative to baseline, the LS Mean changes in systolic blood pressure (SBP) at week 20 were - 8.3%, -7.4%, -10.0% and -6.7% in the 1.0 mg, 2.0 mg, 3.0 mg and 4.5 mg GLP-1 analogue groups, respectively (placebo group: -1.2%).

Furthermore, at week 20, all doses of the GLP-1 analogue group were superior to placebo in reducing fasting plasma glucose, 2h postprandial plasma glucose, 7-point self-monitored blood glucose, and systolic blood pressure.

Most adverse events in the GLP-1 analogue groups were mild or moderate, the most common being diarrhea, decreased appetite, nausea, abdominal distension, and upper respiratory tract infection, as shown in Table 4. The incidence of serious adverse events was comparable between the GLP-1 analogue group and the placebo group. No clinically significant hypoglycemia (< 3 mmol/L) or severe hypoglycemia was reported.

**Table 4: Adverse reactions**

| | GLP-1 analogue | | | | Placebo (N=39) |
|---|---|---|---|---|---|
| | 1.0 mg (N=40) | 2.0 mg (N=41) | 3.0 mg (N=39) | 4.5 mg (N=40) | |
| AE | 30 (75.0) | 35 (85.4) | 32 (82.1) | 34 (85.0) | 30 (76.9) |
| Drug-related gastrointestinal adverse reactions with an incidence of ≥5% during treatment | | | | | |
| Diarrhea | 6 (15.0) | 10 (24.4) | 10 (25.6) | 14 (35.0) | 2 (5.1) |
| Nausea | 4 (10.0) | 7 (17.1) | 9 (23.1) | 9 (22.5) | 0 |
| Abdominal distension | 2 (5.0) | 1 (2.4) | 3 (7.7) | 6 (15.0) | 0 |
| Vomiting | 2 (5.0) | 1 (2.4) | 3 (7.7) | 3 (7.5) | 0 |
| Flatulence | 1 (2.5) | 1 (2.4) | 0 | 2 (5.0) | 0 |
| Abdominal discomfort | 0 | 2 (4.9) | 0 | 2 (5.0) | 0 |
| Abdominal pain | 1 (2.5) | 0 | 0 | 2 (5.0) | 0 |
| Gastroesophag eal reflux disease | 0 | 0 | 0 | 3 (7.5) | 1 (2.6) |
| Hypoglycemia Grade 2-3 | 0 | 0 | 0 | 0 | 0 |

In the metformin subgroup, the LS Mean changes in HbA_{1C} from baseline at week 20 were - 2.0% (95% CI: -2.4% to -1.6%), -2.4% (95% CI: -2.8% to -2.0%), -2.9% (95% CI: -3.3% to - 2.5%), and -2.2% (95% CI: -2.6% to -1.8%) in the 1.0 mg, 2.0 mg, 3.0 mg, and 4.5 mg GLP-1 analogue groups, respectively, compared with -0.1% (95% CI: -0.4% to 0.5%) in the placebo group (one-sided p < 0.0001 for all comparisons versus placebo). In the non-metformin subgroup, the LS Mean changes in HbA_{1C} from baseline at week 20 were -2.3% (95% CI: -2.7% to -2.0%), -2.7% (95% CI: -3.1% to -2.4%), -2.5% (95% CI: -2.9% to -2.2%) and -2.7% (95% CI: -3.1% to -2.4%) in the 1.0 mg, 2.0 mg, 3.0 mg and 4.5 mg GLP-1 analogue groups, respectively, versus -0.7% (95% CI: -1.0% to 0.3%) in the placebo group (one-sided p < 0.0001 for all comparisons versus placebo).

Compared with placebo, the GLP-1 analogue demonstrated superior blood glucose control and weight reduction in patients with T2DM, with a favorable safety profile and a low risk of hypoglycemia. These results support the further clinical development of the GLP-1 analogue for the treatment of T2DM.

### Example 5. Study on the effect of GLP-1 analogue injection on metformin pharmacokinetics in healthy subjects

### 1. Investigational drug

1) The GLP-1 analogue prepared in Example 1, presented as an injection.
2) Commercially available metformin hydrochloride tablets (Glucophage), Merck Pharmaceutical Co., Ltd.

### 2. Enrolled subjects

Key inclusion criteria:
1. Healthy males aged 18 to 45 years (inclusive);
2. Males weighing ≥50kg, with body mass index (BMI) between 20.0 and 30.0 kg/m² (inclusive);
3. Glycated hemoglobin (HbA1c) < 6.0%.

### 3. Experimental objectives

### Primary study objective:

To evaluate the effect of a single subcutaneous injection of 2 mg GLP-1 analogue on metformin exposure in healthy subjects.

### Secondary study objectives:

To evaluate the effects of a single subcutaneous injection of 2 mg GLP-1 analogue injection on other pharmacokinetic (PK) parameters of metformin in healthy subjects.

To evaluate the safety of a single subcutaneous injection of 2 mg GLP-1 analogue in combination with metformin hydrochloride tablets in healthy subjects.

To characterize the pharmacokinetic profile of a single 2 mg subcutaneous injection of GLP-1 analogue injection in healthy subjects.

### 4. Dosing regimen

Metformin hydrochloride tablets (Glucophage): Administration was initiated on Day 1, administered orally twice daily (BID) at a dose of 500 mg per time after standard breakfast and dinner, for 3.5 consecutive days until after breakfast on Day 4. The second course of oral administration started after breakfast on Day 7 at the same dose of 500 mg per time, BID, and continued for 3.5 consecutive days until after breakfast on Day 10. Daily administration time was maintained as consistent as possible.

GLP-1 analogue injection of 2 mg was administered as a single subcutaneous injection on the morning of Day 7 (prior to oral administration of metformin hydrochloride tablets).

### 5. PK evaluation

The concentrations of metformin and GLP-1 analogues in plasma were determined using the validated liquid chromatography-tandem mass spectrometry (LC-MS/MS) method, and PK parameters were calculated using a standard non-compartmental model.

### 6. Safety

Adverse events, physical examination, vital signs, laboratory tests, and 12-lead electrocardiogram, etc.

### Example 6. A study comparing the efficacy and safety of GLP-1 analogues versus semaglutide in type 2 diabetes subjects with suboptimal blood glucose control treated with metformin monotherapy or in combination with a sodium-glucose cotransporter 2 (SGLT2) inhibitor

### 1. Investigational drug

1) The GLP-1 analogue prepared in Example 1, presented as an injection.
2) Commercially available semaglutide injection, Novo Nordisk, Denmark.

### 2. Trial population

Subjects with type 2 diabetes who had suboptimal blood glucose control on metformin monotherapy or in combination with SGLT2 inhibitors.

### 3. Enrolled subjects

### Key inclusion criteria:

1. Aged ≥ 18 years, regardless of gender;
2. Diagnosed with type 2 diabetes in accordance with the "Guidelines for the Prevention and Treatment of Type 2 Diabetes in China (2020 Edition)", and with a duration of >90 days since meeting the diagnostic criteria at screening;
3. Received stable treatment with the following antidiabetic medications from 3 months prior to screening until randomization: metformin alone, or metformin in combination with one SGLT2 inhibitor;
   Note: Stable treatment is defined as no changes in medication type and daily total dose. The metformin dose is ≥ 1500 mg/day or the maximum tolerated dose (≥ 1000 mg/day); the SGLT2 inhibitor dose is the recommended daily dose as per the package insert.
4. 7.5%≤HbA1c≤11.0% at screening;
5. Body mass index (BMI)≥24.0kg/m² at screening;
6. Body weight fluctuation (the difference between the maximum and minimum body weight within 3 months, including the screening visit) shall not exceed 5 kg in the 3 months prior to screening.

### 4. Dosing regimen

The GLP-1 analogue or semaglutide was administered via subcutaneous injection into the abdomen, thigh, or upper arm once a week (QW), either before or after meals.

The target doses for the first phase of treatment were 2 mg of GLP-1 analogue (titrated over 4 weeks), 4 mg of GLP-1 analogue (titrated over 12 weeks), and 1 mg of semaglutide (titrated over 8 weeks), for a total of 36 weeks.

During the second treatment phase, the dosage of the GLP-1 analogue injection/semaglutide used in the first treatment phase remained unchanged, and the treatment period lasted for 16 weeks. The specific dosing regimens for all of the groups are as follows:

**Table 5: Dosing regimen**

| Dosing duration | GLP-1 analogue dose group | | Semaglutide |
|---|---|---|---|
| | 2 mg group | 4 mg group | 1 mg group |
| First Treatment Period - titration period (maximum 12 weeks) | | | |
| W0-W3 (weeks 1-4, 4 weeks in total) | 1 mg QW | 1 mg QW | 0.25 mg QW |
| W4-W7 (weeks 5-8, 4 weeks in total) | 2 mg QW | 2 mg QW | 0.5 mg QW |
| W8-W11 (weeks 9-12, 4 weeks in total) | 2 mg QW | 3 mg QW | 1 mg QW |

| First treatment period - maintenance period (24 weeks in total) | | | |
|---|---|---|---|
| W12-W35 (weeks 13-36, a total of 24 weeks) | 2 mg QW | 4 mg QW | 1 mg QW |

| Second treatment period (16 weeks in total) | | | |
|---|---|---|---|
| W36-W51 (weeks 37-52, 16 weeks in total) | 2 mg QW | 4 mg QW | 1 mg QW |

| | | | |
|---|---|---|---|
| Note: QW = once a week | | | |

### 5. Outcome assessments

### 5.1 Efficacy assessments

Efficacy evaluation indicators include HbA1c, fasting and 2h mixed meal post-mixed-meal plasma glucose, fasting and 2h mixed meal post-mixed-meal serum insulin and C-peptide, pancreatic β-cell function (HOMA-β), insulin resistance (HOMA-IR), mean 7-point self-monitored blood glucose (SMBG), body weight, waist circumference, BMI, blood pressure (systolic and diastolic blood pressure), and blood lipids (TG, TC, LDL-C, non-HDL-C, HDL-C), and the like.

### 5.2 Safety evaluation

Safety evaluation indicators cover AE, hypoglycemic episodes, laboratory parameters, electrocardiogram, vital signs, physical examination, etc.

### Example 7. A multi-center, randomized, double-blind, placebo-controlled parallel Phase III clinical study to evaluate the efficacy and safety of GLP-1 analogue injection in overweight or obese subjects

### 1. Investigational drug

1) The GLP-1 analogue prepared in Example 1, presented as an injection with a specification of 3 ml: 12 mg, 3 ml: 24 mg, and 3 ml: 30 mg.

### 2. Enrolled subjects

Subjects must meet all of the following criteria to be eligible for this study:
1) Aged ≥ 18 years, regardless of gender;
2) Met the following BMI criteria at screening:
   ≥28.0 kg/m², or
   ≥24.0 kg/m² and accompanied by at least one weight-related comorbidity;
3) Self-reported diet and exercise intervention for 3 months or more prior to screening, with a body weight fluctuation (the difference between the maximum and minimum body weight within 3 months) ≤5kg in the latest 3 months.

### 3. Dosing regimen

The GLP-1 analogue injection or placebo was administered subcutaneously in the abdomen, lateral thigh or lateral upper arm, either before or after meals. The specific dosage regimen is presented in Table 6 below:

**Table 6. Dosing regimen**

| Dosing duration | 2.0 mg dose group | 4.0 mg dose group | 6.0 mg dose group |
|---|---|---|---|
| First treatment period | | | |
| 4 weeks | 1.0 mg QW | 1.0 mg QW | 1.0 mg QW |
| 4 weeks | 2.0 mg QW | 2.0 mg QW | 2.0 mg QW |
| 4 weeks | 2.0 mg QW | 3.0 mg QW | 3.0 mg QW |
| 4 weeks | 2.0 mg QW | 4.0 mg QW | 4.0 mg QW |
| 4 weeks | 2.0 mg QW | 4.0 mg QW | 5.0 mg QW |
| 4 weeks | 2.0 mg QW | 4.0 mg QW | 6.0 mg QW |

| Second treatment period | | | |
|---|---|---|---|
| 24 weeks | 2.0 mg QW | 4.0 mg QW | 6.0 mg QW |

### 4. Outcome evaluation

### 4.1 Efficacy evaluation

Efficacy evaluation indicators include body weight, waist circumference, BMI, blood pressure (systolic blood pressure, diastolic blood pressure), blood lipids (TG, TC, LDL-C, non-HDL-C, HDL-C), glucose metabolism (fasting plasma glucose, fasting serum insulin, HbA1c, HOMA-IR), physiological function score (SF-36v2), hsCRP, total fat mass, lean body mass, and UACR.
*Note: Insulin resistance under the Homeostasis Model Assessment (HOMA) (HOMA-IR) is calculated using the following formula: $HOMA - IR = \left[Fasting plasma glucose \left(FPG, mmol/L\right)\times Fasting insulin \left(FINS, mIU/L\right)\right] / 22.5$

### 4.2 Safety evaluation

Safety evaluation indicators include adverse events, vital signs, physical examination, laboratory indicators, 12-lead electrocardiogram, PHQ-9 scores, etc.

### Example 8. A multi-center, randomized, double-blind, placebo-controlled parallel clinical study to evaluate the efficacy and safety of GLP-1 analogue injection in overweight or obese subjects

### 1. Investigational drug

1) The GLP-1 analogue prepared in Example 1, presented as an injection.

### 2. Trial population

Subjects who are overweight with at least one weight-related comorbidity or obese subjects who had suboptimal response to dietary and lifestyle intervention alone.

### 3. Enrolled subjects

Subjects must meet all of the following criteria to be eligible for this study:
1) Aged 18-65 years (inclusive), without restriction on gender;
2) Met the following body mass index (BMI) criteria at screening:
   -28.0-40.0 kg/m² (inclusive), or
   -24.0 (inclusive) - 28.0 (exclusive) kg/m² and having at least one weight-related comorbidity
3) Self-reported diet and exercise intervention for 3 months or more prior to screening, with a body weight fluctuation (the difference between the maximum and minimum body weight within 3 months) not exceeding 5 kg in the latest 3 months;

### 3. Dosing regimen

The GLP-1 analogue injection or placebo was administered via abdominal subcutaneous injection once a week, either before or after meals. GLP-1 analogue injection or placebo was administered starting at 1.0 mg QW, with dose escalation every 4 weeks, gradually titrated to reach the target dose of 8.0 mg QW. The specific dosing regimen is shown in Table 7 below:

**Table 7: Dosing regimen**

| Dosing duration | Administration dose |
|---|---|
| Titration period 24 weeks | |
| 4 weeks | 1.0 mg QW |
| 4 weeks | 2.0 mg QW |
| 4 weeks | 3.0 mg QW |
| 4 weeks | 4.0 mg QW |
| 4 weeks | 5.0 mg QW |
| 4 weeks | 6.0 mg QW |

| Maintenance period 12 weeks | |
|---|---|
| 12 weeks | 8.0 mg QW |

### 4. Outcome evaluation

### 4.1 Efficacy evaluation

Efficacy evaluation indicators include body weight, waist circumference, BMI, blood pressure (systolic and diastolic blood pressure), glucose metabolism (fasting plasma glucose, fasting serum insulin, HbA1c), and blood lipids (TG, TC, LDL-C, non-HDL-C, HDL-C).

### 4.2 Safety evaluation

Safety evaluation indicators include adverse events, laboratory indicators, 12-lead electrocardiogram, vital signs, physical examination, PHQ-9 score, etc.

### 4.3 Results

After 36 weeks of administration, the percentage change in body weight from baseline in the GLP-1 analogue 8.0 mg group was -22.82%, and the least squares mean difference compared with the placebo group was -21.09%, which was significantly better than placebo.

After 36 weeks of administration, 59% of the subjects in the GLP-1 analogue 8.0 mg group achieved a weight loss of ≥20%.

The 8.0 mg group of GLP-1 analogues showed good safety. Most adverse events were mild. Gastrointestinal-related adverse events mainly occurred during the dose escalation period.

## Claims

1. A method for preventing or treating metabolic diseases, comprising administering a target drug to a subject in need thereof approximately once a week, wherein the dosing regimen of the target drug comprises:
administering an initial dose of the target drug once a week for at least about 1 week, followed by,
optionally, administering an escalating dose of the target drug once a week for at least about 1 week, followed by,
administering a maintenance dose of the target drug once a week for at least about 2 weeks;
wherein the maintenance dose is selected from about 0.5 mg to about 10.0 mg, or about 1.0 mg to about 8.0 mg; preferably about 1.0 mg, about 2.0 mg, about 3.0 mg, about 4.0 mg, about 4.5 mg, about 6.0 mg or about 8.0 mg;
wherein the target drug is selected from GLP-1 analogues or pharmaceutically acceptable salts thereof.

2. The method according to claim 1, wherein the target drug is a dual agonist of GLP-1 receptor and GIP receptor, preferably a GLP-1 analogue comprising the structure shown below or a pharmaceutically acceptable salt thereof,
H-YAibEGTFTSDYSIYK(OEG-OEG-γGlu-C20-OH)EKIAAQEFVNWLLAGGPSSGAPPP S-NH₂.

3. The method according to claim 1 or 2, wherein the initial dose is selected from about 0.1 mg to about 2.5 mg, or about 0.5 mg to about 1.5 mg; preferably about 0.5 mg, about 1.0 mg or about 1.5 mg.

4. The method according to any one of claims 1 to 3, wherein the escalating dose is selected from about 0.5 mg to about 10.0 mg, about 1.0 mg to about 6.0 mg; preferably about 2.0 mg, about 3.0 mg, about 4.0 mg, about 4.5 mg, about 5.0 mg or about 6.0 mg.

5. The method according to any one of claims 1 to 4, wherein the escalating dose includes at least one of a second dose, a third dose, a fourth dose, a fifth dose, and a sixth dose;
the second dose is selected from about 1.0 mg to about 5.0 mg, or about 2.0 mg to about 3.0 mg; preferably about 2.0 mg or about 3.0 mg;
the third dose is selected from about 1.0 mg to about 7.5 mg, or about 3.0 mg to about 4.5 mg; preferably about 3.0 mg or about 4.5 mg;
the fourth dose is selected from about 1.0 mg to about 9.0 mg, or about 4.0 mg to about 5.0 mg; preferably about 4.0 mg;
the fifth dose is selected from about 1.0 mg to about 11.0 mg, or about 5.0 mg to about 6.0 mg; preferably about 5.0 mg;
the sixth dose is selected from about 1.0 mg to about 11.0 mg, or about 6.0 mg to about 7.0 mg; preferably about 6.0 mg.

6. The method according to any one of claims 1 to 5, wherein
the initial dose of the target drug is administered for at least about 2 weeks, preferably about 4 weeks; and/or,
the escalating dose of the target drug is administered for at least about 2 weeks, preferably about 4 weeks; and/or,
the maintenance dose of the target drug is administered for at least about 4 weeks.

7. The method according to any one of claims 1 to 6, wherein the dosing regimen of the target drug comprises the following regimens:
a) administering the target drug at a dose of about 1.0 mg once a week for at least about 2 weeks; followed by
d) administering the target drug at a dose of about 1.0 mg once a week for at least about 4 weeks;
or,
a) administering the target drug at a dose of about 0.5 mg once a week for at least about 2 weeks, followed by
d) administering the target drug at a dose of about 1.0 mg once a week for at least about 4 weeks;
or,
a) administering the target drug at a dose of about 1.0 mg once a week for at least about 2 weeks, followed by
d) administering the target drug at a dose of about 2.0 mg once a week for at least about 4 weeks;
or,
a) administering the target drug at a dose of about 1.0 mg once a week for at least about 2 weeks, followed by
b) administering the target drug at a dose of about 2.0 mg once a week for at least about 2 weeks, followed by
d) administering the target drug at a dose of about 3.0 mg once a week for at least about 4 weeks;
or,
a) administering the target drug at a dose of about 1.0 mg once a week for at least about 2 weeks, followed by
b) administering the target drug at a dose of about 2.0 mg once a week for at least about 2 weeks, followed by
c) administering the target drug at a dose of about 3.0 mg once a week for at least about 2 weeks, followed by
d) administering the target drug at a dose of about 4.5 mg once a week for at least about 4 weeks;
or
a) administering the target drug at a dose of about 1.0 mg once a week for at least about 2 weeks, followed by
b) administering the target drug at a dose of about 2.0 mg once a week for at least about 2 weeks, followed by
c) administering the target drug at a dose of about 3.0 mg once a week for at least about 2 weeks, followed by
d) administering the target drug at a dose of about 4.0 mg once a week for at least about 4 weeks;
or,
a) administering the target drug at a dose of about 1.5 mg once a week for at least about 2 weeks, followed by
b) administering the target drug at a dose of about 3.0 mg once a week for at least about 2 weeks, followed by
d) administering the target drug at a dose of about 4.5 mg once a week for at least about 4 weeks;
or,
a) administering the target drug at a dose of about 1.5 mg once a week for at least about 2 weeks, followed by
b) administering the target drug at a dose of about 3.0 mg once a week for at least about 2 weeks, followed by
c) administering the target drug at a dose of about 4.5 mg once a week for at least about 2 weeks, followed by
d) administering the target drug at a dose of about 6.0 mg once a week for at least about 4 weeks;
or,
a) administering the target drug at a dose of about 1.0 mg once a week for at least about 2 weeks, followed by
b) administering the target drug at a dose of about 2.0 mg once a week for at least about 2 weeks, followed by
c) administering the target drug at a dose of about 3.0 mg once a week for at least about 2 weeks, followed by
administering the target drug at a dose of about 4.0 mg once a week for at least about 2 weeks, followed by
administering the target drug at a dose of about 5.0 mg once a week for at least about 2 weeks, followed by
d) administering the target drug at a dose of about 6.0 mg once a week for at least about 4 weeks;
or,
a) administering the target drug at a dose of about 1.0 mg once a week for at least about 2 weeks, followed by
b) administering the target drug at a dose of about 2.0 mg once a week for at least about 2 weeks, followed by
c) administering the target drug at a dose of about 3.0 mg once a week for at least about 2 weeks, followed by
administering the target drug at a dose of about 4.0 mg once a week for at least about 2 weeks, followed by
administering the target drug at a dose of about 5.0 mg once a week for at least about 2 weeks, followed by
administering the target drug at a dose of about 6.0 mg once a week for at least about 2 weeks, followed by
d) administering the target drug at a dose of about 8.0 mg once a week for at least about 4 weeks.

8. The method according to any one of claims 1 to 7, wherein the dosing regimen of the target drug comprises the following regimens:
a) administering the target drug at a dose of about 1.0 mg once a week for at least about 4 weeks; followed by
d) administering the target drug at a dose of about 1.0 mg once a week for at least about 4 weeks, such as about 20 weeks;
or,
a) administering the target drug at a dose of about 0.5 mg once a week for at least about 4 weeks, followed by
d) administering the target drug at a dose of about 1.0 mg once a week for at least about 4 weeks, such as about 20 weeks;
or,
a) administering the target drug at a dose of about 1.0 mg once a week for at least about 4 weeks, followed by
d) administering the target drug at a dose of about 2.0 mg once a week for at least about 4 weeks, such as about 20 weeks;
or,
a) administering the target drug at a dose of about 1.0 mg once a week for at least about 4 weeks, followed by
b) administering the target drug at a dose of about 2.0 mg once a week for at least about 4 weeks, followed by
d) administering the target drug at a dose of about 3.0 mg once a week for at least about 4 weeks, such as about 16 weeks;
or,
a) administering the target drug at a dose of about 1.0 mg once a week for at least about 4 weeks, followed by
b) administering the target drug at a dose of about 2.0 mg once a week for at least about 4 weeks, followed by
c) administering the target drug at a dose of about 3.0 mg once a week for at least about 4 weeks, followed by
d) administering the target drug at a dose of about 4.5 mg once a week for at least about 4 weeks, such as about 12 weeks;
or,
a) administering the target drug at a dose of about 1.5 mg once a week for at least about 4 weeks, followed by
b) administering the target drug at a dose of about 3.0 mg once a week for at least about 4 weeks, followed by
d) administering the target drug at a dose of about 4.5 mg once a week for at least about 4 weeks, such as about 16 weeks;
or,
a) administering the target drug at a dose of about 1.5 mg once a week for at least about 4 weeks, followed by
b) administering the target drug at a dose of about 3.0 mg once a week for at least about 4 weeks, followed by
c) administering the target drug at a dose of about 4.5 mg once a week for at least about 4 weeks, followed by
d) administering the target drug at a dose of about 6.0 mg once a week for at least about 4 weeks, such as about 12 weeks;
or,
a) administering the target drug at a dose of about 1.0 mg once a week for at least about 4 weeks, followed by
b) administering the target drug at a dose of about 2.0 mg once a week for at least about 4 weeks, followed by
c) administering the target drug at a dose of about 3.0 mg once a week for at least about 4 weeks, followed by
d) administering the target drug at a dose of about 4.0 mg once a week for at least about 4 weeks, such as about 12 weeks;
or,
a) administering the target drug at a dose of about 1.0 mg once a week for at least about 4 weeks, followed by
b) administering the target drug at a dose of about 2.0 mg once a week for at least about 4 weeks, followed by
c) administering the target drug at a dose of about 3.0 mg once a week for at least about 4 weeks, followed by
administering the target drug at a dose of about 4.0 mg once a week for at least about 4 weeks, followed by
administering the target drug at a dose of about 5.0 mg once a week for at least about 4 weeks, followed by
d) administering the target drug at a dose of about 6.0 mg once a week for at least about 4 weeks, such as about 4 weeks;
or,
a) administering the target drug at a dose of about 1.0 mg once a week for at least about 4 weeks, followed by
b) administering the target drug at a dose of about 2.0 mg once a week for at least about 4 weeks, followed by
c) administering the target drug at a dose of about 3.0 mg once a week for at least about 4 weeks, followed by
administering the target drug at a dose of about 4.0 mg once a week for at least about 4 weeks, followed by
administering the target drug at a dose of about 5.0 mg once a week for at least about 4 weeks, followed by
administering the target drug at a dose of about 6.0 mg once a week for at least about 4 weeks, followed by
d) administering the target drug at a dose of about 8.0 mg once a week for at least about 4 weeks, such as about 12 weeks.

9. The method according to any one of claims 1 to 8, wherein the dosing regimen further comprises a second treatment period, the second treatment period comprising:
1) administering a maintenance dose of the target drug once a week for at least about 1 week;
or,
2) administering a maintenance dose of the target drug once every two weeks for at least about 2 weeks; or,
3) administering a maintenance dose of the target drug once a week for at least about 1 week, followed by
administering a maintenance dose of the target drug once every two weeks for at least about 2 weeks; or
4) administering a maintenance dose of the target drug once a week for at least about 1 week, followed by
administering a seventh dose of the target drug once a week for at least about 1 week, followed by
administering an eighth dose of the target drug once a week for at least about 2 weeks;
preferably, the maintenance dose is selected from about 0.5 mg to about 10.0 mg, or about 1.0 mg to about 8.0 mg; preferably about 1.0 mg, about 2.0 mg, about 3.0 mg, about 4.0 mg, about 4.5 mg, about 6.0 mg or about 8.0 mg;
Preferably, the seventh dose and the eighth dose are each independently selected from about 0.5 mg to about 10.0 mg, about 1.0 mg to about 5.0 mg, or about 2.0 mg to about 3.0 mg; preferably about 2.0 mg or about 3.0 mg.

10. The method according to any one of claims 1 to 9, wherein the second treatment period comprises the following regimens:
e) administering the target drug at a dose of about 3.0 mg once a week for at least about 2 weeks, followed by
f) administering the target drug at a dose of about 3.0 mg once every two weeks for at least about 4 weeks;
or,
e) administering the target drug at a dose of about 4.5 mg once a week for at least about 2 weeks, followed by
f) administering the target drug at a dose of about 4.5 mg once every two weeks for at least about 4 weeks;
or,
e) administering the target drug at a dose of about 6.0 mg once a week for at least about 2 weeks, followed by
f) administering the target drug at a dose of about 6.0 mg once every two weeks for at least about 4 weeks;
or,
e) administering the target drug at a dose of about 1.0 mg once a week for at least about 4 weeks;
or,
e) administering the target drug at a dose of about 2.0 mg once a week for at least about 4 weeks;
or,
e) administering the target drug at a dose of about 3.0 mg once a week for at least about 4 weeks;
or,
e) administering the target drug at a dose of about 4.0 mg once a week for at least about 4 weeks;
or,
e) administering the target drug at a dose of about 4.5 mg once a week for at least about 4 weeks;
or,
e) administering the target drug at a dose of about 6.0 mg once a week for at least about 4 weeks;
or,
e) administering the target drug at a dose of about 1.0 mg once a week for at least about 2 weeks, followed by
g) administering the target drug at a dose of about 2.0 mg once a week for at least about 2 weeks, followed by
h) administering the target drug at a dose of about 3.0 mg once a week for at least about 4 weeks.

11. The method according to any one of claims 1 to 10, wherein the second treatment period comprises the following regimens:
e) administering the target drug at a dose of about 3.0 mg once a week for at least about 4 weeks, such as about 8 weeks, followed by
f) administering the target drug at a dose of about 3.0 mg once every two weeks for at least about 4 weeks, such as about 20 weeks;
or,
e) administering the target drug at a dose of about 4.5 mg once a week for at least about 4 weeks, such as about 8 weeks, followed by
f) administering the target drug at a dose of about 4.5 mg once every two weeks for at least about 4 weeks, such as about 20 weeks;
or,
e) administering the target drug at a dose of about 6.0 mg once a week for at least about 4 weeks, such as about 8 weeks, followed by
f) administering the target drug at a dose of about 6.0 mg once every two weeks for at least about 4 weeks, such as about 20 weeks;
or,
e) administering the target drug at a dose of about 1.0 mg once a week for at least about 4 weeks, such as about 8 weeks;
or,
e) administering the target drug at a dose of about 2.0 mg once a week for at least about 4 weeks, such as about 8 weeks, about 24 weeks, or about 28 weeks;
or,
e) administering the target drug at a dose of about 3.0 mg once a week for at least about 4 weeks, such as about 8 week, about 28 weeks;
or,
e) administering the target drug at a dose of about 4.0 mg once a week for at least about 4 weeks, such as about 24 weeks or about 28 weeks;
or,
e) administering the target drug at a dose of about 4.5 mg once a week for at least about 4 weeks, such as about 8 week or about 28 weeks;
or,
e) administering the target drug at a dose of about 6.0 mg once a week for at least about 4 weeks, such as about 24 weeks or about 28 weeks;
or,
e) administering the target drug at a dose of about 1.0 mg once a week for at least about 4 weeks, such as about 8 weeks, followed by
g) administering the target drug at a dose of about 2.0 mg once a week for at least about 4 weeks, such as about 4 weeks, followed by
h) administering the target drug at a dose of about 3.0 mg once a week for at least about 4 weeks, such as about 16 weeks.

12. The method according to any one of claims 1 to 11, comprising any of the following regimens:
(1) administering the target drug at a dose of about 0.5 mg once a week for at least about 2 weeks, followed by
administering the target drug at a dose of about 1.0 mg once a week for at least about 4 weeks, followed by
administering the target drug at a dose of about 1.0 mg once a week for at least about 2 weeks, followed by
administering the target drug at a dose of about 2.0 mg once a week for at least about 2 weeks, followed by
administering the target drug at a dose of about 3.0 mg once a week for at least about 4 weeks;
(2) administering the target drug at a dose of about 1.0 mg once a week for at least about 2 weeks, followed by
administering the target drug at a dose of about 2.0 mg once a week for at least about 2 weeks, followed by
administering the target drug at a dose of about 3.0 mg once a week for at least about 4 weeks, followed by
administering the target drug at a dose of about 3.0 mg once a week for at least about 2 weeks, followed by
administering the target drug at a dose of about 3.0 mg once every two weeks for at least about 4 weeks;
(3) administering the target drug at a dose of about 1.0 mg once a week for at least about 2 weeks, followed by
administering the target drug at a dose of about 2.0 mg once a week for at least about 2 weeks, followed by
administering the target drug at a dose of about 3.0 mg once a week for at least about 4 weeks, followed by
administering the target drug at a dose of about 3.0 mg once a week for at least about 4 weeks;
(4) administering the target drug at a dose of about 1.0 mg once a week for at least about 2 weeks, followed by
administering the target drug at a dose of about 2.0 mg once a week for at least about 2 weeks, followed by
administering the target drug at a dose of about 3.0 mg once a week for at least about 2 weeks, followed by
administering the target drug at a dose of about 4.5 mg once a week for at least about 4 weeks, followed by
administering the target drug at a dose of about 4.5 mg once a week for at least about 2 weeks, followed by
administering the target drug at a dose of about 4.5 mg once every two weeks for at least about 4 weeks;
(5) administering the target drug at a dose of about 1.0 mg once a week for at least about 2 weeks, followed by
administering the target drug at a dose of about 2.0 mg once a week for at least about 2 weeks, followed by
administering the target drug at a dose of about 3.0 mg once a week for at least about 2 weeks, followed by
administering the target drug at a dose of about 4.5 mg once a week for at least about 4 weeks, followed by
administering the target drug at a dose of about 4.5 mg once a week for at least about 4 weeks;
(6) administering the target drug at a dose of about 1.5 mg once a week for at least about 2 weeks, followed by
administering the target drug at a dose of about 3.0 mg once a week for at least about 2 weeks, followed by
administering the target drug at a dose of about 4.5 mg once a week for at least about 2 weeks, followed by
administering the target drug at a dose of about 6.0 mg once a week for at least about 4 weeks, followed by
administering the target drug at a dose of about 6.0 mg once a week for at least about 2 weeks, followed by
administering the target drug at a dose of about 6.0 mg once every two weeks for at least about 4 weeks;
(7) administering the target drug at a dose of about 1.5 mg once a week for at least about 2 weeks, followed by
administering the target drug at a dose of about 3.0 mg once a week for at least about 2 weeks, followed by
administering the target drug at a dose of about 4.5 mg once a week for at least about 2 weeks, followed by
administering the target drug at a dose of about 6.0 mg once a week for at least about 4 weeks, followed by
administering the target drug at a dose of about 6.0 mg once a week for at least about 4 weeks;
(8) administering the target drug at a dose of about 1.0 mg once a week for at least about 2 weeks, followed by
administering the target drug at a dose of about 1.0 mg once a week for at least about 2 weeks, followed by
administering the target drug at a dose of about 1.0 mg once a week for at least about 4 weeks;
(9) administering the target drug at a dose of about 1.0 mg once a week for at least about 2 weeks, followed by
administering the target drug at a dose of about 2.0 mg once a week for at least about 2 weeks, followed by
administering the target drug at a dose of about 2.0 mg once a week for at least about 4 weeks;
(10) administering the target drug at a dose of about 1.5 mg once a week for at least about 2 weeks, followed by
administering the target drug at a dose of about 3.0 mg once a week for at least about 2 weeks, followed by
administering the target drug at a dose of about 4.5 mg once a week for at least about 4 weeks, followed by
administering the target drug at a dose of about 4.5 mg once a week for at least about 4 weeks;
(11) administering the target drug at a dose of about 1.0 mg once a week for at least about 2 weeks, followed by
administering the target drug at a dose of about 2.0 mg once a week for at least about 2 weeks, followed by
administering the target drug at a dose of about 3.0 mg once a week for at least about 2 weeks, followed by
administering the target drug at a dose of about 4.0 mg once a week for at least about 4 weeks, followed by
administering the target drug at a dose of about 4.0 mg once a week for at least about 4 weeks;
(12) administering the target drug at a dose of about 1.0 mg once a week for at least about 2 weeks, followed by
administering the target drug at a dose of about 2.0 mg once a week for at least about 2 weeks, followed by
administering the target drug at a dose of about 3.0 mg once a week for at least about 2 weeks, followed by
administering the target drug at a dose of about 4.0 mg once a week for at least about 2 weeks, followed by
administering the target drug at a dose of about 5.0 mg once a week for at least about 2 weeks, followed by
administering the target drug at a dose of about 6.0 mg once a week for at least about 2 weeks, followed by
administering the target drug at a dose of about 6.0 mg once a week for at least about 4 weeks.

13. The method according to any one of claims 1 to 12, comprising any of the following regimens:
(1) administering the target drug at a dose of about 0.5 mg once a week for at least about 4 weeks, followed by
administering the target drug at a dose of about 1.0 mg once a week for at least about 4 weeks, such as about 20 weeks, followed by
administering the target drug at a dose of about 1.0 mg once a week for at least about 4 weeks, such as about 8 weeks, followed by
administering the target drug at a dose of about 2.0 mg once a week for at least about 4 weeks, followed by
administering the target drug at a dose of about 3.0 mg once a week for at least about 4 weeks, such as about 16 weeks;
(2) administering the target drug at a dose of about 1.0 mg once a week for at least about 4 weeks, followed by
administering the target drug at a dose of about 2.0 mg once a week for at least about 4 weeks, followed by
administering the target drug at a dose of about 3.0 mg once a week for at least about 4 weeks, such as about 16 weeks, followed by
administering the target drug at a dose of about 3.0 mg once a week for at least about 4 weeks, such as about 8 weeks, followed by
administering the target drug at a dose of about 3.0 mg once every two weeks for at least about 4 weeks, such as about 20 weeks;
(3) administering the target drug at a dose of about 1.0 mg once a week for at least about 4 weeks, followed by
administering the target drug at a dose of about 2.0 mg once a week for at least about 4 weeks, followed by
administering the target drug at a dose of about 3.0 mg once a week for at least about 4 weeks, such as about 16 weeks, followed by
administering the target drug at a dose of about 3.0 mg once a week for at least about 4 weeks, such as about 8 week or about 28 weeks;
(4) administering the target drug at a dose of about 1.0 mg once a week for at least about 4 weeks, followed by
administering the target drug at a dose of about 2.0 mg once a week for at least about 4 weeks, followed by
administering the target drug at a dose of about 3.0 mg once a week for at least about 4 weeks, followed by
administering the target drug at a dose of about 4.5 mg once a week for at least about 12 weeks, followed by
administering the target drug at a dose of about 4.5 mg once a week for at least about 4 weeks, such as about 8 weeks, followed by
administering the target drug at a dose of about 4.5 mg once every two weeks for at least about 4 weeks, such as about 20 weeks;
(5) administering the target drug at a dose of about 1.0 mg once a week for at least about 4 weeks, followed by
administering the target drug at a dose of about 2.0 mg once a week for at least about 4 weeks, followed by
administering the target drug at a dose of about 3.0 mg once a week for at least about 4 weeks, followed by
administering the target drug at a dose of about 4.5 mg once a week for at least about 4 weeks, such as about 12 weeks, followed by
administering the target drug at a dose of about 4.5 mg once a week for at least about 4 weeks, such as about 28 weeks;
(6) administering the target drug at a dose of about 1.5 mg once a week for at least about 4 weeks, followed by
administering the target drug at a dose of about 3.0 mg once a week for at least about 4 weeks, followed by
administering the target drug at a dose of about 4.5 mg once a week for at least about 4 weeks, followed by
administering the target drug at a dose of about 6.0 mg once a week for at least about 4 weeks, such as about 12 weeks, followed by
administering the target drug at a dose of about 6.0 mg once a week for at least about 4 weeks, such as about 8 weeks, followed by
administering the target drug at a dose of about 6.0 mg once every two weeks for at least about 4 weeks, such as about 20 weeks;
(7) administering the target drug at a dose of about 1.5 mg once a week for at least about 4 weeks, followed by
administering the target drug at a dose of about 3.0 mg once a week for at least about 4 weeks, followed by
administering the target drug at a dose of about 4.5 mg once a week for at least about 4 weeks, followed by
administering the target drug at a dose of about 6.0 mg once a week for at least about 4 weeks, such as about 12 weeks, followed by
administering the target drug at a dose of about 6.0 mg once a week for at least about 4 weeks, such as about 28 weeks;
(8) administering the target drug at a dose of about 1.0 mg once a week for at least about 4 weeks, followed by
administering the target drug at a dose of about 1.0 mg once a week for at least about 4 weeks, such as about 20 weeks, followed by
administering the target drug at a dose of about 1.0 mg once a week for at least about 4 weeks, such as about 8 weeks;
(9) administering the target drug at a dose of about 1.0 mg once a week for at least about 4 weeks, followed by
administering the target drug at a dose of about 2.0 mg once a week for at least about 4 weeks, such as about 20 weeks, followed by
administering the target drug at a dose of about 2.0 mg once a week for at least about 4 weeks, such as about 8 weeks, about 24 weeks, or about 28 weeks;
(10) administering the target drug at a dose of about 1.5 mg once a week for at least about 4 weeks, followed by
administering the target drug at a dose of about 3.0 mg once a week for at least about 4 weeks, followed by
administering the target drug at a dose of about 4.5 mg once a week for at least about 4 weeks, such as about 16 weeks, followed by
administering the target drug at a dose of about 4.5 mg once a week for at least about 4 weeks, such as about 8 weeks;
(11) administering the target drug at a dose of about 1.0 mg once a week for at least about 4 weeks, followed by
administering the target drug at a dose of about 2.0 mg once a week for at least about 4 weeks, followed by
administering the target drug at a dose of about 3.0 mg once a week for at least about 4 weeks, followed by
administering the target drug at a dose of about 4.0 mg once a week for at least about 4 weeks, such as about 12 weeks, followed by
administering the target drug at a dose of about 4.0 mg once a week for at least about 4 weeks, such as about 28 weeks;
(12) administering the target drug at a dose of about 1.0 mg once a week for at least about 4 weeks, followed by
administering the target drug at a dose of about 2.0 mg once a week for at least about 4 weeks, followed by
administering the target drug at a dose of about 3.0 mg once a week for at least about 4 weeks, followed by
administering the target drug at a dose of about 4.0 mg once a week for at least about 4 weeks, followed by
administering the target drug at a dose of about 5.0 mg once a week for at least about 4 weeks, followed by
administering the target drug at a dose of about 6.0 mg once a week for at least about 4 weeks, followed by
administering the target drug at a dose of about 6.0 mg once a week for at least about 4 weeks, such as about 24 weeks.

14. The method according to any one of claims 1 to 13, wherein the target drug comprises a GLP-1 analogue comprising the structure shown below, or a pharmaceutically acceptable salt thereof,

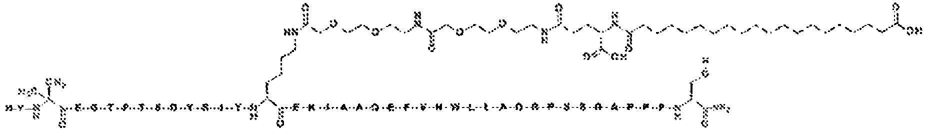

15. The method according to any one of claims 1 to 14, wherein the metabolic disease is obesity or overweight.

16. A method for improving weight control in a subject in need thereof, comprising administering a target drug to the subject according to the method of any one of claims 1 to 13,
wherein the target drug is a GLP-1 analogue comprising the structure shown below, or a pharmaceutically acceptable salt thereof: H-YAibEGTFTSDYSIYK(OEG-OEG-γGlu-C20-OH)EKIAAQEFVNWLLAGGPSSGAPPPS-NH₂.

17. A method for improving blood glucose control in a subject in need thereof, comprising administering a target drug to the subject according to the method of any one of claims 1 to 13,
wherein the target drug is a GLP-1 analogue comprising the structure shown below, or a pharmaceutically acceptable salt thereof: H-YAibEGTFTSDYSIYK(OEG-OEG-γGlu-C20-OH)EKIAAQEFVNWLLAGGPSSGAPPPS-NH₂.

18. A method for improving blood lipid control in a subject in need thereof, comprising administering a target drug to the subject according to the method of any one of claims 1 to 13,
wherein the target drug is a GLP-1 analogue comprising the structure shown below, or a pharmaceutically acceptable salt thereof: H-YAibEGTFTSDYSIYK(OEG-OEG-γGlu-C20-OH)EKIAAQEFVNWLLAGGPSSGAPPPS-NH₂.

19. The method according to any one of claims 1 to 18, wherein the metabolic disease is diabetes, preferably type 2 diabetes.

20. A method for treating type 2 diabetes in a subject in need thereof, comprising administering a target drug to the subject according to the method of any one of claims 1 to 13,
wherein the target drug is a GLP-1 analogue comprising the structure shown below, or a pharmaceutically acceptable salt thereof: H-YAibEGTFTSDYSIYK(OEG-OEG-γGlu-C20-OH)EKIAAQEFVNWLLAGGPSSGAPPPS-NH₂,
wherein the subject with type 2 diabetes satisfies at least one of the following conditions:
1) poor efficacy with lifestyle intervention alone;
2) poor efficacy after treatment with metformin, or metformin in combination with an SGLT2 inhibitor;
3) HbA1c ≥ 7.5% and/or HbA1c ≤ 11%;
4) BMI≥22kg/m² and/or BMI≤40.0kg/m².

21. The method according to claim 19 or 20, wherein the subject is further administered metformin, or metformin and an SGLT2 inhibitor.

22. The method according to any one of claims 19 to 21, wherein the method ameliorates or reduces at least one of the following indicators in the subject:
1) HbA1c;
2) the proportions of subjects with HbA1c < 7.0%, HbA1c ≤ 6.5%, or HbA1c < 5.7%;
3) fasting plasma glucose, serum insulin, and C-peptide;
4) 2h post-mixed-meal plasma glucose, serum insulin, and C-peptide after a mixed meal;
5) pancreatic β-cell function and insulin resistance;
6) average 7-point self-monitored blood glucose;
7) body weight, waist circumference, and BMI;
8) the proportions of subjects with a body weight reduction of ≥ 5%, ≥ 10%, or ≥ 15%;
9) the proportion of subjects with HbA1c < 7.0% and a body weight reduction of ≥ 5%;
10) blood lipids, preferably including one or more of TC, LDL-C, HDL-C and TG;
11) blood pressure, preferably including systolic and/or diastolic blood pressure.
